(19) Europäisches Patentamt
European Patent Office
Office européen des brevets

(11) EP 1 544 202 A1

(12) **EUROPEAN PATENT APPLICATION**
published in accordance with Art. 158(3) EPC

(43) Date of publication:
**22.06.2005 Bulletin 2005/25**

(21) Application number: **03753959.0**

(22) Date of filing: **26.09.2003**

(51) Int Cl.[7]: **C07D 495/04**, A01N 43/90

(86) International application number:
**PCT/JP2003/012356**

(87) International publication number:
**WO 2004/029060 (08.04.2004 Gazette 2004/15)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **26.09.2002 JP 2002281271**

(71) Applicant: **Nihon Nohyaku Co., Ltd.**
**Tokyo 103-8236 (JP)**

(72) Inventors:
- **OTA, Chikako Nihon Nohyaku Co., Ltd.**
  **Osaka 586-0094 (JP)**
- **KUMATA, Shuji Nihon Nohyaku Co., Ltd.**
  **Osaka 586-0094 (JP)**
- **KAWAGUCHI, Shinji Nihon Nohyaku Co., Ltd.**
  **Osaka 586-0094 (JP)**

(74) Representative: **HOFFMANN - EITLE**
**Patent- und Rechtsanwälte**
**Arabellastrasse 4**
**81925 München (DE)**

(54) **NOVEL HERBICIDES, USAGE THEREOF, NOVEL THIENOPYRIMIDINE DERIVATIVES, INTERMEDIATES OF THE SAME, AND PROCESS FOR PRODUCTION THEREOF**

(57) A herbicide comprising, as an active ingredient, a substituted thienopyrimidine derivative represented by the formula (I):

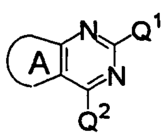

(I)

wherein all symbols are defined in the description, a method of using the same, a novel compound useful as the herbicide and a process for producing the same, and an intermediate thereof.

EP 1 544 202 A1

## Description

Technical Field

The present invention relates to a herbicide containing a substituted thienopyrimidine derivative as an active ingredient, a method of using the same, a novel compound useful as the herbicide and a process for producing the same, and an intermediate thereof

Background of the Invention

It is indispensable to supply important crops stably for solving the food crisis caused by world population growth that is expected to come near future. For stable supply of the crops, it is necessary to kill or control weeds harmful at cultivation and harvest of the crops economically and efficiently. Therefore, it is increasingly important to develop a novel herbicide or plant growth regulator which can offer a solution.

On the other hand, most of the reports on substituted thienopyrimidines are those on physiological activities thereof in pharmaceutical application and no description thereof as herbicides have not been found in the reports (for example, cf. WO98/50037, WO96/14319, EP-150469-A1, DE-2323149 and WO02/55524).

Disclosure of the Invention

In order to respond to such a social request, an object of the present invention is to provide a novel herbicide having a high safety for crops and also an excellent herbicidal activity against weeds.

As a result of intensive studies for solving the above-described problem, inventors of the present invention have found that a thienopyrimidine having a specific substituent exhibit a herbicidal activity, and have accomplished the invention.

Namely, the present invention relates to the following (1) to (20):

(1) A herbicide comprising, as an active ingredient, a substituted thienopyrimidine derivative represented by formula (I):

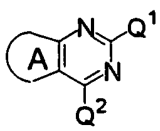

(I)

wherein A represents the following A1 or A2:

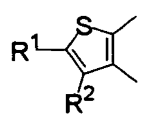

A1

wherein $R^1$ represents hydrogen or alkyl which may be substituted, and
$R^2$ represents hydrogen, halogen or alkyl which may be substituted:

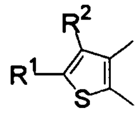

A2

wherein $R^1$ and $R^2$ have the same meanings as described above,
$Q^1$ represents hydrogen, halogen, cyano, hydroxyl, carboxyl, or $-X^1R^3$,
wherein $X^1$ is a single bond, -O-, $-SO_n-$ in which n represents an integer of 0 to 2, $-OSO_n-$ in which n has the same meaning as described above, -CO-, $-CO_2-$, $-OCO_2-$, or -OC(O)-, and
$R^3$ represents alkyl which may be substituted, alkenyl which may be substituted, alkynyl which may be substituted, amino which may be substituted, aryl which may be substituted, or a heterocyclic group which may be substituted,

$Q^2$ represents hydrogen, halogen, hydroxyl, or $-X^2R^4$,

wherein $X^2$ is a single bond, -O-, $-SO_n-$ in which n has the same meaning as described above, $-OSO_n-$ in which n has the same meaning as described above, -CO-, $-CO_2-$, $-OCO_2-$, or -OC(O)-, and

$R^4$ represents alkyl which may be substituted, alkenyl which may be substituted, alkynyl which may be substituted, amino which may be substituted, aryl which may be substituted, or a heterocyclic group which may be substituted.

(2) A herbicide comprising, as an active ingredient, a substituted thienopyrimidine derivative represented by formula (I):

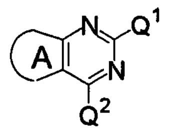

(I)

wherein A represents the following A1 or A2:

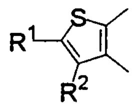

A1

wherein $R^1$ represents hydrogen, $(C_1-C_6)$alkyl, or halo$(C_1-C_6)$alkyl, and

$R^2$ represents hydrogen, halogen, $(C_1-C_6)$alkyl, or halo$(C_1-C_6)$alkyl:

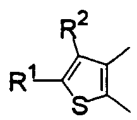

A2

wherein $R^1$ and $R^2$ have the same meanings as described above,

$Q^1$ represents hydrogen, halogen, cyano, hydroxyl, carboxyl, or $-X^1R^3$,

wherein $X^1$ is a single bond, -O-, $-SO_n-$ in which n represents an integer of 0 to 2, $-OSO_n-$ in which n has the same meaning as described above, -CO-, $-CO_2-$, $-OCO_2-$, or -OC(O)-, and

$R^3$ represents $(C_1-C_{10})$alkyl; halo$(C_1-C_{10})$alkyl; cyclo$(C_3-C_6)$alkyl; halocyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkyl$(C_3-C_6)$cycloalkyl; cyclo$(C_3-C_6)$alkyl$(C_1-C_4)$alkyl; $(C_1-C_3)$alkoxycarbonyl$(C_1-C_3)$alkyl; (amino)hydroxy$(C_2-C_6)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl, halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which dihaloalkyl moieties are the same or different;

phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl($C_1$-$C_6$)alkyl; substituted aryl($C_1$-$C_6$)alkyl having on its ring one or more substituents which are the same or different and are selected from the group consisting of halogen atom, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazol, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy,

$Q^2$ represents hydrogen, halogen, hydroxyl, or -$X^2R^4$;

wherein $X^2$ represents a single bond, -O-, -$SO_n$- in which n has the same meaning as described above, -$OSO_n$- in which n has the same meaning as described above, -CO-, -$CO_2$-, -$OCO_2$-, or -OC(O)-, and

$R^4$ represents ($C_1$-$C_{10}$)alkyl; halo($C_1$-$C_{10}$)alkyl; cyclo($C_3$-$C_6$)alkyl; halocyclo($C_3$-$C_6$)alkyl; ($C_1$-$C_6$)alkoxy($C_1$-$C_6$)alkyl; halo($C_1$-$C_6$)alkoxy($C_1$-$C_6$)alkyl; ($C_1$-$C_4$)alkylcyclo($C_3$-$C_6$)alkyl; cyclo($C_3$-$C_6$)alkyl($C_1$-$C_4$)alkyl; ($C_1$-$C_3$)alkoxycarbonyl($C_1$-$C_3$)alkyl; ($C_1$-$C_4$)alkylthio($C_1$-$C_4$)alkyl; ($C_1$-$C_4$)alkylsulfinyl($C_1$-$C_4$)alkyl; ($C_1$-$C_4$)alkylsulfonyl($C_1$-$C_4$)alkyl; ($C_2$-$C_6$)alkenyl; halo($C_2$-$C_6$)alkenyl; hydroxy($C_2$-$C_6$)alkenyl; hydroxyhalo($C_2$-$C_6$)alkenyl; phenyl($C_2$-$C_6$)alkenyl; ($C_2$-$C_6$)alkynyl; halo($C_2$-$C_6$)alkynyl; amino; mono($C_1$-$C_6$)alkylamino; monohalo($C_1$-$C_6$)alkylamino; di($C_1$-$C_6$)alkylamino of which alkyl moieties are the same or different; dihalo($C_1$-$C_6$)alkylamino of which haloalkyl moieties are the same or different;

phenylamino; substituted phenylamino having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

aryl; substituted aryl having one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

aryl($C_1$-$C_6$)alkyl; substituted aryl($C_1$-$C_6$)alkyl having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

a heterocyclic group of which heterocycle has the same meaning as described above; a substituted heterocyclic group of which heterocycle has the same meaning as described above, having one or more substituents which are the same or different and are selected from the group consisting of halogen, cyano, nitro, ($C_1$-$C_6$)alkyl,

halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy.

(3) The herbicide according to (1) or (2),

wherein $Q^1$ is $OR^3$,

wherein $R^3$ represents ($C_1$-$C_{10}$)alkyl; halo($C_1$-$C_{10}$)alkyl; cyclo($C_3$-$C_6$)alkyl; ($C_1$-$C_6$)alkoxy($C_1$-$C_6$)alkyl; halo($C_1$-$C_6$)alkoxy($C_1$-$C_6$)alkyl; ($C_1$-$C_4$)alkyl($C_3$-$C_6$)cycloalkyl; cyclo($C_3$-$C_6$)alkyl($C_1$-$C_4$)alkyl; ($C_1$-$C_3$)alkoxycarbonyl($C_1$-$C_3$)alkyl; (amino)hydroxy($C_2$-$C_6$)alkyl; ($C_1$-$C_4$)alkylthio($C_1$-$C_4$)alkyl; ($C_1$-$C_4$)alkylsulfinyl($C_1$-$C_4$)alkyl; ($C_1$-$C_4$)alkylsulfonyl($C_1$-$C_4$)alkyl; ($C_2$-$C_6$)alkenyl; halo($C_2$-$C_6$)alkenyl; hydroxy($C_2$-$C_6$)alkenyl; hydroxyhalo($C_2$-$C_6$)alkenyl; phenyl($C_2$-$C_6$)alkenyl; ($C_2$-$C_6$)alkynyl; halo($C_2$-$C_6$)alkynyl; amino; mono($C_1$-$C_6$)alkylamino; monohalo($C_1$-$C_6$)alkylamino; di($C_1$-$C_6$)alkylamino of which alkyl moieties are the same or different; dihalo($C_1$-$C_6$)alkylamino of which dihaloalkyl moieties are the same or different;

phenylamino; substituted phenylamino having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, ($C_1$-$C_6$)alkylthio, halo($C_1$-$C_6$)alkylthio, ($C_1$-$C_6$)alkylsulfinyl, halo($C_1$-$C_6$)alkylsulfinyl, ($C_1$-$C_6$)alkylsulfonyl, halo($C_1$-$C_6$)alkylsulfonyl, mono($C_1$-$C_6$)alkylamino, and di($C_1$-$C_6$)alkylamino of which alkyl moieties are the same or different;

aryl; substituted aryl having one or more substituents which are the same or different and are selected from the group consisting of halogen, cyano, nitro, hydroxyl, amino, SH, ($C_1$-$C_6$)alkyl, halo($C_1$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, ($C_1$-$C_6$)alkylthio, halo($C_1$-$C_6$)alkylthio, ($C_1$-$C_6$)alkylsulfinyl, halo($C_1$-$C_6$)alkylsulfinyl, ($C_1$-$C_6$)alkylsulfonyl, halo($C_1$-$C_6$)alkylsulfonyl, mono($C_1$-$C_6$)alkylamino, and di($C_1$-$C_6$)alkylamino of which alkyl moieties are the same or different;

aryl($C_1$-$C_6$)alkyl; substituted aryl($C_1$-$C_6$)alkyl having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, ($C_1$-$C_6$)alkylthio, halo($C_1$-$C_6$)alkylthio, ($C_1$-$C_6$)alkylsulfinyl, halo($C_1$-$C_6$)alkylsulfinyl, ($C_1$-$C_6$)alkylsulfonyl, halo($C_1$-$C_6$)alkylsulfonyl, mono($C_1$-$C_6$)alkylamino, and di($C_1$-$C_6$)alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazol, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, ($C_1$-$C_6$)alkyl, halo($C_1$-$C_6$)alkyl, ($C_1$-$C_6$)alkylcarbonyloxy, ($C_1$-$C_6$)alkoxy, and halo($C_1$-$C_6$)alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are same or different and are selected from the group consisting of halogen, cyano, nitro, ($C_1$-$C_6$)alkyl, halo($C_1$-$C_6$)alkyl, ($C_1$-$C_6$)alkylcarbonyloxy, ($C_1$-$C_6$)alkoxy, and halo($C_1$-$C_6$)alkoxy.

(4) The herbicide according to any one of (1) to (3),

wherein $Q^2$ is ($C_1$-$C_{10}$)alkyl; cyclo($C_3$-$C_6$)alkyl; ($C_1$-$C_6$)alkylthio; cyclo($C_3$-$C_6$)alkylthio; ($C_1$-$C_6$)alkylsulfinyl; cyclo($C_3$-$C_6$)alkylsulfinyl; ($C_1$-$C_6$)alkylsulfonyl; cyclo($C_3$-$C_6$)alkylsulfonyl;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazol, tetrahydropyran, pyridine, pyrimidine, pyridazine, morpholine, thiomorpholine, piperazine, piperidine, or oxazine;

a substituted heterocyclic group of which heterocycle has the same meaning as described above, having, its on ring, one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio;

heterocyclic oxy of which heterocycle has the same meaning as described above; heterocyclic oxy of which heterocycle has the same meaning as described above, having, its on ring, one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio; or

$-X^2R^4$,

wherein $X^2$ is a single bond, -O-, -S-, -SO- or $-SO_2-$, and

$R^4$ is phenyl which is substituted with one or more substituents which are the same or different and selected from the group consisting of fluorine-containing $(C_1-C_6)$alkyl, fluorine-containing $(C_1-C_6)$alkoxy, and fluorine-containing $(C_1-C_6)$alkylthio and which is optionally substituted with one or more substituents which are the same or different and are selected from the group consisting of halogen, cyano, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, and halocyclo$(C_3-C_6)$alkylsulfonyl.

(5) The herbicide according to (1) or (2),

wherein $Q^1$ is hydrogen; halogen; cyano; hydroxyl; carboxyl; $(C_1-C_{10})$alkyl; halo$(C_1-C_6)$alkyl; $(C_1-C_6)$alkoxy$(C_1-C_6)$alkyl; halo$(C_1-C_6)$alkoxy$(C_1-C_6)$alkyl; $(C_1-C_{10})$alkoxy; halo$(C_1-C_6)$alkoxy; cyclo$(C_3-C_6)$alkoxy; halocyclo$(C_3-C_6)$alkoxy; $(C_1-C_6)$alkoxy$(C_1-C_6)$alkoxy; halo$(C_1-C_6)$alkoxy$(C_1-C_6)$alkoxy;$(C_1-C_3)$alkoxycarbonyl$(C_1-C_3)$alkoxy; (amino)hydroxy$(C_2-C_6)$alkoxy; $(C_1-C_6)$alkylthio$(C_1-C_6)$alkoxy; $(C_1-C_6)$alkylsulfinyl$(C_1-C_6)$alkoxy; $(C_1-C_6)$alkylsulfonyl$(C_1-C_6)$alkoxy; di$(C_1-C_3)$alkylamino$(C_1-C_3)$alkoxy of which alkyl moieties are the same or different; $(C_2-C_6)$alkenyl; halo$(C_2-C_6)$alkenyl; hydroxyhalo$(C_2-C_6)$alkenyl; $(C_2-C_6)$alkenyloxy; halo$(C_2-C_6)$alkenyloxy; $(C_2-C_6)$alkynyloxy; amino; mono$(C_1-C_6)$alkylamino; di$(C_1-C_6)$alkylamino of which alkyl moieties are the same or different; substituted phenyl which is substituted with halo$(C_1-C_3)$alkyls which are the same or different; pyrrolyl; imidazolyl; substituted imidazolyl which is substituted with one or more $(C_1-C_3)$ alkyl which are the same or different; pyrazolyl; substituted pyrazolyl which is substituted with one or more $(C_1-C_3)$alkyls or halo$(C_1-C_6)$alkyls which are the same or different; substituted pyrazolyl$(C_1-C_3)$alkyl which is substituted with one or more halo$(C_1-C_3)$alkyls which are the same or different; triazolyl; phenoxy; substituted phenoxy which is substituted with one or more substituents which are the same or different and are selected from the group consisting of halo$(C_1-C_3)$alkyl and halo$(C_1-C_3)$alkoxy; $(C_1-C_3)$alkylthio; $(C_1-C_3)$alkylsulfinyl; $(C_1-C_3)$alkylsulfonyl; $(C_1-C_6)$alkylsulfonyloxy; halo$(C_1-C_6)$alkylsulfonyloxy; phenylsulfonyloxy; substituted phenylsulfonyloxy which is substituted with one or more $(C_1-C_3)$alkyls which are the same or different; di$(C_1-C_3)$alkylaminosulfonyloxy of which alkyl moieties are the same or different; $(C_2-C_4)$alkenylsulfonyloxy; $(C_1-C_3)$alkylcarbonyloxy; $(C_1-C_3)$alkoxycarbonyloxy; $(C_1-C_3)$alkoxycarbonyl; aminocarbonyl; or substituted phenylaminocarbonyl which is substituted with one or more halogens which are the same or different,

$Q^2$ represents halogen; hydroxyl; $(C_1-C_6)$alkyl; halo$(C_1-C_6)$alkyl; cyclo$(C_3-C_6)$alkyl; halocyclo$(C_3-C_6)$alkyl; $(C_1-C_6)$alkoxy; halo$(C_1-C_6)$alkoxy; $(C_1-C_6)$alkylthio; $(C_1-C_6)$alkylsulfinyl; $(C_1-C_6)$alkylsulfonyl; substituted phenyl having one or more substituents which are the same or different and are selected from the group consisting of halogen, cyano, $(C_1-C_3)$alkyl, halo$(C_1-C_3)$alkyl, $(C_1-C_3)$alkoxy, halo$(C_1-C_3)$alkoxy, and halo$(C_1-C_3)$alkylthio; substituted pyrazolyl having one or more substituents which are the same or different and are selected from the group consisting of halogen, $(C_1-C_3)$alkyl, and halo$(C_1-C_3)$alkyl; furyl; substituted thienyl which is substituted with one or more $(C_1-C_3)$alkyls which are the same or different; substituted pyridyl having one halogens which are the same or different; or substituted phenoxy having one or more substituents which are the same or different and are selected from the group consisting of halogen, cyano, $(C_1-C_3)$alkyl, halo$(C_1-C_3)$alkyl, $(C_1-C_3)$alkoxy, halo$(C_1-C_3)$alkoxy, and halo$(C_1-C_3)$alkylthio.

(6) A herbicidal method, which comprises carrying out a soil treatment, a field foliar treatment, or an irrigation treatment with an effective amount of the herbicide according to any one of (1) to (5).

(7) A substituted thienopyrimidine derivative represented by formula (I):

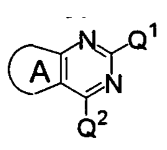

(I)

wherein A represents the following A1 or A2:

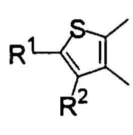

A1

wherein $R^1$ represents hydrogen, $(C_1-C_6)$alkyl, or halo$(C_1-C_6)$alkyl,
$R^2$ represents hydrogen, halogen, $(C_1-C_6)$alkyl, or halo$(C_1-C_6)$alkyl:

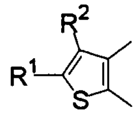

A2

wherein $R^1$ and $R^2$ have the same meanings as described above,
$Q^1$ represents hydrogen, halogen, cyano, hydroxyl, carboxyl, or $-X^1R^3$,
wherein $X^1$ is a single bond, -O-, $-SO_n-$ in which n represents an integer of 0 to 2, $-OSO_n-$ in which n has the same meaning as described above, -CO-, $-CO_2-$, $-OCO_2-$, or -OC(O)-,
$R^3$ represents $(C_1-C_{10})$alkyl; halo$(C_1-C_{10})$alkyl; cyclo$(C_3-C_6)$alkyl; halocyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkyl$(C_3-C_6)$cycloalkyl; cyclo$(C_3-C_6)$alkyl$(C_1-C_4)$alkyl; $(C_1-C_3)$alkoxycarbonyl$(C_1-C_3)$alkyl; (amino)hydroxy$(C_2-C_6)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which haloalkyl moieties are the same or different; phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;
aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;
aryl$(C_1-C_6)$alkyl; substituted aryl$(C_1-C_6)$alkyl having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;
a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrahydropyran, pyridine, pyrimidine, pyridazine, tetrazol, morpholine, thiomorpholine, piperazine, piperidine, or oxazine;
a substituted heterocyclic group of which heterocycle has the same meaning as described above, having one or more substituents which are the same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy;
heterocyclic $(C_1-C_6)$alkyl of which heterocycle has the same meaning as described above; or substituted heterocyclic $(C_1-C_6)$alkyl of which heterocycle has the same meaning as described above, having, on its ring, one or more substituents which are the same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy,
$Q^2$ represents hydrogen, halogen, hydroxyl, or $-X^2R^4$,
wherein $X^2$ is a single bond, -O-, $-SO_n-$ in which n has the same meaning as described above, $-OSO_n-$ in which n has the same meaning as described above, -CO-, $-CO_2-$, $-OCO_2-$, or -OC(O)-, and

$R^4$ represents $(C_1-C_{10})$alkyl; halo$(C_1-C_{10})$alkyl; cyclo$(C_3-C_6)$alkyl; halocyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkylcyclo$(C_3-C_6)$alkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which haloalkyl moieties are the same or different;

phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl$(C_1-C_6)$alkyl; substituted aryl$(C_1-C_6)$alkyl having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

a heterocyclic group of which heterocycle has the same meaning as described above; a substituted heterocyclic group of which heterocycle has the same meaning as described above, having one or more substituents which are same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy;

heterocyclic $(C_1-C_6)$alkyl of which heterocycle has the same meaning as described above; or substituted heterocyclic $(C_1-C_6)$alkyl of which heterocycle has the same meaning as described above, having, on its ring, one or more substituents which are the same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy.

(8) The substituted thienopyrimidine derivative according to (7),

wherein A represents A1,

$Q^1$ represents $OR^3$, wherein $R^3$ has the same meaning as defined in (7),

$Q^2$ represents hydrogen, halogen, hydroxyl, or $-X^2R^4$,

wherein $X^2$ has the same meaning as defined in (7), and

$R^4$ is $(C_1-C_{10})$alkyl; halo$(C_1-C_{10})$alkyl; cyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkylcyclo$(C_3-C_6)$alkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which haloalkyl moieties are the same or different;

phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;

substituted aryl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

a heterocyclic ring of which heterocycle has the same meaning as defined in (7); or a substituted heterocyclic ring of which heterocycle has the same meaning as described above, having one or more substituents which are the same or different and

are selected from the group consisting of halogen, cyano, nitro, $(C_1-C_6)$alkyl, halo$(C_1-C_6)$alkyl, $(C_1-C_6)$alkylcarbonyloxy, $(C_1-C_6)$alkoxy, and halo$(C_1-C_6)$alkoxy, and

wherein

(a) when $X^2$ is a single bond, $R^4$ is not amino, mono$(C_1-C_6)$alkylamino or monohalo$(C_1-C_6)$alkylamino, and
(b) when $X^2$ is -O-, $R^4$ is not $(C_1-C_{10})$alkyl.

(9) The substituted thienopyrimidine derivative according to (7),

wherein A represents A1,

$Q^1$ represents hydrogen, halogen, cyano, hydroxyl, carboxyl, or $-X^1R^3$;

wherein $X^1$ is a single bond, $-SO_n-$ in which n represents an integer of 0 to 2, $-OSO_n-$ in which n has the same meaning as described above, -CO-, $-CO_2-$, $-OCO_2-$, or -OC(O)-;

$R^3$ has the same meaning as defined in (7),

$Q^2$ represents substituted aryl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

substituted aryloxy having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

substituted arylthio having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

substituted arylsulfinyl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio; or

substituted arylsulfonyl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio, and

wherein, when $Q^1$ is $(C_1-C_6)$alkyl in which $X^1$ is a single bond and $R^3$ is $(C_1-C_6)$alkyl, $Q^2$ is not substituted aryloxy which is substituted with at least one fluorine-containing alkyl.

(10) The substituted thienopyrimidine derivative according to (7),

wherein A represents A1,

$Q^1$ represents hydrogen; halogen; cyano; hydroxyl; carboxyl; $(C_1-C_{10})$alkyl; halo$(C_1-C_6)$alkyl; $(C_1-C_6)$alkoxy$(C_1-C_6)$alkyl; halo$(C_1-C_6)$alkoxy$(C_1-C_6)$alkyl; $(C_1-C_{10})$alkoxy; halo$(C_1-C_6)$alkoxy; cyclo$(C_3-C_6)$alkoxy; halocyclo$(C_3-C_6)$alkoxy; $(C_1-C_6)$alkoxy$(C_1-C_6)$alkoxy; halo$(C_1-C_6)$alkoxy$(C_1-C_6)$alkoxy; $(C_1-C_3)$alkoxycarbonyl$(C_1-C_3)$alkoxy; (amino)hydroxy$(C_2-C_6)$alkoxy; $(C_1-C_6)$alkylthio$(C_1-C_6)$alkoxy; $(C_1-C_6)$alkylsulfinyl$(C_1-C_6)$alkoxy; $(C_1-C_6)$alkylsulfonyl$(C_1-C_6)$alkoxy; di$(C_1-C_3)$alkylamino$(C_1-C_3)$ alkoxy of which alkyl moieties are the same or different; $(C_2-C_6)$alkenyl; halo$(C_2-C_6)$alkenyl; hydroxyhalo$(C_2-C_6)$alkenyl; $(C_2-C_6)$alkenyloxy; halo$(C_2-C_6)$alkenyloxy; $(C_1-C_6)$alkynyloxy; amino; mono$(C_1-C_6)$alkylamino; di$(C_1-C_6)$alkylamino of which alkyl moieties are the same or different; substituted phenyl which is substituted with one or more halo$(C_1-C_3)$alkyls which are the same or different; pyrrolyl; imidazolyl; substituted imidazolyl which is substituted with one or more $(C_1-C_3)$alkyls which are the same or different; pyrazolyl; substituted pyrazolyl having one or more substituents which are the same or different and are selected from $(C_1-C_3)$alkyl and halo$(C_1-C_3)$alkyl; substituted pyrazolyl$(C_1-C_3)$alkyl having one or more substituents which are the same or different and are selected from the group consisting of halo$(C_1-C_3)$alkyl; triazolyl; phenoxy; substituted phenoxy having one or more substituents which are the same or different and are selected from the group consisting of halo$(C_1-C_3)$alkyl and halo$(C_1-C_3)$alkoxy; $(C_1-C_3)$alkylthio; $(C_1-C_3)$alkylsulfinyl; $(C_1-C_3)$alkylsulfonyl; $(C_1-C_6)$alkylsulfonyloxy; halo$(C_1-C_6)$alkylsulfonyloxy; phenylsulfonyloxy; substituted phenylsulfonyloxy having one or more substituents which is substituted with one or more $(C_1-C_3)$alkyls which are the same or different; di$(C_1-C_3)$alkylaminosulfonyloxy of which alkyl moieties are the same or different; $(C_2-C_4)$alkenylsulfonyloxy; $(C_1-C_3)$alkylcarbonyloxy; $(C_1-C_3)$alkoxycarbonyl; aminocarboxyl; substituted phenylaminocarbonyl which is substituted with one or more halogens on its ring which are the same or different,

$Q^2$ represents halogen; hydroxyl; $(C_1-C_6)$alkyl; halo$(C_1-C_6)$alkyl; cyclo$(C_3-C_6)$alkyl; halocyclo$(C_3-C_6)$alkyl;

$(C_1-C_6)$alkoxy; halo$(C_1-C_6)$alkoxy; $(C_1-C_6)$alkylthio; $(C_1-C_6)$alkylsulfinyl; $(C_1-C_6)$alkylsulfonyl; substituted phenyl having one or more substituents which are the same or different and are selected from the group consisting of halogen, cyano, $(C_1-C_3)$alkyl, halo$(C_1-C_3)$alkyl, $(C_1-C_3)$alkoxy, halo$(C_1-C_3)$alkoxy, and halo$(C_1-C_3)$alkylthio; substituted pyrazolyl having one or more substituents which are the same or different and are selected from the group consisting of halogen, $(C_1-C_3)$alkyl, and halo$(C_1-C_3)$alkyl; furyl; substituted thienyl substituted with one or more $(C_1-C_3)$alkyls which are the same or different; substituted pyridyl substituted with one or more halogens which are the same or different; or substituted phenoxy having one or more substituents which are the same or different and are selected from the group consisting of halogen, cyano, $(C_1-C_3)$alkyl, halo$(C_1-C_3)$alkyl, $(C_1-C_3)$alkoxy, halo $(C_1-C_3)$alkoxy, and halo$(C_1-C_3)$alkylthio.

(11) The substituted thienopyrimidine derivative according to any one of (7), (9) and (10), wherein A represents A1, and $Q^1$ represents halogen or hydroxyl.

(12) The substituted thienopyrimidine derivative according to (7),

wherein A is A2,

$Q^1$ represents $-OR^3$, wherein $R^3$ has the same meaning as defined in (7),

$Q^2$ represents hydrogen, halogen, hydroxyl, or $-X^2R^4$,

wherein $X^2$ is a single bond, -O-, $-SO_n-$ in which n represents an integer of 0 to 2, $-OSO_n-$ in which n has the same meaning as described above, $-CO_2-$, $-OCO_2-$, or -OC(O)-;

$R^4$ represents $(C_1-C_{10})$alkyl; halo$(C_1-C_{10})$alkyl; cyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkylcyclo$(C_3-C_6)$alkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which alkyl moieties are the same or different;

phenylamino; substituted phenylamino which having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;

aryl; substituted aryl which having one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different, and

wherein, when $X^2$ is a single bond, $R^4$ is not amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which alkyl moieties are the same or different; phenylamino; substituted phenylamino which has one or more substituents on its ring which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different; $(C_1-C_{10})$alkyl; halo$(C_1-C_{10})$alkyl; cyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkyl$(C_3-C_6)$cycloalkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; or $(C_1-C_4)$alkylsulfonyl $(C_1-C_4)$alkyl.

(13) The substituted thienopyrimidine derivative according to (7),

wherein A is A2,

$Q^1$ represents halogen, cyano, carboxyl, or $-X^1R^3$;

wherein $X^1$ is a single bond, $-SO_n-$ in which n represents an integer of 0 to 2, $-OSO_n-$ in which n has the same meaning as described above, -CO-, $-CO_2-$, $-OCO_2-$, or -OC(O)-, and

$R^3$ has the same meaning as defined in (7), and

$Q^2$ represents:

substituted aryl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

substituted aryloxy having one or more substituents which are the same or different and are selected from the

group consisting of fluorine-containing ($C_1$-$C_6$)alkyl, fluorine-containing ($C_1$-$C_6$)alkoxy, and fluorine-containing ($C_1$-$C_6$)alkylthio;

substituted arylthio having one or more substituents which are the same or different and are selected from the group consisting of fluorine-containing ($C_1$-$C_6$)alkoxy, and fluorine-containing ($C_1$-$C_6$)alkylthio;

substituted arylsulfinyl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio; or

substituted arylsulfonyl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio.

(14) The substituted thienopyrimidine derivative according to (7),

wherein A is A2,

$Q^1$ represents halogen; cyano; carboxyl; ($C_1$-$C_{10}$)alkyl; halo($C_1$-$C_6$)alkyl; ($C_1$-$C_6$)alkoxy($C_1$-$C_6$)alkyl; halo ($C_1$-$C_6$)alkoxy($C_1$-$C_6$)alkyl; ($C_1$-$C_{10}$)alkoxy; halo($C_1$-$C_6$)alkoxy; cyclo($C_3$-$C_6$)alkoxy; halocyclo($C_3$-$C_6$)alkoxy; ($C_1$-$C_6$)alkoxy($C_1$-$C_6$)alkoxy; halo($C_1$-$C_6$)alkoxy($C_1$-$C_6$)alkoxy; ($C_1$-$C_3$)alkoxycarbonyl($C_1$-$C_3$)alkoxy; hydroxyamino($C_1$-$C_3$)alkoxy($C_1$-$C_3$)alkoxy; ($C_1$-$C_6$)alkylthio($C_1$-$C_6$)alkoxy; ($C_1$-$C_6$)alkylsulfinyl($C_1$-$C_6$)alkoxy; ($C_1$-$C_6$)alkylsulfonyl($C_1$-$C_6$)alkoxy; di($C_1$-$C_3$)alkylamino($C_1$-$C_3$)alkoxy; ($C_2$-$C_6$)alkenyl; halo($C_2$-$C_6$)alkenyl; hydroxyhalo($C_2$-$C_6$) alkenyl; ($C_2$-$C_6$)alkenyloxy; halo($C_2$-$C_6$)alkenyloxy; ($C_2$-$C_6$)alkynyloxy; amino; mono($C_1$-$C_6$)alkylamino; di($C_1$-$C_6$) alkylamino of which alkyl moieties are the same or different; substituted phenyl which is substituted with one or more halo($C_1$-$C_3$)alkyls which are the same or different; pyrolyl; imidazolyl; substituted imidazolyl which is substituted with one or more ($C_1$-$C_3$)alkyls which are the same or different; pyrazolyl; substituted pyrazolyl having one or more substituents which are the same or different and are selected from the group consisting of ($C_1$-$C_3$)alkyl and halo($C_1$-$C_3$)alkyl; substituted pyrazolyl($C_1$-$C_3$)alkyl which is substituted on its ring with one or more halo($C_1$-$C_3$) alkyls which are the same or different; triazolyl; phenoxy; substituted phenoxy having one or more substituents which are the same or different and are selected from the group consisting of halo($C_1$-$C_3$)alkyl and halo($C_1$-$C_3$) alkoxy; ($C_1$-$C_3$)alkylthio; ($C_1$-$C_3$)alkylsulfinyl; ($C_1$-$C_3$)alkylsulfonyl; ($C_1$-$C_3$)alkylsulfonyloxy; halo($C_1$-$C_3$)alkylsulfonyloxy; phenylsulfonyloxy; substituted phenylsulfonyloxy which is substituted with one or more ($C_1$-$C_3$)alkyls which are the same or different; di($C_1$-$C_3$)alkylaminosulfonyloxy of which alkyl moieties are the same or different; ($C_2$-$C_4$) alkenylsulfonyloxy; ($C_1$-$C_3$)alkylcarbonyloxy; ($C_1$-$C_3$)alkoxycarbonyloxy; ($C_1$-$C_3$)alkoxycarbonyl; aminocarbonyl; or substituted phenylaminocarbonyl which is substituted with one or more halogens which are the same or different,

$Q^2$ is halogen; hydroxyl; ($C_1$-$C_6$)alkyl; halo($C_1$-$C_6$)alkyl; cyclo($C_3$-$C_6$)alkyl; halocyclo($C_3$-$C_6$)alkyl; ($C_1$-$C_6$) alkoxy; halo($C_1$-$C_6$)alkoxy; ($C_1$-$C_6$)alkylthio; ($C_1$-$C_6$)alkylsulfinyl; ($C_1$-$C_6$)alkylsulfonyl; substituted phenyl having one or more substituents which are the same or different and are selected from the group consisting of halogen, cyano, ($C_1$-$C_3$)alkyl, halo($C_1$-$C_3$)alkyl, ($C_1$-$C_3$)alkoxy, halo($C_1$-$C_3$)alkoxy, and halo($C_1$-$C_3$)alkylthio; or substituted phenoxy having one or more substituents which are the same or different and are selected from the group consisting of halogen, cyano, ($C_1$-$C_3$)alkyl, halo($C_1$-$C_3$)alkyl, ($C_1$-$C_3$)alkoxy, halo($C_1$-$C_3$)alkoxy, and halo($C_1$-$C_3$)alkylthio.

(15) A method for producing a substituted thienopyrimidine derivative represented by formula (I-2):

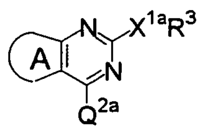

( I - 2 )

wherein A, $R^3$, $X^{1a}$ and $Q^{2a}$ have the same meanings as described below;

which comprises reacting a compound of formula (I-1):

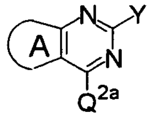

( I - 1)

wherein A represents the following A1 or A2:

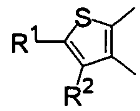 A1

wherein $R^1$ represents hydrogen, $(C_1\text{-}C_6)$alkyl, or halo$(C_1\text{-}C_6)$alkyl, and
$R^2$ represents hydrogen, halogen, $(C_1\text{-}C_6)$alkyl, or halo$(C_1\text{-}C_6)$alkyl:

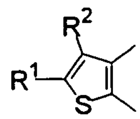 A2

wherein $R^1$ and $R^2$ have the same meanings as described above,
Y represents halogen,
$Q^{2a}$ represents:

substituted aryl having one or more substituents which are the same or different and are selected from the group consisting of fluorine-containing $(C_1\text{-}C_6)$alkyl, fluorine-containing $(C_1\text{-}C_6)$alkoxy, and fluorine-containing $(C_1\text{-}C_6)$alkylthio;
substituted aryloxy having one or more substituents which are the same or different and are selected from the group consisting of fluorine-containing $(C_1\text{-}C_6)$alkyl, fluorine-containing $(C_1\text{-}C_6)$alkoxy, and fluorine-containing $(C_1\text{-}C_6)$alkylthio;
substituted arylthio having one or more substituents which are the same or different and are selected from the group consisting of fluorine-containing $(C_1\text{-}C_6)$alkyl, fluorine-containing $(C_1\text{-}C_6)$alkoxy, and fluorine-containing $(C_1\text{-}C_6)$alkylthio;
substituted arylsulfinyl having one or more substituents which are the same or different and are selected from the group consisting of fluorine-containing $(C_1\text{-}C_6)$alkyl, fluorine-containing $(C_1\text{-}C_6)$alkoxy, and fluorine-containing $(C_1\text{-}C_6)$alkylthio;
substituted arylsulfonyl having one or more substituents which are the same or different and are selected from the group consisting of fluorine-containing $(C_1\text{-}C_6)$alkyl, fluorine-containing $(C_1\text{-}C_6)$alkoxy, and fluorine-containing $(C_1\text{-}C_6)$alkylthio;
with a compound represented by formula (II):

$$R^3X^{1a}H \qquad \text{(II)}$$

wherein $X^{1a}$ is a single bond, -O-, or -S-,

$R^3$ represents $(C_1\text{-}C_{10})$alkyl; halo$(C_1\text{-}C_{10})$alkyl; cyclo$(C_3\text{-}C_6)$alkyl; halocyclo$(C_3\text{-}C_6)$alkyl; $(C_1\text{-}C_6)$alkoxy$(C_1\text{-}C_6)$alkyl; halo$(C_1\text{-}C_6)$alkoxy$(C_1\text{-}C_6)$alkyl; $(C_1\text{-}C_4)$alkyl$(C_3\text{-}C_6)$cycloalkyl; cyclo$(C_3\text{-}C_6)$alkyl$(C_1\text{-}C_4)$alkyl; $(C_1\text{-}C_3)$alkoxycarbonyl$(C_1\text{-}C_3)$alkyl; $(C_1\text{-}C_4)$alkylthio$(C_1\text{-}C_4)$alkyl; $(C_1\text{-}C_4)$alkylsulfinyl$(C_1\text{-}C_4)$alkyl; $(C_1\text{-}C_4)$alkylsulfonyl$(C_1\text{-}C_4)$alkyl; $(C_2\text{-}C_6)$alkenyl; halo$(C_2\text{-}C_6)$alkenyl; hydroxy$(C_2\text{-}C_6)$alkenyl; hydroxyhalo$(C_2\text{-}C_6)$alkenyl; phenyl$(C_2\text{-}C_6)$alkenyl; $(C_2\text{-}C_6)$alkynyl; halo$(C_2\text{-}C_6)$alkynyl; amino; mono$(C_1\text{-}C_6)$alkylamino; monohalo$(C_1\text{-}C_6)$alkylamino; di$(C_1\text{-}C_6)$alkylamino of which alkyl moieties are the same or different; dihalo$(C_1\text{-}C_6)$alkylamino of which dihaloalkyl moieties are the same or different;

phenylamino; substituted phenylamino having on its ring one or more substituents which are the same or different and are selected from the group consisting of halogen, cyano, nitro, hydroxyl, amino, SH, $(C_1\text{-}C_6)$alkyl, halo$(C_1\text{-}C_6)$alkyl, cyclo$(C_3\text{-}C_6)$alkyl, halocyclo$(C_3\text{-}C_6)$alkyl, $(C_2\text{-}C_6)$alkenyl, halo$(C_2\text{-}C_6)$alkenyl, $(C_2\text{-}C_6)$alkynyl, halo$(C_2\text{-}C_6)$alkynyl, $(C_1\text{-}C_6)$alkoxy, halo$(C_1\text{-}C_6)$alkoxy, cyclo$(C_3\text{-}C_6)$alkoxy, halocyclo$(C_3\text{-}C_6)$alkoxy, $(C_1\text{-}C_6)$alkylthio, halo$(C_1\text{-}C_6)$alkylthio, cyclo$(C_3\text{-}C_6)$alkylthio, halocyclo$(C_3\text{-}C_6)$alkylthio, $(C_1\text{-}C_6)$alkylsulfinyl, halo$(C_1\text{-}C_6)$alkylsulfinyl, cyclo$(C_3\text{-}C_6)$alkylsulfinyl, halocyclo$(C_3\text{-}C_6)$alkylsulfinyl, $(C_1\text{-}C_6)$alkylsulfonyl, halo$(C_1\text{-}C_6)$alkylsulfonyl, cyclo$(C_3\text{-}C_6)$alkylsulfonyl, halocyclo$(C_3\text{-}C_6)$alkylsulfonyl, mono$(C_1\text{-}C_6)$alkylamino, and di$(C_1\text{-}C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1\text{-}C_6)$alkyl, halo$(C_1\text{-}C_6)$alkyl, cyclo$(C_3\text{-}C_6)$alkyl, halocyclo$(C_3\text{-}C_6)$alkyl, $(C_2\text{-}C_6)$alkenyl, halo$(C_2\text{-}C_6)$alkenyl, $(C_2\text{-}C_6)$alkynyl, halo$(C_2\text{-}C_6)$alkynyl, $(C_1\text{-}C_6)$

alkoxy, halo($C_1$-$C_6$)alkoxy, cyclo($C_3$-$C_6$)alkexy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

aryl($C_1$-$C_6$)alkyl; substituted aryl($C_1$-$C_6$)alkyl having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo ($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazole, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$) alkoxy, halo($C_1$-$C_6$)alkoxy, cyclo($C_3$-$C_6$)alkoxy, and halocyclo($C_3$-$C_6$)alkoxy.

(16) A process for producing a substituted thieno[2,3-d]pyrimidine derivative represented by formula (I-3):

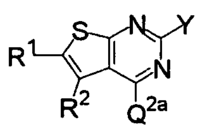

(I - 3)

wherein $R^1$, $R^2$, $Q^{2a}$, and Y have the same meanings as described below,

which comprises carrying out a coupling reaction of 2,4-dihalogenothieno[2,3-d]pyrimidine derivative represented by formula (III):

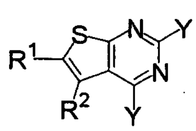

( III )

wherein $R^1$ represents hydrogen, ($C_1$-$C_6$)alkyl, or halo($C_1$-$C_6$)alkyl,
$R^2$ represents hydrogen, halogen, ($C_1$-$C_6$)alkyl, or halo($C_1$-$C_6$)alkyl, and
Y represents halogen which are the same or different,
with a compound represented by formula (IV):

$$Q^{2a}\text{-}L \qquad (IV)$$

wherein $Q^{2a}$ represents substituted aryl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio, and

L represents a leaving group.

(17) 2-Amino-3-(substituted benzoyl)thiophene derivative represented by formula (V):

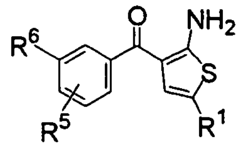

( V )

wherein $R^1$ represents hydrogen, $(C_1-C_6)$alkyl, or halo$(C_1-C_6)$alkyl,
$R^5$ represents hydrogen, halogen, $(C_1-C_6)$alkyl, or halo$(C_1-C_6)$alkyl,
$R^6$ represents fluorine-containing $(C_1-C_6)$alkyl, fluorine-containing $(C_1-C_6)$alkoxy, or fluorine-containing $(C_1-C_6)$alkylthio.

(18) A ureidethiophene derivative represented by formula (VI):

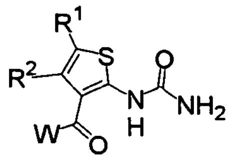

( VI )

wherein $R^1$ represents hydrogen, $(C_1-C_6)$alkyl, or halo$(C_1-C_6)$alkyl,
$R^2$ represents hydrogen, halogen, $(C_1-C_6)$alkyl, or halo$(C_1-C_6)$alkyl, and
W represents aryl or substituted aryl having one or more substituents which are the same or different and are selected from the group consisting of halogen, cyano, nitro, $(C_1-C_6)$alkyl, halo$(C_1-C_6)$alkyl, $(C_1-C_6)$alkoxy, halo $(C_1-C_6)$alkoxy, and halo$(C_1-C_6)$alkylthio.

(19) A substituted benzoylacetonitrile derivative represented by formula (VII):

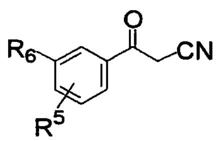

(VII)

wherein $R^5$ represents hydrogen, halogen, $(C_1-C_6)$alkyl, or halo$(C_1-C_6)$alkyl,
$R^6$ represents fluorine-containing $(C_1-C_6)$alkyl, fluorine-containing $(C_1-C_6)$alkoxy, and fluorine-containing $(C_1-C_6)$alkylthio,
and wherein, when $R^6$ is fluorine-containing $(C_1-C_6)$alkyl, $R^5$ is not hydrogen.

(20) 3-(Trifluoromethoxy)ethyl benzoate.

The present invention is described below in detail.

Best Mode for Carrying Out the Invention

The substituted thienopyrimidine derivatives useful as the herbicide according to the present invention are represented by the above formula (I), and a use of the compounds including the compounds disclosed in prior art documents as a novel herbicide has been found.

1. Compounds to be used as herbicide of the present invention

$R^1$ in formula (I) is hydrogen or alkyl which may be substituted.

The substituent for the alkyl group is not particularly limited as far as the resulting compound exhibits a herbicidal activity. Examples thereof include halogens, e.g., fluorine, chlorine, bromine and iodine. Moreover, the above alkyl which may be substituted has preferably from 1 to 6 carbon atoms, more preferably from 1 to 4 carbon atoms.

Preferred specific examples of the alkyl group which may be substituted include $(C_1-C_4)$alkyl, e.g., methyl, ethyl, propyl, isopropyl, butyl, or isobutyl; and halo$(C_1-C_4)$alkyl, e.g., chloromethyl, fluoromethyl, trifluoromethyl, 2-chloroethyl, 2-fluoroethyl, 2,2-difluoroethyl, 2,2,2-trifluoroethyl, 2,2,2-trichloroethyl, 3-chloropropyl, 3-bromopropyl, 3,3,3-trifluoropropyl, 2,2,3,3-tetrafluoropropyl, 2,2,3,3,3-pentafluoropropyl, 1-methyl-2,2,2-trifluoroethyl, 2,2,2-trifluoro-1-(trifluoromethyl)ethyl, 1-methyl-2,2,3,3,3-pentafluoropropyl, 4-chlorobutyl, or 4,4,4-trifluorobutyl.

Among these, the above $R^1$ is preferably $(C_1-C_4)$alkyl, more preferably methyl or ethyl.

$R^2$ is hydrogen; halogen, e.g., fluorine, chlorine, bromine, or iodine; or alkyl which may be substituted. The alkyl for $R^2$, which may be substituted, may be selected from the same groups mentioned for the above $R^1$.

Among these, $R^2$ is preferably hydrogen or halogen.

$Q^1$ is hydrogen; halogen, e.g., fluorine, chlorine, bromine, or iodine; cyano; hydroxyl; carboxyl; or a group represented by $-X^1R^3$.

In the group represented by $-X^1R^3$ of the above $Q^1$, $X^1$ is a single bond, -O-, $-SO_n-$ (n represents an integer of 0 to 2), $-OSO_n-$ (n has the same meaning as described above), -CO-, $-CO_2-$, $-OCO_2-$, or -OC(O)-, and is preferably a single bond, -O-, -S-, or - $SO_2$-, and is particularly preferably -O-.

In the group represented by $-X^1R^3$ of the above $Q^1$, $R^3$ is alkyl which may be substituted, alkenyl which may be substituted, alkynyl which may be substituted, amino which may be substituted, aryl which may be substituted, or a heterocyclic group which may be substituted. The heterocycle of the heterocyclic group is a 5- or 6-membered heterocycle containing one or more hetero atoms selected from oxygen, sulfur, or nitrogen and examples thereof include oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazol, tetrahydropyran, pyridine, pyrimidinine, pyridazine, morpholine, thiomorpholine, piperazine, piperidine, and oxazine.

The substituents for the above alkyl, alkenyl, alkynyl, amino, aryl, and heterocyclic group are not particularly limited as far as the resulting compounds exhibit a herbicidal activity. Examples thereof include halogen, alkoxy, haloalkoxy, alkylthio, haloalkylthio, alkylsulfinyl, haloalkylsulfinyl, alkylsulfonyl, haloalkylsulfonyl, alkyl, aryl, and nitro.

Moreover, the above alkyl, alkenyl, and alkynyl have preferably from 1 to 10 carbon atoms, more preferably from 1 to 6 carbon atoms. The above amino, aryl, and heterocyclic group have preferably 15 carbon atoms or less, more preferably 12 carbon atoms or less, particularly preferably 10 carbon atoms or less.

$R^3$ is preferably halogen, alkyl which may be substituted, alkenyl which may be substituted, alkynyl which may be substituted, aryl which may be substituted, or a heterocyclic group which may be substituted.

Preferred examples of the above $R^3$ include $(C_1-C_{10})$alkyl, e.g., methyl, ethyl, propyl, isopropyl, butyl, isobutyl, sec-butyl, tert-butyl, pentyl, isopentyl, hexyl, isohexyl, heptyl, octyl, nonyl, or decyl; cyclo$(C_3-C_6)$alkyl, e.g., cyclopropyl, cyclobutyl, cyclopentyl, or cyclohexyl;

halo$(C_1-C_{10})$alkyl, e.g., chloromethyl, 2-chloroethyl, 2-fluoroethyl, 2,2-difluoroethyl, 2,2,2-trifluoroethyl, 2,2,2-trichloroethyl, 3-chloropropyl, 3-bromopropyl, 3,3,3-trifluoropropyl, 2,2,3,3-tetrafluoropropyl, 2,2,3,3,3-pentafluoropropyl, 1-methyl-2,2,2-trifluoroethyl, 2,2,2-trifluoro-1-(trifluoromethyl)ethyl, 1-methyl-2,2,3,3,3-pentafluoropropyl, 3-chloropropyl, 3-bromopropyl, 4-chlorobutyl, 4,4,4-trifluorobutyl, 5,5,5-trifluoropentyl, 6,6,6-trifluorohexyl, 7-fluoroheptyl, 8-chlorooctyl, 9-fluorononyl, or 10-fluorodecyl;

$(C_1-C_6)$alkoxy$(C_1-C_6)$alkyl, e.g., methoxymethyl, ethoxymethyl, propoxymethyl, isopropoxymethyl, butoxymethyl, pentyloxymethyl, hexyloxymethyl, 2-methoxyethyl, 2-methoxy-1-methylethyl, 2-ethoxyethyl, 2-propoxyethyl, 2-isopropoxyethyl, 2-methoxypropyl, 3-methoxypropyl, 4-methoxybutyl, 5-methoxypentyl, or 6-methoxyhexyl;

halo$(C_1-C_6)$alkoxy$(C_1-C_6)$alkyl, e.g., (2,2,2-trifluoroethoxy)methyl, 2-(2,2,2-trifluoroethoxy)ethyl, 3-(2,2,2-trifluoroethoxy)propyl, 4-(2,2,2-trifluoroethoxy)butyl, 5-(2,2,2-trifluoroethoxy)pentyl, 6-(2,2,2-trifluoroethoxy)hexyl, 1-methyl-2-(2,2,2-trifluoroethoxy)ethyl, (trifluoromethoxy)ethyl, (3-chloropropoxy)ethyl, (4-chlorobutoxy)ethyl, (5-chloropentyloxy)ethyl, or (6-fluorohexyloxy)methyl;

(amino)hydroxy$(C_2-C_6)$alkyl, e.g., 2-amino-3-hydroxy-2-methylpropyl;

$(C_1-C_4)$alkyl$(C_3-C_6)$cycloalkyl, e.g., 1-methyl-cyclopropyl, 2-methylcyclopropyl, 2-ethyl-cyclopropyl, 2-propyl-cyclopropyl, 2-butyl-cyclopropyl, 2,2-dimethyl-cyclopropyl, 2-methyl-cyclobutyl, 2-methyl-cyclopentyl, or 4-tert-butylcyclohexyl;

cyclo$(C_3-C_6)$alkyl$(C_1-C_4)$alkyl, e.g., cyclopropylmethyl, 2-(cyclopropyl)ethyl, 3-(cyclopropyl)propyl, 4-(cyclopropyl)butyl, cyclobutylmethyl, cyclopentylmethyl, or cyclohexylmethyl;

$(C_1-C_4)$alkylthio$(C_1-C_4)$alkyl, e.g., methylthiomethyl, ethylthiomethyl, propylthiomethyl, butylthiomethyl, 1-(methylthio)ethyl, 2-(methylthio)ethyl, 3-(methylthio)propyl, or 4-(methylthio)butyl;

$(C_1-C_4)$alkylsulfinyl$(C_1-C_4)$alkyl, e.g., methylsulfinylmethyl, ethylsulfinylmethyl, propylsulfinylmethyl, butylsulfinylmethyl, 1-(methylsulfinyl)ethyl, 2-(methylsulfinyl)ethyl, 3-(methylsulfinyl)propyl, or 4-(methylsulfinyl)butyl;

$(C_1-C_4)$alkylsulfonyl$(C_1-C_4)$alkyl, e.g., methylsulfonylmethyl, ethylsulfonylmethyl, propylsulfonylmethyl, butylsulfonylmethyl, 1-(methylsulfonyl)ethyl, 2-(methylsulfonyl)ethyl, 3-(methylsulfonyl)propyl, or 4-(methylsulfonyl)butyl;

$(C_2-C_6)$alkenyl, e.g., vinyl, allyl, methallyl, crotyl, 2-butenyl, 3-butenyl, 2-methyl-2-butenyl, 3-methyl-2-butenyl, 4-pentenyl, or 5-hexenyl;

halo$(C_2-C_6)$alkenyl, e.g., 2-chloroallyl, 3-chloroallyl, or 4-chloro-2-butenyl;

$(C_2-C_6)$alkynyl, e.g., ethynyl, propargyl, $\alpha$-methylpropargyl, 2-butynyl, 3-butynyl, 4-pentynyl, or 5-hexynyl;

halo$(C_2-C_6)$alkynyl, e.g., 3-chloropropargyl, 4-chloro-2-butynyl, or 5-chloro-3-pentynyl;

phenyl and substituted aryl, e.g., 2-methylphenyl, 3-methylphenyl, 4-methylphenyl, 2-methoxyphenyl, 3-methoxyphenyl, 4-methoxyphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2,3-difluorophenyl, 2,4-difluorophenyl, 2,5-difluorophenyl, 2,6-difluorophenyl, 3,4-difluorophenyl, 3,5-difluorophenyl, 2,3-dichlorophenyl, 2,4-dichlorophenyl, 2,5-dichlorophenyl, 2,6-dichlorophenyl, 3,4-dichlorophenyl, 3,5-dichlorophenyl, 2,3-dibromophenyl, 2,4-dibromophenyl, 2,5-dibromophenyl, 2,6-dibromophenyl, 3,4-dibromophenyl, 3,5-dibromophenyl, 2-(difluoromethyl)phenyl, 3-(difluoromethyl)phenyl, 4-(difluoromethyl)phenyl, 2-(difluoromethoxy)phenyl, 3-(difluoromethoxy)phenyl, 4-(difluoromethoxy)phenyl, 2-(difluoromethylthio)phenyl, 3-(difluoromethylthio)phenyl, 4-(difluoromethylthio)phenyl, 2-(trifluoromethyl)phenyl, 3-(trifluoromethyl)phenyl, 4-(trifluoromethyl)phenyl, 2-(trifluoromethoxy)phenyl, 3-(trifluoromethoxy)phenyl, 4-(trifluoromethoxy)phenyl, 2-(trifluoromethylthio)phenyl, 3-(trifluoromethylthio)phenyl, 4-(trifluoromethylthio)phenyl, 2,4-bis(trifluoromethyl)phenyl, 2,5-bis(trifluoromethyl)phenyl, 3,5-bis(trifluoromethyl)phenyl, 2,4-bis(trifluoromethoxy)phenyl, 2,5-bis(trifluoromethoxy)phenyl, 3,5-bis(trifluoromethoxy)phenyl, 2,4-bis(trifluoromethylthio)phenyl, 2,5-bis(trifluoromethylthio)phenyl, 3,5-bis(trifluoromethylthio)phenyl, 2-fluoro-3-(trifluoromethyl)phenyl, 4-fluoro-3-(trifluoromethyl)phenyl, 5-fluoro-3-(trifluoromethyl)phenyl, 6-fluoro-3-(trifluoromethyl)phenyl, 2-chloro-3-(trifluoromethyl)phenyl, 4-chloro-3-(trifluoromethyl)phenyl, 5-chloro-3-(trifluoromethyl)phenyl, 6-chloro-3-(trifluoromethyl)phenyl, 2-bromo-3-(trifluoromethyl)phenyl, 4-bromo-3-(trifluoromethyl)phenyl, 5-bromo-3-(trifluoromethyl)phenyl, 6-bromo-3-(trifluoromethyl)phenyl, 2-methyl-3-(trifluoromethyl)phenyl, 4-methyl-3-(trifluoromethyl)phenyl, 5-methyl-3-(trifluoromethyl)phenyl, 6-methyl-3-(trifluoromethyl)phenyl, 2-methoxy-3-(trifluoromethyl)phenyl, 4-methoxy-3-(trifluoromethyl)phenyl, 5-methoxy-3-(trifluoromethyl)phenyl, 6-methoxy-3-(trifluoromethyl)phenyl, 4-ethoxy-3-(trifluoromethyl)phenyl, 4-n-propoxy-3-(trifluoromethyl)phenyl, 4-isopropoxy-3-(trifluoromethyl)phenyl, 2-(2,2,2-trifluoroethyl)phenyl, 3-(2,2,2-trifluoroethyl)phenyl, 4-(2,2,2-trifluoroethyl)phenyl, 2,6-dichloro-4-(trifluoromethyl)phenyl, 2-nitrophenyl, 3-nitrophenyl, or 4-nitrophenyl;

amino and ($C_1$-$C_{12}$)amino, e.g., methylamino, dimethylamino, ethylamino, diethylamino, methylethylamino, propylamino, isopropylamino, butylamino, phenylamino, 2-methylphenylamino, 3-methylphenylamino, 4-methylphenylamino, 2-fluorophenylamino, 3-fluorophenylamino, 4-fluorophenylamino, 2-(trifluoromethyl)phenylamino, 3-(trifluoromethyl)phenylamino, 4-(trifluoromethyl)phenylamino, 2-(trifluoromethoxy)phenylamino, 3-(trifluoromethoxy)phenylamino, 4-(trifluoromethoxy)phenylamino, 2-chlorophenylamino, 3-chlorophenylamino, 4-chlorophenylamino, 2-methoxyphenylamino, 3-methoxyphenylamino, 4-methoxyphenylamino, 2-nitrophenylamino, 3-nitrophenylamino, or 4-nitrophenylamino;

a heterocyclic group, e.g., oxiranyl, oxetanyl, tetrahydrofuryl, furyl, thienyl, pyrrolyl, pyrrolidinyl, oxazolidyl, thiazolyl, imidazolyl, isoxazolyl, isothiazolidyl, pyrazolidyl, tetrahydropyranyl, pyridyl, piperidyl, pyrimidinyl, pyridazinyl, morpholinyl, piperazinyl, triazolidyl, 1-pyrrolidinyl, 1-pyrrolyl, 2-isooxazolidinyl, 1-pyrazolyl, 1-imidazolyl, 1-triazolyl, 1-tetrazolyl, 1-piperidyl, 4-morpholinyl, 4-thiomorpholinyl, 2-oxazin-2-yl, or 1-piperazinyl; and

the above described heterocyclic group substituted with halogen, e.g., fluorine, chlorine, bromine, or iodine; ($C_1$-$C_6$)alkyl, e.g., methyl, ethyl, propyl, isopropyl, butyl, isobutyl, sec-butyl, tert-butyl, pentyl, or hexyl; cyclo($C_3$-$C_6$) alkyl, e.g., cyclopropyl, cyclobutyl, cyclopentyl, or cyclohexyl; ($C_1$-$C_4$)haloalkyl, e.g., chloromethyl, trifluoromethyl, 2-chloroethyl, 2-fluoroethyl, 2,2-difluoroethyl, 2,2,2-trifluoroethyl, 2,2,2-trichloroethyl, 3-chloropropyl, 3-bromopropyl, 3,3,3-trifluoropropyl, 2,2,3,3-tetrafluoropropyl, 2,2,3,3,3-pentafluoropropyl, 1-methyl-2,2,2-trifluoroethyl, 2,2,2-trifluoro-1-(trifluoromethyl)ethyl, 1-methyl-2,2,3,3,3-pentafluoropropyl, 4-chlorobutyl, or 4,4,4-trifluorobutyl; ($C_1$-$C_6$) alkoxy, e.g., methoxy, ethoxy, propoxy, isopropoxy, butoxy, or tert-butoxy.

As the above $Q^1$, preferred is the case that $Q^1$ is a group represented by $OR^3$. Preferred examples thereof include ($C_1$-$C_6$)alkoxy, e.g., methoxy, ethoxy, propoxy, isopropoxy, butoxy, tert-butoxy, pentyloxy, or hexyloxy;

cyclo($C_3$-$C_6$)alkoxy, e.g., cyclopropoxy, cyclobutoxy, cyclopentyloxy, or cyclohexyloxy;

halo($C_1$-$C_6$)alkoxy, e.g., chloromethoxy, fluoromethoxy, trifluoromethoxy, 2-chloroethoxy, 2-fluoroethoxy, 2,2-difluoroethoxy, 2,2,2-trifluoroethoxy, 2,2,2-trichloroethoxy, 3-chloropropoxy, 3-bromopropoxy, 3,3,3-trifluoropropoxy, 2,2,3,3-tetrafluoropropoxy, 2,2,3,3,3-pentafluoropropoxy, 1-methyl-2,2,2-trifluoroethoxy, 2,2,2-trifluoro-1-(trifluoromethyl)ethoxy, 1-methyl-2,2,3,3,3-pentafluoropropoxy, 3-chloropropoxy, 3-bromopropoxy, 4-chlorobutoxy, 4,4,4-trifluorobutoxy, 5,5,5-trifluoropentyloxy, or 6,6,6-trifluorohexyloxy;

($C_2$-$C_6$)alkenyloxy, e.g., vinyloxy, allyloxy, methallyloxy, crotyloxy, 2-butenyloxy, 3-butenyloxy, 2-methyl-2-butenyloxy, or 3-methyl-2-butenyloxy;

halo($C_2$-$C_4$)alkenyloxy, e.g., 2-chloroallyloxy, 3-chloroallyloxy, or 4-chloro-2-butenyloxy;

halo($C_2$-$C_4$)alkynyloxy, e.g., ethynyloxy, propargyloxy, $\alpha$-methylpropargyloxy, 2-butynyloxy, or 3-butynyloxy;

halo($C_2$-$C_4$)alkynyloxy, e.g., 3-chloropropargyloxy or 4-chloro-2-butynyloxy;

heterocyclic oxy, e.g., tetrahydrofuryloxy, furyloxy, imidazolyloxy, isoxazolyloxy, isothiazolidyloxy, pyrazolidyloxy, triazolidyloxy, 2-isoxazolidinyloxy, 1-pyrazolyloxy, or 1-imidazolyloxy; and phenoxy or benzyloxy, wherein the heterocyclic oxy, phenoxy, and benzyloxy may be substituted with a substituent selected from the group consisting of halogen, ($C_1$-$C_3$)alkyl, ($C_3$-$C_6$)cycloalkyl, ($C_1$-$C_2$)haloalkyl, and ($C_1$-$C_3$)alkoxy. More preferred is ($C_1$-$C_3$)alkoxy or haloalkoxy.

$Q^2$ is hydrogen; halogen, e.g., fluorine, chlorine, bromine, or iodine; hydroxyl; or a group represented by -$X^2R^4$.

In the group represented by $-X^2R^4$ of the above $Q^2$, $X^2$ may be a group selected from the same groups as those mentioned above for the above $X^1$, and $R^4$ may be a group selected from the same groups as those mentioned above for the above $R^3$.

Among these, preferred examples of $Q^2$ include:

1) alkylthio, e.g., methylthio, ethylthio, propylthio, isopropylthio, butylthio, isobutylthio, sec-butylthio, tert-butylthio, pentylthio, isopentylthio, or hexylthio; alkylsulfinyl, e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, or hexylsulfinyl; or alkylsulfonyl, e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, or hexylsulfonyl;
2) aryl, aryloxy, arylthio, arylsulfinyl, or arylsulfonyl substituted with at least one substituent which are the same or different and is selected from the group consisting of fluorine-containing alkyl, e.g., fluoromethyl, difluoromethyl, trifluoromethyl, 2-fluoroethyl, 2,2-difluoroethyl, 2,2,2-trifluoroethyl, 3-fluoropropyl, 3,3,3-trifluoropropyl, 2,2,3,3-tetrafluoropropyl, 2,2,3,3,3-pentafluoropropyl, 1-methyl-2,2,2-trifluoroethyl, 2,2,2-trifluoro-1-(trifluoromethyl)ethyl, or 1-methyl-2,2,3,3,3-pentafluoropropyl; fluorine-containing alkoxy, e.g., fluoromethoxy, difluoromethoxy, trifluoromethoxy, 2-fluoroethoxy, 2,2-difluoroethoxy, 2,2,2-trifluoroethoxy, 3-fluoropropoxy, 3,3,3-trifluoropropoxy, 2,2,3,3-tetrafluoropropoxy, 2,2,3,3,3-pentafluoropropoxy, 1-methyl-2,2,2-trifluoroethoxy, 2,2,2-trifluoro-1-(trifluoromethyl)ethoxy, or 1-methyl-2,2,3,3,3-pentafluoropropoxy; and fluorine-containing alkylthio, e.g., fluoromethylthio, difluoromethylthio, trifluoromethylthio, 2-fluoroethylthio, 2,2-difluoroethylthio, 2,2,2-trifluoroethylthio, 3-fluoropropylthio, 3,3,3-trifluoropropylthio, 2,2,3,3-tetrafluoropropylthio, 2,2,3,3,3-pentafluoropropylthio, 1-methyl-2,2,2-trifluoroethylthio, 2,2,2-trifluoro-1-(trifluoromethyl)ethylthio, or 1-methyl-2,2,3,3,3-pentafluoropropylthio; and
3) heterocyclic group or heterocyclic oxy which may be substituted with substituent selected from the group consisting of the same fluorine-containing alkyl, fluorine-containing alkoxy, and fluorine-containing alkylthio as above. The heterocycle is 5- or 6-membered heterocycle having one or more hetero atoms selected from oxygen, sulfur, or nitrogen as exemplified for the above $Q^1$.

The above alkylthio, alkylsulfinyl, and alkylsulfonyl, and the fluorine-containing alkyl, fluorine-containing alkoxy, and fluorine-containing alkylthio which are substituents for the above aryl and the like preferably have from 1 to 6 carbon atoms, more preferably from 1 to 4 carbon atoms.

Preferred specific examples of the aryl, aryloxy, arylthio, arylsulfinyl, or arylsulfonyl substituted with at least one substituent selected from the group consisting of the above fluorine-containing alkyl, fluorine-containing alkoxy, and fluorine-containing alkylthio include 2-(fluoromethyl)phenyl, 2-(fluoromethoxy)phenyl, 2-(fluoromethylthio)phenyl, 3-(fluoromethyl)phenyl, 3-(fluoromethoxy)phenyl, 3-(fluoromethylthio)phenyl, 4-(fluoromethyl)phenyl, 4-(fluoromethoxy)phenyl, 4-(fluoromethylthio)phenyl, 2-(difluoromethyl)phenyl, 2-(difluoromethoxy)phenyl, 2-(difluoromethylthio)phenyl, 3-(difluoromethyl)phenyl, 3-(difluoromethoxy)phenyl, 3-(difluoromethylthio)phenyl, 4-(difluoromethyl)phenyl, 4-(difluoromethoxy)phenyl, 4-(difluoromethylthio)phenyl, 2-(trifluoromethyl)phenyl, 2-(trifluoromethoxy)phenyl, 2-(trifluoromethylthio)phenyl, 3-(trifluoromethyl)phenyl, 3-(trifluoromethoxy)phenyl, 3-(trifluoromethylthio)phenyl, 4-(trifluoromethyl)phenyl, 4-(trifluoromethoxy)phenyl, 4-(trifluoromethylthio)phenyl, 2-(2-fluoroethyl)phenyl, 2-(2-fluoroethoxy)phenyl, 2-(2-fluoroethylthio)phenyl, 3-(2-fluoroethyl)phenyl, 3-(2-fluoroethoxy)phenyl, 3-(2-fluoroethylthio)phenyl, 4-(2-fluoroethyl)phenyl, 4-(2-fluoroethoxy)phenyl, 4-(2-fluoroethylthio)phenyl, 2-(2,2-difluoroethyl)phenyl, 2-(2,2-difluoroethoxy)phenyl, 2-(2,2-difluoroethylthio)phenyl, 3-(2,2-difluoroethyl)phenyl, 3-(2,2-difluoroethoxy)phenyl, 3-(2,2-difluoroethylthio)phenyl, 4-(2,2-difluoroethyl)phenyl, 4-(2,2-difluoroethoxy)phenyl, 4-(2,2-difluoroethylthio)phenyl, 2-(2,2,2-trifluoroethyl)phenyl, 2-(2,2,2-trifluoroethoxy)phenyl, 2-(2,2,2-tridifluoroethylthio)phenyl, 3-(2,2,2-trifluoroethyl)phenyl, 3-(2,2,2-trifluoroethoxy)phenyl, 3-(2,2,2-tridifluoroethylthio)phenyl, 4-(2,2,2-trifluoroethyl)phenyl, 4-(2,2,2-trifluoroethoxy)phenyl, 4-(2,2,2-tridifluoroethylthio)phenyl, and phenoxys and phenylthios having the same substituents as those of the phenyls exemplified in the above. Among these, preferred are 3-(fluoromethyl)phenyl, 3-(fluoromethoxy)phenyl, 3-(fluoromethylthio)phenyl, 3-(difluoromethyl)phenyl, 3-(difluoromethoxy)phenyl, 3-(difluoromethylthio)phenyl, 3-(trifluoromethyl)phenyl, 3-(trifluoromethoxy)phenyl, 3-(trifluoromethylthio)phenyl, 3-(2-fluoroethyl)phenyl, 3-(2-fluoroethoxy)phenyl, 3-(2-fluoroethylthio)phenyl, 3-(2,2-difluoroethyl)phenyl, 3-(2,2-difluoroethoxy)phenyl, 3-(2,2-difluoroethylthio)phenyl, 3-(2,2,2-trifluoroethyl)phenyl, 3-(2,2,2-trifluoroethoxy)phenyl, 3-(2,2,2-trifluoroethylthio)phenyl, and phenoxys and phenylthios having the same substituents as those of the phenyls exemplified in the above.

The heterocycle for the heterocyclic group or heterocyclic oxy which may be substituted with a substituent selected from the group consisting of the above fluorine-containing alkyl group, fluorine-containing alkoxy, and fluorine-containing alkylthio is preferably a nitrogen-containing heterocycle which may be substituted with the substituent, particularly preferably a nitrogen-containing 5- or 6-membered heterocycle.

Preferred specific examples of the heterocyclic group or heterocyclic oxy which may be substituted with a substituent selected from the group consisting of the fluorine-containing alkyl, fluorine-containing alkoxy, and fluorine-containing alkylthio include 3-(trifluoromethyl)pyrazol-1-yl, 3-(trifluoromethoxy)pyrazol-1-yl, 3-(trifluoromethylthio)pyrazol-1-yl, 1-[3-(trifluoromethylthio)]pyrazolyloxy, 3-(1,1,2,2,2-pentafluoroethyl)pyrazol-1-yl, 3-(1,1,2,2,2-pentafluoroethoxy)pyrazol-1-yl, 3-(1,1,2,2,2-pentafluoroethylthio)pyrazol-1-yl, 4-(trifluoromethyl)pyrazol-1-yl, 4-(trifluoromethoxy)pyrazol-1-yl, 4-(trifluoromethylthio)pyrazol-1-yl, 1-[3-(1,1,2,2,2-pentafluoroethyl)]pyrazolyloxy, 1-[3-(1,1,2,2,2-pentafluoroethoxy)]pyrazolyloxy, 1-[3-(1,1,2,2,2-pentafluoroethylthio)]pyrazolyloxy, 1-[4-(trifluoromethyl)]pyrazolyloxy, 1-[3-(trifluoromethyl)]pyrazolyloxy, 1-[3-(trifluoromethoxy)]pyrazolyloxy, 1-[4-(trifluoromethoxy)]pyrazolyloxy, 1-[4-(trifluoromethylthio)]pyrazolyloxy, 2-(trifluoromethyl)imidazol-1-yl, 2-(trifluoromethoxy)imidazol-1-yl, 4-(trifluoromethyl)imidazol-1-yl, 4-(trifluoromethoxy)imidazol-1-yl, 4-(trifluoromethylthio)imidazol-1-yl, 2-(trifluoromethylthio)imidazol-1-yl, 4-(1,1,2,2,2-pentafluoroethyl)imidazol-1-yl,4-(1,1,2,2,2-pentafluoroethoxy)imidazol-1-yl,4-(1,1,2,2,2-pentafluoroethylthio)imidazol-1-yl, 1-[2-(trifluoromethyl)]imidazolyloxy, 1-[2-(trifluoromethoxy)]imidazolyloxy, 1-[2-(trifluoromethylthio)]imidazolyloxy, 1-[4-(trifluoromethyl)]imidazolyloxy, 1-[4-(trifluoromethoxy)]imidazolyloxy, and 1-[4-(trifluoromethylthio)]imidazolyloxy. Among these, preferred are 3-(trifluoromethyl)pyrazol-1-yl, 3-(trifluoromethoxy)pyrazol-1-yl, 3-(trifluoromethylthio)pyrazol-1-yl, 3-(1,1,2,2,2-pentafluoroethyl)pyrazol-1-yl, 3-(1,1,2,2,2-pentafluoroethoxy)pyrazol-1-yl, 3-(1,1,2,2,2-pentafluoroethylthio)pyrazol-1-yl, 2-(trifluoromethyl)imidazol-1-yl, 2-(trifluoromethoxy)imidazol-1-yl, 2-(trifluoromethylthio)imidazol-1-yl, 4-(trifluoromethyl)imidazol-1-yl, 4-(trifluoromethoxy)imidazol-1-yl, and 4-(trifluoromethylthio)imidazol-1-yl.

2. Novel compounds represented by formula (I)

Among the compounds represented by the above formula (I) to be used as the herbicide of present invention, the compounds to be described below are novel compounds and are more preferable as the herbicide.

In the above formula (I),

(1) When A is A1, $R^1$ and $R^2$ have the same meanings as described above, and $Q^1$ is $-OR^3$ wherein $R^3$ has the same meaning as described above, $Q^2$ is hydrogen; halogen, e.g., fluorine, chlorine, or bromine; hydroxyl, or $-X^2R^4$ wherein $X^2$ and $R^4$ have the same meanings as described above, provided that $R^4$ is not amino, mono($C_1$-$C_6$)alkylamino, or monohalo($C_1$-$C_6$)alkylamino in the case that $X^2$ represents a single bond, and $R^4$ is not ($C_1$-$C_{10}$)alkyl in the case that $X^2$ is -O-.

(2) When A is A1, $R^1$ and $R^2$ have the same meanings as described above, and $Q^1$ is hydrogen, halogen, e.g., fluorine, chlorine, or bromine; hydroxyl, or $-X^1R^3$ wherein $X^1$ is a single bond, $-SO_n-$ (n has the same meaning as described above), - $OSO_n-$ (n has the same meaning as described above), -CO-, $-CO_2-$, $-OCO_2-$, or -OC(O)-and $R^3$ has the same meaning as described above, $Q^2$ is aryl, aryloxy, arylthio, arylsulfinyl, or arylsulfonyl substituted with at least one substituent selected from the group consisting of the fluorine-containing alkyl, fluorine-containing alkoxy, and fluorine-containing alkylthio, provided that $Q^2$ is not substituted aryloxy which is substituted with at least one fluorine-containing alkyl in the case that $Q^1$ represents ($C_1$-$C_6$)alkyl wherein $X^1$ represents a single bond and $R^3$ represents ($C_1$-$C_6$)alkyl.

(3) When A is A2, $R^1$ and $R^2$ have the same meanings as described above, and $Q^1$ is $-OR^3$, $Q^2$ is hydrogen; halogen, e.g., fluorine, chlorine, or bromine; hydroxyl, or - $X^2R^4$ wherein $X^2$ is a single bond, $-SO_n-$ (n has the same meaning as described above), $-OSO_n-$ (n has the same meaning as described above), -CO-, $-CO_2-$, $-OCO_2-$, or -OC(O)-and $R^4$ is alkyl which may be substituted, alkenyl which may be substituted, amino which may be substituted, or aryl which may be substituted, provided that $R^4$ is not amino which may be substituted or aryl which may be substituted in the case that $X^2$ represents a single bond.

(4) When A is A2, $R^1$ and $R^2$ have the same meanings as described above, and $Q^1$ is halogen, e.g., fluorine, chlorine, or bromine; hydroxyl, or $-X^1R^3$ wherein $X^1$ is a single bond, $-SO_n-$ (n has the same meaning as described above), $-OSO_n-$ (n has the same meaning as described above), -CO-, $-CO_2-$, $-OCO_2-$, or -OC(O)-, and $R^3$ has the same meaning as described above, $Q^2$ is aryl, aryloxy, arylthio, arylsulfinyl, or arylsulfonyl substituted with at least one substituent selected from the group consisting of the fluorine-containing alkyl, fluorine-containing alkoxy, and fluorine-containing alkylthio.

The above $Q^2$ is preferably aryl, aryloxy, arylthio, arylsulfinyl, or arylsulfonyl substituted with at least one substituent selected from the group consisting of the fluorine-containing alkyl, fluorine-containing alkoxy, and fluorine-containing alkylthio.

Moreover, the compound wherein A is A1 and $Q^1$ is halogen or hydroxyl itself has a herbicidal activity and further, is also useful as an intermediate for synthesizing other compounds to be used as the herbicide of the present invention.

3. Process for producing substituted thienopyrimidine derivatives and intermediates thereof

The compounds represented by the above formula (I) can be produced according to known methods, similar methods thereof, or combinations thereof. In particular, novel compounds are preferably produced according to

Methods 1 to 8 shown below.

Production Method 1

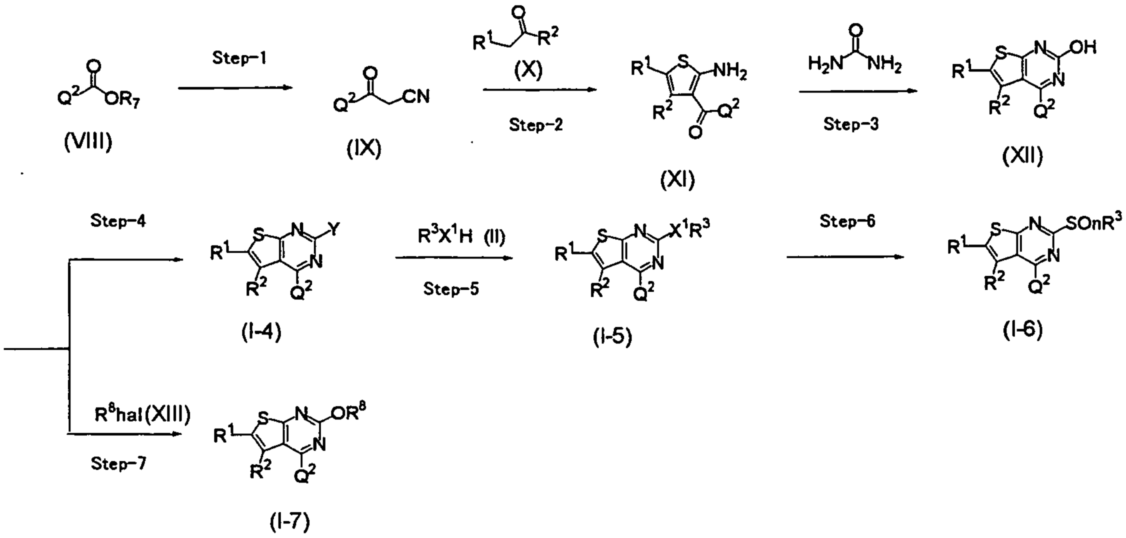


wherein $R^1$, $R^2$, $R^3$, $Q^2$, n, Y, and $X^1$ have the same meanings as described above, $R^7$ represents methyl, ethyl, or propyl, $R^8$ represents alkylsulfonyl, arylsulfonyl, acyl, or carbamoyl which may be substituted, and hal represents halogen.

Step 1 is a step of reacting an ester derivative represented by formula (VIII) with acetonitrile to produce acylacetonitrile derivative represented by formula (IX) Therein, ethyl 3-(trifluoromethoxy)benzoate is a novel compound and among the acylacetonitrile derivatives represented by formula (IX), a substituted benzoylacetonitrile derivative represented by the following formula (VII):

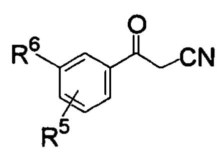

(VII)

wherein $R^5$ represents hydrogen, halogen, or alkyl which may be substituted, $R^6$ represents fluorine-containing alkyl, fluorine-containing alkoxy, and fluorine-containing alkylthio,
is a novel compound.

The reaction of this step is preferably carried out in the presence of a base in view of the yield. As the base, an alkali metal base, e.g., sodium hydride, potassium hydride, lithium amide, sodium amide, lithium diisopropylamide (LDA), n-butyllithium, sec-butyllithium, t-butyllithium, lithium hexamethyldisilazide, or potassium t-butoxide may be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as a nitrile solvent, e.g., acetonitrile; an aromatic hydrocarbon solvent, e.g., benzene, toluene, or xylene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; liquid ammonia; or a mixed solvent thereof, can be used.

The objective compound can be obtained in good yields by carrying out the reaction at a temperature suitably selected from the range of -78°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used

in the next reaction without isolation.

Step 2 is a step of reacting an acylacetonitrile derivative represented by formula (IX), a compound represented by formula (X), and sulfur to produce a 2-aminothiophene derivative represented by formula (XI). Among the 2-aminothiophene derivatives represented by formula (XI), a 2-amino-3-(substituted benzoyl)thiophene derivative represented by the following formula (V):

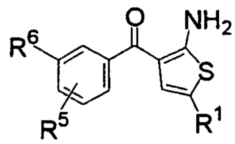

(V)

wherein $R^1$, $R^5$, and $R^6$ have the same meanings as described above, is a novel compound.

This step can be carried out in accordance with the method described in *J. Med Chem.*, Vol. 16, p. 214 (1973) and it is preferable to carry out the reaction in the presence of a base in view of the yield. As the base, an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole; or an alkali metal base, e.g., sodium hydride, potassium hydride, lithium amide, sodium amide, lithium diisopropylamide (LDA), butyllithium, t-butyllithium, lithium hexamethyldisilazide, sodium methoxide, sodium ethoxide, or potassium t-butoxide can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as an amide solvent, e.g., N,N-dimethylformamide (DMF), N,N-dimethylacetamide (DMAC), or N-methylpyrrolidone (NMP); an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide; an alcoholic solvent, e.g., methanol, ethanol, or isopropanol (IPA); or a mixed solvent thereof, can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 3 is a step of reacting the 2-aminothiophene derivative represented by formula (XI) with urea to produce a 2-hydroxythieno[2,3-d]pyrimidine derivative represented by formula (XII).

This step can be carried out in accordance with the method described in *Chem. Pharm. Bull.,* Vol. 28, p. 3172 (1980).

The reaction can be carried out in a solvent or without solvent and any solvent harmless to the reaction can be used. As the solvent, any solvent harmless to the reaction, such as an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); an alcoholic solvent, e.g., methanol, ethanol, or isopropanol (IPA); or a mixed solvent thereof, can be used.

The objective compound can be obtained in good yields by carrying out the reaction at a temperature suitably selected from the range of room temperature to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 4 is a step of halogenating the hydroxyl of the 2-hydroxythieno[2,3-d]pyrimidine derivative represented by formula (XII) with a halogenating agent to produce a thieno[2,3-d]pyrimidine derivative represented by formula (I-4).

This step can be also carried out in accordance with the method described in *Chem. Pharm. Bull.,* Vol. 28, p. 3172 (1980). That is, as the halogenating agent to be used in the step, use can be made of a halogenating agent, e.

g., sulfuryl chloride, thionyl chloride, phosphorus oxychloride, phosgene, phosphorus trichloride, phosphorus pentachloride, or phosphorus tribromide. At that time, the objective compound can be obtained in good yields by using, as a catalyst for the halogenation reaction, N,N-dimethylformamide (DMF), N,N-dimethylacetamide, N,N-dimethylaniline, or N,N-diethylaniline as well as an alkylamine, e.g., triethylamine, diisopropylethylamine, or tributylamine. The amount to be used may be from about 0.1 to about 2 equivalents to the substrate.

The reaction can be also carried out in a solvent and any solvent harmless to the reaction can be used. As the solvent, water; an aromatic hydrocarbon solvent, e.g., chlorobenzene or dichlorobenzene; a halogenated hydrocarbon solvent, e.g., chloroform, dichloromethane, or carbon tetrachloride; or a mixed solvent thereof can be used.

The objective compound can be obtained in good yields by carrying out the reaction at a temperature suitably selected from the range of -20°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 5 is a step of reacting an thieno[2,3-d]pyrimidine derivative represented by formula (I-4) with the compound represented by formula (II) wherein $R^3$ and $X^1$ have the same meanings as described above to produce a thieno [2,3-d]pyrimidine derivative represented by formula (I-5).

It is preferable to carry out the reaction of this step in the presence of a base in view of the yield. As the base, an alkali metal base, e.g., sodium hydride, potassium hydride, lithium amide, sodium amide, lithium diisopropylamide (LDA), n-butyllithium, sec-butyllithium, t-butyllithium, trimethylsilyllithium, lithium hexamethyldisilazide, sodium carbonate, potassium carbonate, sodium hydroxide, potassium hydroxide, sodium methoxide, sodium ethoxide, or potassium t-butoxide; or an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e. g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); an alcoholic solvent, e.g., methanol, ethanol, or isopropanol (IPA); or a mixed solvent thereof, can be used.

The objective compound can be obtained in good yields by carrying out the reaction at a temperature suitably selected from the range of -78°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 6 is a step of oxidizing the compound of the thieno[2,3-d]pyrimidine derivative represented by formula (I-5), wherein $X^1$ is represented by -S-, to produce a thieno[2,3-d]pyrimidine derivative represented by formula (I-6).

This step can be also carried out by using an oxidizing agent. The oxidizing agent to be used includes an oxidizing agent, e.g., m-chloroperbenzoic acid, aqueous hydrogen peroxide, or peracetic acid.

The reaction can be also carried out in a solvent and any solvent harmless to the reaction can be used. As the solvent, water; an aromatic hydrocarbon solvent, e.g., toluene, chlorobenzene, or dichlorobenzene; a halogenated hydrocarbon solvent, e.g., chloroform, dichloromethane, or carbon tetrachloride; acetic acid; or a mixed solvent thereof can be used.

The objective compound can be obtained in good yields by carrying out the reaction at a temperature suitably selected from the range of -20°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography.

Step 7 is a step of reacting a 2-hydroxythieno[2,3-d]pyrimidine derivative represented by formula (XII) with the compound represented by formula (XIII) to produce a thieno[2,3-d]pyrimidine derivative represented by formula (I-7).

It is preferable to carry out the reaction of this step in the presence of a base or a trialkylammonium chloride, e.g., trimethylamine hydrochloride. As the base, an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo [2.2.2]octane (DABCO), or imidazole can be used. The objective compound can be obtained in good yields by using the base or the trialkylammonium chloride in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); or a mixed solvent thereof, can be used.

The objective compound can be obtained in good yields by carrying out the reaction at a temperature suitably selected from the range of -78°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography.

Production Method 2

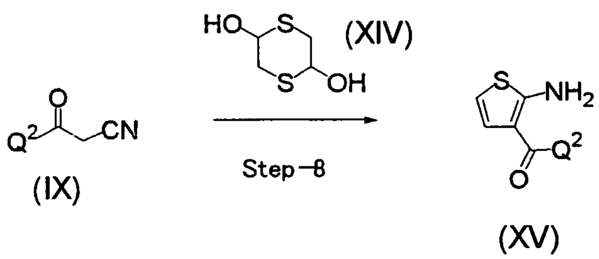

(XIV)
(IX)
(XV)

wherein $Q^2$ has the same meaning as described above.

Step 8 is a step of reacting an acylacetonitrile derivative represented by formula (IX), with 2,5-dihydroxy-1,4-dithian represented by formula (XIV) to produce a 2-aminothiophene derivative represented by formula (XV).

In this step, it is preferable to carry out the reaction in the presence of a base in view of the yield. As the base, an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole; or an alkali metal base, e.g., sodium hydride, potassium hydride, lithium amide, sodium amide, lithium diisopropylamide (LDA), butyllithium, t-butyllithium, trimethylsilyllithium, lithium hexamethyldisilazide, sodium methoxide, sodium ethoxide, or potassium t-butoxide can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); an alcoholic solvent, e.g., methanol, ethanol, or isopropanol (IPA); or a mixed solvent thereof, can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used

in the next reaction without isolation.

Production Method 3

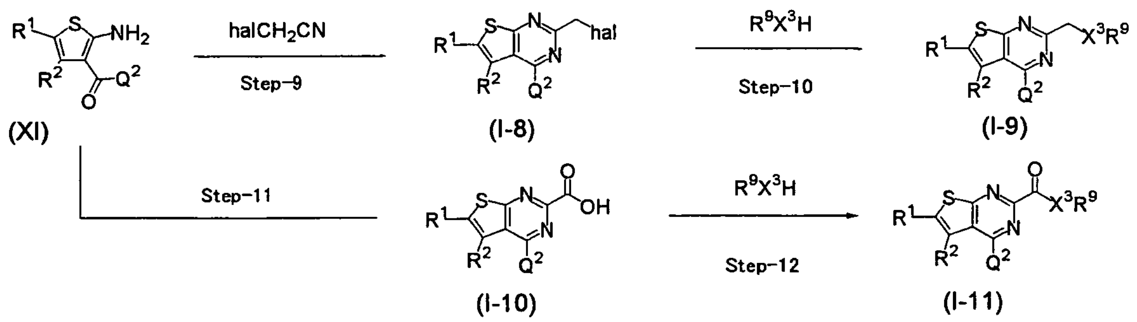


wherein $R^1$, $R^2$, hal, and $Q^2$ have the same meanings as described above, $X^3$ represents a single bond, -O-, or -S-, and $R^9$ represents an amino which may be substituted, an alkyl which may be substituted, or an aryl which may be substituted.

Step 9 is a step of reacting the aminothiophene derivative represented by formula (XI) with a haloacetonitrile represented by formula (XVI) in the presence of a Lewis acid, e.g., aluminum chloride, to produce a thieno[2,3-d] pyrimidine derivative represented by formula (I-8).

This step can be carried out in accordance with the method described in *J. Med. Chem.*, Vol. 39, No. 16, p. 5176 (1996).

The reaction can be also carried out in a solvent and any solvent harmless to the reaction can be used. As the solvent, water; an aromatic hydrocarbon solvent, e.g., chlorobenzene or dichlorobenzene; a halogenated hydrocarbon solvent, e.g., chloroform, dichloromethane, or carbon tetrachloride; or a mixed solvent thereof can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 10 is a step of reacting the thieno[2,3-d]pyrimidine derivative represented by formula (I-8) with a compound represented by formula (XVII) in a similar manner to the above-described Step 5 to produce a thieno[2,3-d] pyrimidine derivative represented by formula (I-9).

Step 11 is a step of reacting the aminothiophene derivative represented by formula (XI) with glyoxylic acid and ammonium acetate to produce a thieno[2,3-d]pyrimidine carboxylate derivative represented by formula (I-10).

This step can be carried out in accordance with the method described in *Tetrahedron,* Vol. 49, No. 31, p. 6899 (1993).

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an alcoholic solvent, e.g., methanol, ethanol, propanol, or isopropanol; an ether solvent, e.g., diethyl ether, diisopropyl ether, tetrahydrofuran (THF), dimethoxyethane (DME), or 1,4-dioxane; dimethyl sulfoxide (DMSO); or a mixed solvent thereof, can be used.

The objective compound can be obtained in good yields by carrying out the reaction at a temperature suitably selected from the range of 0°C to a reflux temperature of the solvent used. This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 12 is a step of producing an acid chloride from the thieno[2,3-d]pyrimidine carboxylate derivative represented by formula (I-10) using thionyl chloride, phosphorus oxychloride, phosphorus pentachloride, oxalyl chloride, or

the like and then reacting the product with the compound represented by formula (XVII) to produce a thieno[2,3-d]pyrimidine derivative represented by formula (I-11).

In the step of producing the thieno[2,3-d]pyrimidine derivative represented by formula (I-11) using the acid halide as a starting material, it is preferable to carry out the reaction in the presence of a base. As the base, an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); or a mixed solvent thereof, can be used.

The objective compound can be obtained in good yields by carrying out the reaction at a temperature suitably selected from the range of -78°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography.

Production Method 4

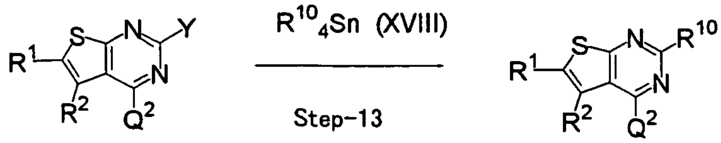


(I-4) (I-12)

wherein $R^1$, $R^2$, $Q^2$ and Y have the same meanings as described above and $R^{10}$ represents an alkyl.

Step 13 is a step of reacting the thieno[2,3-d]pyrimidine derivative represented by formula (I-4) with a tin compound represented by formula (XVIII) to produce a thieno[2,3-d]pyrimidine derivative represented by formula (I-12).

In this step, the reaction can be carried out in good yields by using a palladium catalyst, e.g., $(Ph_3P)_2PdCl_2$. The amount of the palladium catalyst to be used is suitably selected from the range of 0.0001 to 0.1 molar equivalent to the thieno[2,3-d]pyrimidine derivative represented by formula (I-4).

As the solvent, any solvent harmless to the reaction, such as water; an aromatic hydrocarbon solvent, e.g., benzene, toluene, or xylene; an ether solvent, e.g., diethyl ether, diisopropyl ether, tetrahydrofuran (THF), dimethoxyethane (DME), or 1,4-dioxane; a halogenated hydrocarbon solvent, e.g., chloroform, dichloromethane, or carbon tetrachloride; or a mixed solvent thereof, can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography.

Production Method 5

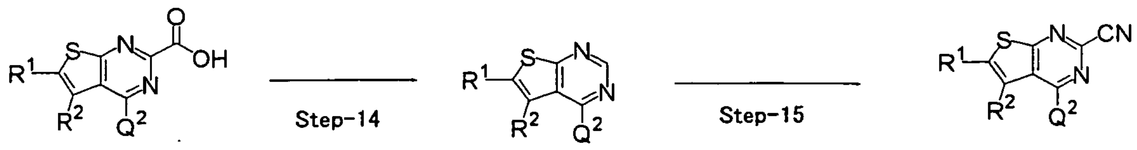

(I-10) (I-13) (I-14)

wherein $R^1$, $R^2$, and $Q^2$ have the same meanings as described above.

Step 14 is a step of decarboxylating the thieno[2,3-d]pyrimidine derivative represented by formula (I-10) to produce a thieno[2,3-d]pyrimidine derivative represented by formula (I-13).

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); or a mixed solvent thereof, can be used.

The objective compound can be obtained in good yields by carrying out the reaction at a temperature suitably selected from the range of room temperature to a reflux temperature of the solvent used.

After completion of the reaction, the objective compound can be isolated in a usual manner and, if necessary, purified by an operation, e.g., recrystallization or silica gel column chromatography. The compound may be also used in the next reaction without isolation.

Step 15 is a step of oxidizing the nitrogen atom(s) at 1-position and/or 3-position of the thieno[2,3-d]pyrimidine derivative represented by formula (I-13) by the reaction with an oxidizing agent and then treating the product with trimethylsilyl cyanide to produce a thieno[2,3-d]pyrimidine derivative represented by formula (I-14).

This step can be also carried out in accordance with the method described in *Synthesis,* p. 681 (1984).

The oxidizing agent to be used in the step includes m-chloroperbenzoic acid, peracetic acid, or the like. The amount of the oxidizing agent to be used may be suitably selected from the range of equimolar to five molar equivalents.

The oxidation reaction can be also carried out in a solvent and any solvent harmless to the reaction can be used. As the solvent, water; an aromatic hydrocarbon solvent, e.g., toluene, chlorobenzene, or dichlorobenzene; a halogenated hydrocarbon solvent, e.g., chloroform, dichloromethane, or carbon tetrachloride; or a mixed solvent thereof can be used.

The objective compound can be obtained in good yields by carrying out the reaction at a temperature suitably selected from the range of 0°C to a reflux temperature of the solvent used.

Moreover, the cyanation reaction is preferably carried out in the presence of a base. As the base, an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); or a mixed solvent thereof, can be used.

The objective compound can be obtained in good yields by carrying out the reaction at a temperature suitably selected from the range of -78°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Production Method 6

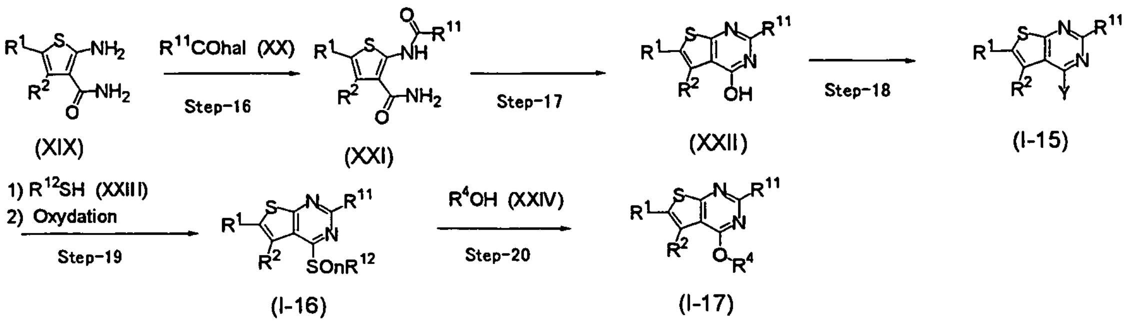


(XIX) (XXI) (XXII) (I-15) (I-16) (I-17)

wherein $R^1$, $R^2$, $R^4$, Y, hal, n, and $Q^2$ have the same meanings as described above, $R^{11}$ represents an alkyl which may be substituted or an aryl which may be substituted, and $R^{12}$ represents an alkyl.

Step 16 is a step of acylating an aminothiophene derivative represented by formula (XIX) with an acid halide represented by formula (XX) to produce a thiophene derivative represented by formula (XXI)

In this step, it is preferable to carry out the reaction in the presence of a base. As the base, an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo [5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); an ester solvent, e.g., ethyl acetate or methyl acetate; a halogenated hydrocarbon solvent, e.g., dichloromethane, chloroform, or carbon tetrachloride; or a mixed solvent thereof, can be used.

The objective compound can be obtained in good yields by carrying out the reaction at a temperature suitably selected from the range of -78°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 17 is a step of cyclizing the thiophene derivative represented by formula (XXI) to produce a 4-hydroxythieno[2,3-d]pyrimidine derivative represented by formula (XXII).

In this step, it is preferable to carry out the reaction in the presence of a base. As the base, an alkali metal base, e.g., sodium hydride, potassium hydride, sodium hydroxide, potassium hydroxide, sodium methoxide, sodium ethoxide, or potassium t-butoxide; or an organic base, e.g., triethylamine, diisopropylethylamine, tributylamine, N-methylmorpholine, 1,5-diazabicyclo[5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); an alcoholic solvent, e.g., methanol, ethanol, or isopropanol (IPA); or a mixed solvent thereof, can be used.

The objective compound can be obtained in good yields by carrying out the reaction at a temperature suitably selected from the range of 0°C to a reflux temperature of the solvent used.

After completion of the reaction, the objective compound can be isolated in a usual manner and, if necessary,

purified by an operation, e.g., recrystallization or silica gel column chromatography. The compound may be also used in the next reaction without isolation.

Step 18 is a step of halogenating the 4-hydroxythieno[2,3-d]pyrimidine derivative represented by formula (XXII) in a similar manner to the above-described Step 4 to produce a thieno[2,3-d]pyrimidine derivative represented by formula (I-15).

Step 19 is a step of reacting the thieno[2,3-d]pyrimidine derivative represented by formula (I-15) with a mercaptan represented by formula (XXIII) and then oxidizing the product to produce a thieno[2,3-d]pyrimidine derivative represented by formula (I-16).

In this step, the reaction with the mercaptan represented by formula (XXIII) can be carried out in good yields by using a base. As the base, an alkali metal base, e.g., sodium hydride, potassium hydride, sodium hydroxide, potassium hydroxide, sodium carbonate, or potassium carbonate; or an organic base, e.g., triethylamine, diisopropylethylamine, or tributylamine can be used. The objective compound can be obtained in good yields by using the base in an amount of 0.1 to 2.0 equivalents to the substrate. Also, the compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

As the oxidizing agent in this step, an oxidizing agent, e.g., m-chloroperbenzoic acid or peracetic acid can be used. The amount of the oxidizing agent to be used may be suitably selected from the range of equimolar to five molar equivalents.

The oxidation reaction can be also carried out in a solvent and any solvent harmless to the reaction can be used. As the solvent, water; an aromatic hydrocarbon solvent, e.g., toluene, chlorobenzene, or dichlorobenzene; a halogenated hydrocarbon solvent, e.g., chloroform, dichloromethane, or carbon tetrachloride; or a mixed solvent thereof can be used.

The objective compound can be obtained in good yields by carrying out the reaction at a temperature suitably selected from the range of 0°C to a reflux temperature of the solvent used.

After completion of the reaction, the objective compound can be isolated in a usual manner and, if necessary, purified by an operation, e.g., recrystallization or silica gel column chromatography. The compound may be also used in the next reaction without isolation.

Step 20 is a step of reacting the thieno[2,3-d]pyrimidine derivative represented by formula (I-16) with a compound represented by formula (XXIV) to produce a thieno[2,3-d]pyrimidine derivative represented by formula (I-17).

In this step, it is preferable to carry out the reaction in the presence of a base. As the base, an alkali metal base, e.g., sodium hydride, potassium hydride, sodium carbonate, potassium carbonate, sodium hydroxide, potassium hydroxide, sodium methoxide, sodium ethoxide, or potassium t-butoxide; or an organic base, e.g., triethylamine, diisopropylethylamine, tributylamine, N-methylmorpholine, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); an alcoholic solvent, e.g., methanol, ethanol, or isopropanol (IPA); or a mixed solvent thereof, can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography.

Production Method 7

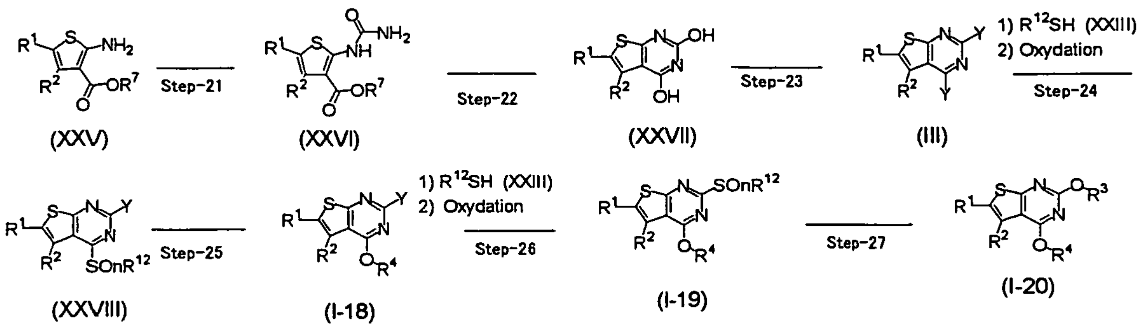


(XXV) (XXVI) (XXVII) (III)

(XXVIII) (I-18) (I-19) (I-20)

wherein $R^1$, $R^2$, $R^3$, $R^4$, $R^7$, $R^{12}$, Y, and n have the same meanings as described above.

Step 21 is a step of reacting an aminothiophene derivative represented by formula (XXV) with potassium cyanate or sodium cyanate to produce a ureidothiophene derivative represented by formula (XXVI).

The reaction can be carried out in a solvent and any solvent harmless to the reaction can be used. As the solvent, any solvent harmless to the reaction, such as water; an amide solvent, e.g., N,N-dimethylformamide (DMF), N,N-dimethylacetamide (DMAC), or N-methylpyrrolidone (NMP); a nitrile solvent, e.g., acetonitrile or propionitrile; an aromatic hydrocarbon solvent, e.g., benzene, toluene, xylene, or chlorobenzene; an aliphatic hydrocarbon solvent, e.g., pentane, hexane, or octane; an ether solvent, e.g., diethyl ether, diisopropyl ether, tetrahydrofuran (THF), dimethoxyethane (DME), or 1,4-dioxane; dimethyl sulfoxide (DMSO); acetic acid; an alcoholic solvent, e.g., methanol, ethanol, or isopropanol (IPA); or a mixed solvent thereof, can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography. The compound may be also used in the next reaction without isolation.

Step 22 is a step of producing a 2,4-dihydroxythieno[2,3-d]pyrimidine derivative represented by formula (XXVII) from the ureidothiophene derivative represented by formula (XXVI) in a similar manner to the above Step 17.

Step 23 is a step of halogenating the 2,4-dihydroxythieno[2,3-d]pyrimidine derivative represented by formula (XXVII) in a similar manner to the above Step 4 to produce a 2,4-dihalogenothieno[2,3-d]pyrimidine derivative represented by formula (III).

Steps 24 and 26 are steps of producing a thieno[2,3-d]pyrimidine derivative represented by formula (XXVIII) or (I-19) from the halogenothieno[2,3-d]pyrimidine derivative represented by formula (III) or (I-18) in a similar manner to the above Step 19.

Steps 25 and 27 are steps of producing a thieno[2,3-d]pyrimidine derivative represented by formula (I-18) or (I-20) from the thieno[2,3-d]pyrimidine derivative represented by formula (XXVIII) or (I-19) in a similar manner to the above Step 20.

Production Method 8

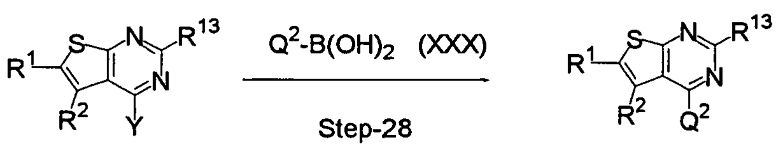


(XXIX) (I-21)

wherein $R^1$, $R^2$, $Q^2$, and Y have the same meanings as described above and $R^{13}$ represents halogen or trifluoromethylsulfonyloxy.

Step 28 is a step of coupling a 4-halogenothieno[2,3-d]pyrimidine derivative represented by formula (XXIX) with a boronic acid derivative represented by formula (XXX) to produce a thieno[2,3-d]pyrimidine derivative represented by formula (I-21).

This step can be carried out in accordance with the method described in *Tetrahedron Lett.,* Vol. 40, No. 51, p. 9005 (1999).

In this step, the reaction can be carried out in good yields by using a catalyst, e.g., triphenylphosphine palladium. The amount of the palladium catalyst to be used is suitably selected from the range of 0.0001 to 0.1 molar equivalent to the 4-halogenothieno[2,3-d]pyrimidine derivative represented by formula (XXIX). Moreover, an alkali metal base, e.g., sodium carbonate or potassium carbonate; or an organic base, e.g., triethylamine, diisopropylethylamine, tributylamine, N-methylmorpholine, N,N-dimethylaniline (DMA), N,N-diethylaniline, 4-t-butyl-N,N-dimethylaniline, pyridine, 4-(dimethylamino)pyridine (DMAP), picoline, lutidine, 1,5-diazabicyclo[5.4.0]undec-5-ene (DBU), 1,4-diazabicyclo[2.2.2]octane (DABCO), or imidazole can be used. The objective compound can be obtained in good yields by using the base in an amount of 0.1 to 2.0 equivalents to the substrate.

The reaction can be carried out in a solvent and any solvent harmless to the reaction can be used. As the solvent, any solvent harmless to the reaction, such as water; an aromatic hydrocarbon solvent, e.g., benzene, toluene, xylene, or chlorobenzene; an ether solvent, e.g., diethyl ether, diisopropyl ether, tetrahydrofuran (THF), dimethoxyethane (DME), or 1,4-dioxane; a halogenated hydrocarbon solvent, e.g., dichloromethane or chloroform; or a mixed solvent thereof, can be used.

The objective compound can be obtained in good yields by carrying out the reaction at a temperature suitably selected from the range of 0°C to a reflux temperature of the solvent used.

This reaction is an equimolar reaction and hence each reactant may be used in an equimolar amount, but it is also possible to use either reactant in an excess amount.

After completion of the reaction, the objective compound can be isolated in a usual manner and, if necessary, purified by an operation, e.g., recrystallization or silica gel column chromatography.

Production Method 9

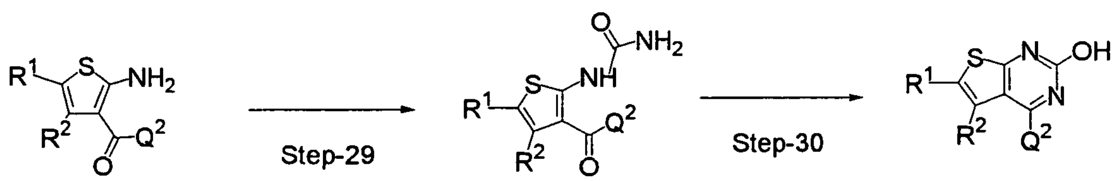


(XI) (XXXI) (XII)

wherein $R^1$, $R^2$, and $Q^2$ have the same meanings as described above.

Step 29 is a step of reacting the aminothiophene derivative represented by formula (XI) with potassium cyanate or sodium cyanate in a similar manner to the above Step 21 to produce a ureidothiophene derivative represented by formula (XXXI). Therein, the ureidothiophene derivative represented by formula (VI):

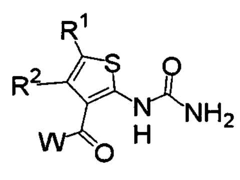

(VI)

wherein $R^1$ and $R^2$ have the same meanings as described above and W represents an aryl which may be substituted, is a novel compound.

Step 30 is a step of producing the 2-hydroxythieno[2,3-d]pyrimidine derivative represented by formula (XII) from the ureidothiophene derivative represented by formula (XXXI) in a similar manner to the above Step 17.

Production Method 10

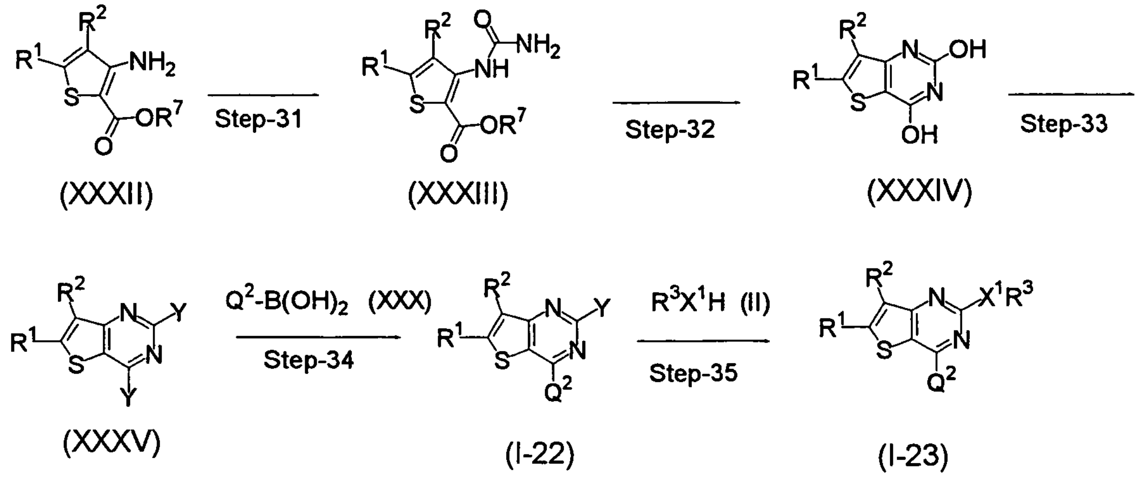


(XXXII) (XXXIII) (XXXIV)

(XXXV) (I-22) (I-23)

wherein $R^1$, $R^2$, $R^3$, $R^7$, $X^1$, Y, and $Q^2$ have the same meanings as described above.

Step 31 is a step of reacting an aminothiophene derivative represented by formula (XXXII) with potassium cyanate or sodium cyanate in a similar manner to the above Step 21 to produce a ureidothiophene derivative represented by formula (XXXIII).

Step 32 is a step of producing a 2,4-dihydroxythieno[3,2-d]pyrimidine derivative represented by formula (XXXIV) from the ureidothiophene derivative represented by formula (XXXIII) in a similar manner to the above Step 17.

Step 33 is a step of halogenating the 2,4-dihydroxythieno[3,2-d]pyrimidine derivative represented by formula (XXXIV) in a similar manner to the above-described Step 4 to produce a 2,4-dihalogenothieno[3,2-d]pyrimidine derivative represented by formula (XXXV).

Step 34 is a step of producing a 2-halogenothieno[3,2-d]pyrimidine derivative represented by formula (I-22) from the 2,4-dihalogenothieno[3,2-d]pyrimidine derivative represented by formula (XXXV) in a similar manner to the above Step 28.

Step 35 is a step of producing a thieno[3,2-d]pyrimidine derivative represented by formula (I-23) from the 2-halogenothieno[3,2-d]pyrimidine derivative represented by formula (I-22) in a similar manner to the above Step 5.

4. Herbicides

As the herbicide of the invention, the compound represented by the above formula (I) may be applied directly but is usually, formulated together with appropriate auxiliary agents into compositions such as wettable powders, granules, emulsifiable concentrates, and flowables.

While it varies depending on the composition form, a suitable content of the compound represented by the above formula (I) in the composition usually ranges from 1 to 90% by weight in wettable powders, from 0.1 to 30% by weight in granules, from 1 to 50% by weight in emulsifiable concentrates, and from 5 to 50% by weight in flowables. The compositions containing the compound of the present invention is used directly or as diluted with water according to the form.

The auxiliary agents to be used in formulating the compositions include solid carriers, such as kaoline, bentonite, talc, diatomaceous earth, white carbon, and starch; liquid carriers, such as water, alcohols (e.g., methanol, ethanol, propanol, butanol, and ethylene glycol), ketones (e.g., acetone, methyl ethyl ketone, cyclohexanone, and isophorone), ethers (e.g., diethyl ether, dioxane, and cellosolve), aliphatic hydrocarbons (e.g., kerosene and coal oil), aromatic hydrocarbons (e.g., benzene, toluene, xylene, cumene, solvent naphtha, and methylnaphthalene), halogenated hydrocarbons (e.g., dichloroethane, carbon tetrachloride, and trichlorobenzene), acid amides (e.g., dimethylformamide and dimethylacetamide), esters (e.g., ethyl acetate, butyl acetate, and fatty acid glycerol esters), and nitriles (e.g., acetonitrile); and surface active agents, such as nonionic surface active agents (e.g., polyoxyethylene glycol alkyl ethers, polyoxyethylene alkyl aryl ethers, polyoxyethylene fatty acid esters, and polyoxyethylene resin acid esters), cationic surface active agents (e.g., alkyldimethylbenzylammonium chlorides and alkylpyridinium chlorides), anionic surface active agents (e.g., alkylbenzenesulfonates, lignin sulfonates, dialkylsulfosuccinates, and higher alcohol sulfate esters), and amphoteric surface active agents (e.g., alkyldimethylbetaines and dodecylaminoethylglycine). These solid

carriers, liquid carriers, surface active agents, and the like may be each used either solely or as a mixture of two or more of them according to necessity.

When the composition containing the compound represented by the above formula (I) is applied after dilution with water, an auxiliary agent such as a spreading agent can be added to the applying liquid for the purpose of improving sticking properties and spreading properties to enhance the herbicidal effect. The auxiliary agents such as a spreading agent to be used include surface active agents (the above-described nonionic surface active agents, cationic surface active agents, anionic surface active agents, and amphoteric surface active agents), paraffin, polyvinyl acetate, polyacrylic acid salts, ethylene glycol, polyethylene glycol, crop oil (e.g., mineral oils, vegetable oils and animal oils), liquid fertilizers, and the like. Two or more kinds of these auxiliary agents can be used simultaneously, if desired. A suitable amount of the auxiliary agent such as a spreading agent is usually from 0.01 to 5% by weight based on the total applying liquid, while it varies depending on the kind of the auxiliary agent. Some of the auxiliary agents can previously be incorporated into the composition as the components.

The amount of the compound represented by the above formula (I) to be applied usually ranges from 2 to 2000 g, preferably 5 to 1000 g, per hectare in terms of the active ingredient, while it varies depending on such conditions as the structure of the compound, the weeds to be controlled, the time of treatment, the method of treatment, the properties of soil, and the like.

The weeds which can be controlled by the herbicides of the present invention include, as those growing in fields, broad-leaved ones such as Chenopodium album, Chenopodium centrorubrum, Polygonum longisetum, Polygonum persicaria, Amaranthus lividus, Amaranthus viridis, Stellaria media, Lamium amplexicaule, Abutilon theophrasi, Xanthium strumarium, Ipomoea purpurea, Datura metel, Brassica juncea, Galium spurium, Viola mandshurica, Matricaria matricarioides, and Bidens pilosa, and narrow-leaved ones such as Digitaria ciliaris, Eleusine indica, Echinochloa crus-galli, and Setaria viridis; and, as those growing in paddies, broad-leaved ones such as Rotala indica, Lindernia procumbens, Monochoria vaginalis, Dopatrium junceum, Elatine triandra, Alisma canaliculatum, Sagittaria trifolia, and Sagittaria pygmaea, and narrow-leaved ones such as Echinochloa oryzicola, Cyperus difformis, Scirpus juncoides, and Cyperus serotinus.

The herbicide of the present invention is effective in controlling the above-described weeds in fields and paddies in any of soil treatment, foliar treatment, and submerged treatment. Furthermore, the herbicide of the invention has small influences on the crops, such as corn, wheat, barley, rice, and soybeans, in either soil treatment or foliar treatment and can be used as a selective herbicide in cultivation of these crops.

The herbicide of the present invention can be used in combination with other agricultural chemicals, such as insecticides, fungicides and plant growth regulators, and fertilizers, which are used in the same field. It is also possible to apply the herbicide of the invention in combination with other herbicides to obtain stabilized herbicidal effects. When the compound represented by the above formula (I) and other herbicide are applied in combination, separately prepared compositions thereof may be mixed on at the time of application or they may be applied as a composition containing both of them beforehand. Examples of the herbicides that can be suitably applied in combination with the compound represented by the above formula (I) are shown below.

Organophosphorus herbicides:

N-(Phosphonomethyl)glycine and salts thereof,
4-[Hydroxy(methyl)phosphinoyl]-DL-homoalanine and salts thereof,
4-[Hydroxy(methyl)phosphinoyl]-L-homoalanyl-L-alanyl-L-alanine and salts thereof,
O-Ethyl O-6-nitro-m-tolyl sec-butylphosphoramidothioate,
S-[N-(4-Clorophenyl)-N-isopropylcarbamoylmethyl] O,O-dimethylphosphorodithioate,
O,O-Diisopropyl S-2-(phenylsulfonylamino)ethyl phosphorodithioate, and the like.

Carbamate herbicides:

2-Chloroallyl diethyldithiocarbamate,
S-2,3-Dichloroallyl diisopropylthiocarbamate,
S-2,2,3-Trichloroallyl diisopropylthiocarbamate,
S-Ethyl dipropylthiocarbamate,
S-Ethyl diisobutylthiocarbamate,
S-Benzyl 1,2-dimethylpropyl(ethyl)thiocarbamate,
S-4-Chlorobenzyl diethylthiocarbamate,
S-Ethyl perhydroazepine-1-thiocarboxylate,
S-Isopropyl perhydroazepine-1-thiocarboxylate,
S-1-Methyl-1-phenylethyl piperidine-1-thiocarboxylate,
O-3-t-Butylphenyl 6-methoxy-2-piridyl(methyl)thiocarbamate,

3-(Methoxycarbonylamino)phenyl 3'-methylphenylcarbamate,
Isopropyl 3'-chlorophenylcarbamate,
Methyl (4-aminophenylsulfonyl)carbamate, and the like.

Urea herbicides:

3-(3,4-Dichlorophenyl)-1,1-dimethylurea,
3-(3,4-Dichlorophenyl)-1-methoxy-1-methylurea,
1,1-Dimethyl-3-[3-(trifluoromethyl)phenyl]urea,
3-[4-(4-Methoxyphenoxy)phenyl]-1,1-dimethylurea,
1-(1-Methyl-1-phenylethyl)3-p-tolylurea,
3-(4-Isopropylphenyl)-1,1-dimethylurea,
3-(5-t-Butylisoxazol-3-yl)-I, 1-dimethylurea,
1-(5-t-Butyl-1,3,4-thiadiazol-2-yl)-1,3-dimethylurea,
1-(Benzothiazol-2-yl)-1,3-dimethylurea, and the like.

Amide herbicides:

2-Chloro-N-(pyrazol-1-ylmethyl)aceto-2',6'-xylidide,
2-Chloro-2',6'-diethyl-N-(methoxymethyl)acetanilide,
N-(Butoxymethyl)-2-chloro-2',6'-diethylacetanilide,
2-Chloro-2',6'-diethyl-N-(2-propoxyethyl)acetanilide,
2-Chloro-N-(ethoxymethyl)-6'-ethylaceto-o-toluidide,
2-Chloro-6'-ethyl-N-(2-methoxy-1-methylethyl)aceto-o-toluidide,
2-Chloro-N-(3-methoxy-2-thenyl)-2',6'-dimethylacetanilide,
N-(Chloroacetyl)-N-(2,6-diethylphenyl)glycine ethyl ester,
2-Chloro-N-(2,4-dimethyl-3-thienyl)-N-(2-methoxy-1-methylethyl)acetamide,
3',4'-Dichloropropionanilide,
2',4'-Difluoro-2-[3-(trifluoromethyl)phenoxy]nicotinanilide,
2-(1,3-Benzothiazol-2-yloxy)-N-methylacetanilide,
4'-Fluoro-N-isopropyl-2-(5-trifluoromethyl-1,3,4-thiadiazol-2-yloxy)acetanilide,
2-Bromo-N-($\alpha$,$\alpha$-dimethylbenzyl)-3,3-dimethylbutyramide,
N-[3-(1-Ethyl-1-methylpropyl)isoxazol-5-yl]-2,6-dimethoxybenzamide, and the like.

Dinitroaniline herbicides:

2,6-Dinitro-N,N-dipropyl-4-(trifluoromethyl)aniline,
N-Butyl-N-ethyl-2,6-dinitro-4-(trifluoromethyl)aniline,
2,6-Dinitro-$N^1$,$N^1$-dipropyl-4-(trifluoromethyl)-m-phenylenediamine,
4-(Dipropylamino)-3,5-dinitrobenzenesulfonamide,
N-sec-Butyl-4-t-butyl-2,6-dinitroaniline,
N-(1-Ethylpropyl)-2,6-dinitro-3,4-xylidine, and the like.

Carboxylic acid herbicides:

(2,4-Dichlorophenoxy)acetic acid and derivatives thereof,
(2,4,5-Trichlorophenoxy)acetic acid and derivatives thereof,
(4-Chloro-2-methylphenoxy)acetic acid and derivatives thereof,
2-(2,4-Dichlorophenoxy)propionic acid and derivatives thereof,
2-(4-Chloro-2-methylphenoxy)propionic acid and derivatives thereof,
4-(2,4-Dichlorophenoxy)butyric acid and derivatives thereof,
2,3,6-Trichlorobenzoic acid and derivatives thereof,
3,6-Dichloro-2-methoxybenzoic acid and derivatives thereof,
3,7-Dichloroquinoline-8-carboxylic acid and derivatives thereof,
7-Chloro-3-methylquinoline-8-carboxylic acid and derivatives thereof,
3,6-Dichloropyridine-2-carboxylic acid and derivatives thereof,
4-Amino-3,5,6-trichloropyridine-2-carboxylic acid and derivatives thereof,
(3,5,6-Trichloro-2-pyridyloxy)acetic acid and derivatives thereof,

(4-Amino-3,5-dichloro-6-fluoro-2-pyridyloxy)acetic acid and derivatives thereof,
(4-Chloro-2-oxobenzothiazolin-3-yl)acetic acid and derivatives thereof,
2-[4-(2,4-Dichlorophenoxy)phenoxy]propionic acid and derivatives thereof,
2-[4-[5-(Trifluoromethyl)-2-pyridyloxy]phenoxy]propionic acid and derivatives thereof,
2-[4-[3-Chloro-5-(trifluoromethyl)-2-pyridyloxy]phenoxy]propionic acid and derivatives thereof,
2-[4-(6-Chloro-1,3-benzoxazol-2-yloxy)phenoxy]propionic acid and derivatives thereof,
2-[4-(6-Chloroquinoxalin-2-yloxy)phenoxy]propionic acid and derivatives thereof,
2-[4-(4-Cyano-2-fluorophenoxy)phenoxy]propionic acid and derivatives thereof, and
the like.

Phenol herbicides:

3,5-Dibromo-4-hydroxybenzonitrile and derivatives thereof,
4-Hydroxy-3,5-diiodobenzonitrile and derivatives thereof,
2-t-Butyl-4,6-dinitrophenol and derivatives thereof, and the like.

Cyclohexanedione hebicides:

Methyl 3-[1-(allyloxyimino)butyl]-4-hydroxy-6,6-dimethyl-2-oxo-3-cyclohexene-1-carboxylate and derivatives thereof,
2-[1-(Ethoxyimino)butyl]-5-[2-(ethylthio)propyl]-3-hydroxy-2-cyclohexen-1-one,
2-[1-(Ethoxyimino)butyl]-3-hydroxy-5-(thian-3-yl)-2-cyclohexen-1-one,
2-[1-(Ethoxyimino)propyl]-3-hydroxy-5-(2,4,6-trimethylphenyl)-2-cyclohexen-1-one,
5-(3-Butyryl-2,4,6-trimethylphenyl)-2-[1-(ethoxyimino)propyl]-3-hydroxy-2-cyclohexen-1-one,
2-[1-(3-Chloroallyloxyimino)propyl]-5-[2-(ethylthio)propyl]-3-hydroxy-2-cyclohexen-1-one,
2-[1-(3-Chloroallyloxyimino)propyl]-3-hydroxy-5-perhydropyran-4-yl-2-cyclohexen-1-one,
2-[2-Chloro-4-(methylsulfonyl)benzoyl]cyclohexane-1,3-dione, and the like.

Diphenyl ether herbicides:

4-Nitrophenyl 2,4,6-trichlorophenyl ether,
5-(2,4-Dichlorophenoxy)-2-nitroanisole,
2-Chloro-4-(trifluoromethyl)phenyl 3-ethoxy-4-nitrophenyl ether,
Methyl 5-(2,4-dichlorophenoxy)-2-nitrobenzoate,
5-[2-Chloro-4-(trifluoromethyl)phenoxy]-2-nitrobenzoic acid and salts thereof,
Ethyl O-[5-[2-chloro-4-(trifluoromethyl)phenoxy]-2-nitrobenzoyl]glycolate
Ethyl O-[5-[2-chloro-4-(trifluoromethyl)phenoxy]-2-nitrobenzoyl]-DL-lactate
5-[2-Chloro-4-(trifluoromethyl)phenoxy]-N-(methylsulfonyl)-2-nitrobenzamide,
2-Chloro-6-nitro-3-phenoxyaniline, and the like.

Sulfonylurea herbicides:

1-(4,6-Dimethoxypyrimidin-2-yl)-3-mesyl(methyl)sulfamoylurea,
Ethyl 2-(4-chloro-6-methoxypyrimidin-2-ylcarbamoylsulfamoyl)benzoate,
Methyl 2-(4,6-dimethylpyrimidin-2-ylcarbamoylsulfamoyl)benzoate,
Methyl 2-[4,6-bis(difluoromethoxy)pyrimidin-2-ylcarbamoylsulfamoyl]benzoate,
Methyl 2-(4,6-dimethoxypyrimidin-2-ylcarbamoylsulfamoylmethyl)benzoate,
1-(4,6-Dimethoxypyrimidin-2-yl)-3-(2-ethoxyphenoxysulfonyl)urea,
1-[2-(Cyclopropylcarbonyl)phenylsulfamoyl]-3-(4,6-dimethoxypyrimidin-2-yl)urea,
1-(2-Chlorophenylsulfonyl)-3-(4-methoxy-6-methyl-1,3,5-triazin-2-yl)urea,
Methyl 2-(4-methoxy-6-methyl-1,3,5-triazin-2-ylcarbamoylsulfamoyl)benzoate,
Methyl 2-[4-methoxy-6-methyl-1,3,5-triazin-2-yl(methyl)carbamoylsulfamoyl)benzoate,
1-[2-(2-Chloroethoxy)phenylsulfonyl)-3-(4-methoxy-6-methyl-1,3,5-triazin-2-yl)urea,
1-(4,6-Dimethoxy-1,3,5-triazin-2-yl)-3-[2-(2-methoxyethoxy)phenylsulfonyl]urea,
Methyl 2-[4-ethoxy-6-(methylamino)-1,3,5-triazin-2-ylcarbamoylsulfamoyl)benzoate,
Methyl 2-[4-(dimethylamino)-6-(2,2,2-trifluoroethoxy)-1,3,5-triazin-2-ylcarbamoylsulfamoyl)-3-methylbenzoate,
1-(4-Methoxy-6-methyl-1,3,5-triazin-2-yl)-3-[2-(3,3,3-trifluoropropyl)phenylsulfonyl]urea,
Methyl 3-(4-methoxy-6-methyl-1,3,5-triazin-2-ylcarbamoylsulfamoyl)thiophene-2-carboxylate,

Ethyl 5-(4,6-dimethoxypyrimidin-2-ylcarbamoylsulfamoyl)-1-methylpyrazole-4-carboxylate,
Methyl 3-chloro-5-(4,6-dimethoxypyrimidin-2-ylcarbamoylsulfamoyl)-1-methylpyrazole-4-carboxylate,
1-(4,6-Dimetoxypyrimidin-2-yl)-3-[3-(trifluoromethyl)-2-pyridylsulfonyl]urea,
1-(4,6-Dimetoxypyrimidin-2-yl)-3-[3-(ethylsulfonyl)-2-pyridylsulfonyl]urea,
2-(4,6-Dimethoxypyrimidin-2-ylcarbamoylsulfamoyl)-N,N-dimethylnicotinamide,
Methyl 2-(4,6-dimethoxypyrimidin-2-ylcarbamoylsulfamoyl)-6-trifluoromethylnicotinate and salts thereof,
1-(2-Chloroimidazo[1,2-a]pyridin-3-ylsulfonyl)-3-(4,6-dimethoxypyrimidin-2-yl)urea,
1-(4,6-Dimethoxypyrimidin-2-yl)-3-[2-(ethylsulfonyl)imidazo[1,2-a]pyridin-3-ylsulfonyl)urea, and the like.

Bipyridinium herbicides:

1,1'-Dimethyl-4,4'-bipyridinium dichloride,
1,1'-Ethylene-2,2'-bipyridinium dibromide, and the like.

Pyrazole herbicides:

4-(2,4-Dichlorobenzoyl)-1,3-dimethylpyrazol-5-yl toluene-4-sulfonate,
2-[4-(2,4-Dichlorobenzoyl)-1,3-dimethylpyrazol-5-yloxy]acetophenone,
2-[4-(2,4-Dichloro-3-methylbenzoyl)-1,3-dimethylpyrazol-5-yloxy]-4'-methylacetophenone, and the like.

Triazine herbicides:

6-Chloro-$N^2$,$N^4$-diethyl-1,3,5-triazine-2,4-diamine,
6-Chloro-$N^2$-ethyl-$N^4$-isopropyl-1,3,5-triazine-2,4-diamine,
2-[4-Chloro-6-(ethylamino)-1,3,5-triazin-2-ylamino]-2-methylpropionitrile,
$N^2$,$N^4$-Diethyl-6-(methylthio)-1,3,5-triazine-2,4-diamine,
$N^2$-(1,2-Dimethylpropyl)-$N^4$-ethyl-6-(methylthio)-1,3,5-triazine-2,4-diamine,
4-Amino-6-t-butyl-3-(methylthio)-1,2,4-triazin-5(4H)-one, and the like.

Imidazolinone Herbicides:

Methyl 2-(4-isopropyl-4-methyl-5-oxo-2-imidazolin-2-yl)-4(5)-methylbenzoate,
2-(4-Isopropyl-4-methyl-5-oxo-2-imidazolin-2-yl)pyridine-3-carboxylic acid and salts thereof,
2-(4-Isopropyl-4-methyl-5-oxo-2-imidazolin-2-yl)quinoline-3-carboxylic acid and salts thereof,
5-Ethyl-2-(4-isopropyl-4-methyl-5-oxo-2-imidazolin-2-yl)pyridine-3-carboxylic acid and salts thereof,
2-(4-Isopropyl-4-methyl-5-oxo-2-imidazolin-2-yl)-5-(methoxymethyl)pyridine-3-carboxylic acid and salts thereof, and the like.

Other Herbicides:

3-[2-Chloro-4-(methylsulfonyl)benzoyl]-2-(phenylthio)bicyclo[3.2.1]-2-octen-4-one,
2-[2-(3-Chlorophenyl)-2,3-epoxypropyl]-2-ethylindane-1,3-dione,
1-Methyl-3-phenyl-5-[3-(trifluoromethyl)phenyl]-4-pyridone,
3-Chloro-4-(chloromethyl)-1-[3-(trifluoromethyl)phenyl]-2-pyrrolidinone,
5-(Methylamino)-2-phenyl-4-[3-(trifluoromethyl)phenyl]furan-3-(2H)-one,
4-Chloro-5-(methylamino)-2-[3-(trifluoromethyl)phenyl]pyridazin-3(2H)-one,
N,N-Diethyl-3-(2,4,6-trimethylphenylsulfonyl)-1H-1,2,4-triazole-1-carboxamide,
4-(2-Chlorophenyl)-N-cyclohexyl-N-ethyl-4,5-dihydro-5-oxotetrazole-1-carboxamide,
N-[2,4-Dichloro-5-[4-(dichloromethyl)-4,5-dihydro-3-methyl-5-oxo-1H-1,2,4-triazol-1-yl]phenyl]methanesulfonamide,
Ethyl 2-chloro-3-[2-chloro-5-[4-(diflfuoromethyl)-4,5-dihydro-3-methyl-5-oxo-1H-1,2,4-triazol-1-yl]-4-fluorophenyl] propionate,
3-[4-Chloro-5-(cyclopentyloxy)-2-fluorophenyl)-5-isopropylidene-1,3-oxazolidine-2,4-dione,
5-t-Butyl-3-(2,4-dichloro-5-isopropoxyphenyl)-1,3,4-oxadiazol-2(3H)-one,
Ethyl [2-chloro-5-[4-chloro-5-(difluoromethoxy)-1-methylpyrazol-3-yl]-4-fluorophenoxy]acetate,
Isopropyl 5-(4-bromo-1-methyl-5-trifluoromethylpyrazol-3-yl]-2-chloro-4-fluorobenzoate,
1-[4-Chloro-3-(2,2,3,3,3-pentafluoropropoxymethyl)phenyl]-5-phenyl-1H-1,2,4-triazole-3-carboxamide,
2-(2-Chlorobenzyl)-4,4-dimethylisoxazolidin-3-one,

5-Cyclopropyl-4-[2-(methylsulfonyl)-4-(trifluoromethyl)benzoyl]isoxazole,
S, S'-Dimethyl 2-(difluoromethyl)-4-isobutyl-6-(trifluoromethyl)pyridine-3, 5-dicarbothioate,
Methyl 2-(difluoromethyl)-5-(4,5-dihydro-1,3-thiazol-2-yl)-4-isobutyl-6-(trifluoromethyl)pyridine-3-carboxylate,
2-Chloro-6-(4,6-dimethoxypyrimidin-2-ylthio)benzoic acid and salts thereof,
Methyl 2-(4,6-dimethoxypyrimidin-2-yloxy)-6-[1-(methoxyimino)ethyl]benzoate,
2,6-Bis(4,6-dimethoxypyrimidin-2-yloxy)benzoic acid and salts thereof,
5-Bromo-3-sec-butyl-6-methylpyrimidine-2,4(1H,3H)-dione,
3-t-Butyl-5-chloro-6-methylpyrimidine-2,4(1H,3H)-dione,
3-Cyclohexyl-1,5,6,7-tetrahydrocyclopentapyrimidine-2,4(3H)-dione,
Isopropyl 2-chloro-5-[1,2,3,6-tetrahydro-3-methyl-2,6-dioxo-4-(trifluoromethyl)pyrimidin-1-yl]benzoate,
3-[1-(3,5-Dichlorophenyl)-1-methylethyl]-3,4-dihydro-6-methyl-5-phenyl-2H-1,3-oxadin-4-one,
1-Methyl-4-isopropyl-2-[(2-methylphenyl)methoxy]-7-oxabicyclo[2.2.1]heptane,
N-(4-Chlorophenyl)-3,4,5,6-tetrahydrophthalimide,
Pentyl [2-chloro-4-fluoro-5-(1,3,4,5,6,7-hexahydro-1,3-dioxo-2H-isoindol-2-yl)phenoxy]acetate,
2-[7-Fluoro-3,4-dihydro-3-oxo-4-(2-propynyl)-2H-1,4-benzoxazin-6-yl]-4,5,6,7-tetrahydro-1H-isoindole-1,3(2H)-dione,
Ethyl 2-chloro-3-[2-chloro-5-(1,3,4,5,6,7-hexahydro-1,3-dioxo-2H-isoindol-2-yl)phenyl]acrylate,
2-[2,4-Dichloro-5-(2-propynyloxy)phenyl]-5,6,7,8-tetrahydro-1,2,4-triazolo[4,3-a]pyridin-3 (2H)-one,
Methyl [[2-chloro-4-fluoro-5-[(tetrahydro-3-oxo-1H,3H-[1,3,4]thiadiazolo[3,4a]pyridazin-1-ylidene)amino]phenyl]thio]acetate,
N-(2,6-Difluorophenyl)-5-methyl[1,2,4]triazolo[1,5-a]pyrimidine-2-sulfonamide, N-(2,6-Dichloro-3-methylphenyl)-5,7-dimethoxy[1,2,4]triazolo[1, 5-a]pyrimidine-2-sulfonamide,
Methyl 3-chloro-2-(5-ethoxy-7-fluoro[1,2,4]triazolo[1,5-c]pyrimidin-2-ylsulfonylamino)benzoate,
2,3-Dihydro-3,3-dimethylbenzofuran-5-yl ethanesulfonate,
2-Ethoxy-2,3-dihydro-3,3-dimethylbenzofuran-5-yl methanesulfonate,
3-Isopropyl-1H-2,1,3-benzothiadiazin-4(3H)-one-2,2-dioxide, and the like.

In these days, IPM (integrated pest management) technologies using transgenic crops (herbicide-resistant crops, insect-resistant crops into which a gene producing an insecticidal protein has been introduced, disease-resistant crops into which a gene producing a disease resistance-inducible substance has been introduced, taste-improved crops, storage stability-improved crops, yield-improved crops, *etc.*), insect pheromones (agents for disturbing communication of *tortrix or barathra*), natural enemy insects and the like have been developed. The agricultural composition of the present invention can be used in combination or systematization with those technologies.

The invention is described below in further detail with reference to Examples, Reference Examples, and Test Examples but the invention is not limited to the following Examples, Reference Examples, and Test Examples.

Example 1: Production of ethyl 3-(trifluoromethoxy)benzoate (Compound No. 1-2)

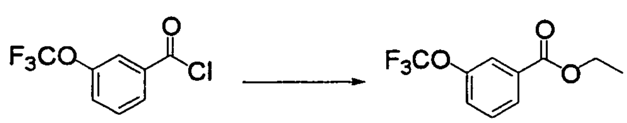

A mixed solution of ethanol (100 mL) and triethylamine (28.0 g, 0.277 mol) was cooled with ice-water and then 3-(trifluoromethoxy)benzoyl chloride (50.0 g, 0.223 mol) was slowly added dropwise thereto, followed by stirring at room temperature for 3 hours. After completion of the reaction, the solvent was removed by evaporation and the resulting residue was dissolved in ethyl acetate. After the solution was washed with water and saturated brine, the solvent was removed by evaporation. The resulting residue was purified on a silica gel column (Kiesel gel 60 manufactured by MERCK, 2% AcOEt-Hex) to obtain ethyl 3-(trifluoromethoxy)benzoate (52.0 g, 99%).
$^1$H-NMR (400MHz, $CDCl_3$) : 1.41(t, 3H, J=7.2Hz), 4.40(q, 2H, J=7.2Hz), 7.40(br. d, 1H, J=8.0Hz), 7.48(t, 1H, J=8.0Hz), 7.89(m, 1H), 7.99(dt, 1H, J=8.0Hz, 1.2Hz). nD(25°C): 1.5890

Example 2: Production of [4-fluoro-3-(trifluoromethyl)phenyl]-3-oxopropionitrile (Compound No. 2-9)

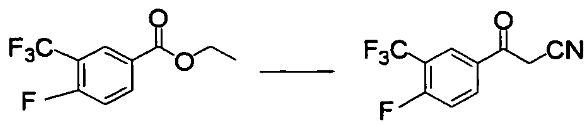

An ammonia gas was bubbled into a four-neck flask cooled in a dry ice-acetone bath to collect liquid ammonia (87 g). Thereto was added sodium amide (8.26 g, 0.212 mol) and then were added a toluene solution (10 mL) of acetonitrile (8.69 g, 0.212 mol) and a toluene solution (10 mL) of ethyl 4-fluoro-3-(trifluoromethyl)benzoate (25.0 g, 0.106 mol), followed by stirring at -70°C for 1 hour. While the reaction liquid was warmed to room temperature, the ammonia gas was released outside the system. After completion of the reaction, the reaction liquid was poured into water and extracted with toluene. The resulting aqueous layer from which insoluble matter had been removed was acidified with concentrated hydrochloric acid, followed by extraction with ethyl acetate. After the resulting extract was washed with water and saturated brine, the solvent was removed by evaporation. The resulting residue was purified on a silica gel column (Kiesel gel 60 manufactured by MERCK, 40% AcOEt-Hex) to obtain [4-fluoro-3-(trifluoromethyl) phenyl]-3-oxopropionitrile (19.4 g, 79%).
$^1$H-NMR (400MHz, $CDCl_3$) : 4.13(s, 2H), 7.40(t, 1H, J=7.6Hz), 8.17(m, 1H), 8.21(d, 1H, J=7.6Hz)

Example 3: Production of 3-oxo-3-[3-(trifluoromethyl)phenyl]propionitrile (Compound No. 2-2)

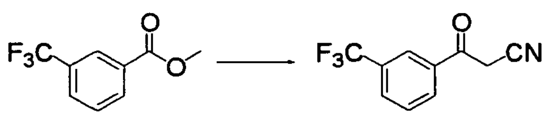

Acetonitrile (4.02 g, 97.8 mmol) was added to a THF (50 mL) suspension of sodium hydride (3.91 g, 97.8 mmol), followed by heating at 70°C for 1 minute. The reaction liquid was cooled to room temperature and a THF (20 mL) solution of methyl 3-(trifluoromethyl)benzoate (10.0 g, 48.9 mmol) was added dropwise thereto, followed by heating under stirring at 60°C for 6 hours. After completion of the reaction, solvent was removed by evaporation and the resulting residue was poured into water, followed by extraction with ethyl acetate. After the resulting extract solution was washed with water and brine, the solvent was removed by evaporation. The resulting residue was purified on a silica gel column (Kiesel gel 60 manufactured by MERCK, 30% AcOEt-Hex) to obtain 3-oxo-3-[3-(trifluoromethyl)phenyl]propionitrile (9.67 g, 93%).
Alternatively, the compound can be also produced by the following method.
Sodium hydride (3.56 g, 89.0 mmol) was added to a toluene (60 mL) solution of methyl 3-(trifluoromethyl) benzoate (9.08 g, 44.5 mmol), followed by heating under stirring to 85°C. While the gas generation state was taken into account, acetonitrile (3.65 g, 89.0 mmol) was slowly added dropwise thereto, followed by continuation of heating under stirring at 85°C for 2 hours. After completion of the reaction, the reaction liquid was poured into water, followed by extraction with toluene. Thereafter, the resulting aqueous layer was cooled with ice water and acidified with concentrated hydrochloric acid. After filtration of precipitated crystals, the resulting crystals were washed with water and hexane and dried to obtain 3-oxo-3-[3-(trifluoromethyl)phenyl] propionitrile (8.17 g, 80%).
$^1$H-NMR (400MHz, $CDCl_3$) : 4.14(s, 2H), 7.71(t, 1H, J=8.0Hz), 7.93(d, 1H, J=8.0Hz), 8.12(d, 1H, J=8.0Hz), 8.18(s, 1H).
mp : 63°C

Example 4: Production of 2-amino-3-[3-(trifluoromethyl)benzoyl]thiophene (Compound No. 3-4)

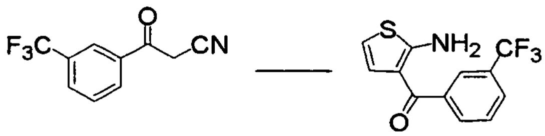

A DMF (30 mL) solution of 3-oxo-3-[3-(trifluoromethyl)phenyl]propionitrile (12.0 g, 0.0563 mol), 2,5-dihydroxy-1,4-dithian (4.56 g, 0.0296 mol), and triethylamine (6.26 g, 0.0620 mol) was heated under stirring at 60°C for 1 hour. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 20% AcOEt-Hex) to obtain 2-amino-3-[3-(trifluoromethyl)benzoyl]thiophene (5.75 g, 38%).
$^1$H-NMR (400MHz, $CDCl_3$):6.16(d, 1H, J=5.6Hz), 6.80(d, 1H, J=5.6Hz), 7.01(br.s, 2H), 7.58(t, 1H, J=8.0Hz), 7.75(d, 1H, J=8.0Hz), 7.86(d, 1H, J=8.0Hz), 7.94(s, 1H).
mp : 143-145°C

Example 5: Production of 2-amino-5-ethyl-3-[3-(trifluoromethyl)benzoyl]thiophene (Compound No. 3-3)

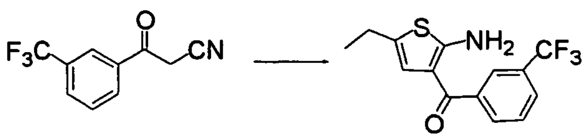

To a DMF (30 mL) solution of 3-oxo-3-[3-(trifluoromethyl)phenyl]propionitrile (4.60 g, 21.5 mmol), sulfur (0.69 g, 21.5 mmol), and triethylamine (2.18 g, 21.5 mmol) heated under stirring at 40°C was added dropwise an ethanol (2 mL) solution of butyraldehyde (1.71 g, 23.7 mmol), followed by heating under stirring at 60°C for 4 hours. After completion of the reaction, the reaction liquid was poured into water and precipitated crystals were collected by filtration. After the crystals were dissolved in ethyl acetate, the solution was washed with water and brine and then the solvent was removed by evaporation. The residue was purified on a silica gel column (Kiesel gel 60 manufactured by MERCK, 40% AcOEt-Hex) and the resulting crystals were washed with hexane to obtain 2-amino-5-ethyl-3-[3-(trifluoromethyl)benzoyl]thiophene (3.00 g, 47%).
$^1$H-NMR (400MHz, $CDCl_3$) : 1.21(t, 3H, J=7.6Hz), 2.60(q, 2H, J=7.6Hz), 6.42(s, 1H), 6.96(br.s, 2H), 7.50(t, 1H, J=8.0Hz), 7.73(d, 1H, J=8.0Hz), 7.83(d, 1H, J=8.0Hz), 7.92(s, 1H).

Example 6: Production of 2-amino-5-methyl-3-[2-(trifluoromethyl)benzoyl]thiophene (Compound No. 3-1)

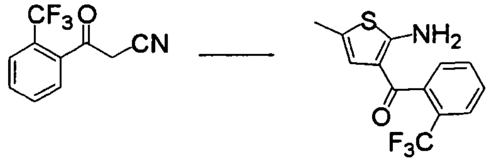

To a DMF (30 mL) solution of 3-oxo-3-[2-(trifluoromethyl)phenyl]propionitrile (3.10 g, 14.5 mmol), sulfur (0.47 g, 14.5 mmol), and triethylamine (1.61 g, 16.0 mmol) heated under stirring at 40°C was added dropwise an ethanol (1 mL) solution of propionaldehyde (0.93 g, 16.0 mmol), followed by heating under stirring at 60°C for 4 hours. After completion of the reaction, the reaction liquid was poured into water and precipitated crystals were collected by filtration.

After the crystals were dissolved in ethyl acetate, the solution was washed with water and brine and then the solvent was removed by evaporation. The residue was purified on a silica gel column (Kiesel gel 60 manufactured by MERCK, 20% AcOEt-Hex) and the resulting crystals were washed with hexane to obtain 2-amino-5-methyl-3-[2-(trifluoromethyl) benzoyl]thiophene (1.74 g, 42%).
$^1$H-NMR (400MHz, $CDCl_3$) : 2.17(d, 3H, J=1.2Hz), 5.98(d, 1H, J=1.2Hz), 6.90(br.s, 2H), 7.39(d, 1 H,J=7.2Hz), 7.5-7.6 (m, 2H), 7.72(m, 1H).
mp : 136-137°C

Example 7: Production of 4-(2-chloropyridine-3-yl)-6-methylthieno[2,3-d]pyrimidine-2-ol (Compound No. 4-22)

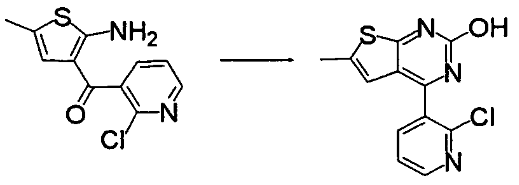

An NMP (20 mL) solution of 2-amino-3-[(2-chloropyridin-3-yl)carbonyl]-5-methylthiophene (7.20 g, 0.0285 mol) and urea (8.55 g, 0.143 mol) was heated under stirring at 180°C for 2 hours. After completion of the reaction, the reaction liquid was poured into water and the precipitated crystals were collected by filtration. The crystals were purified by washing with acetone to obtain 4-(2-chloropyridine-3-yl)-6-methylthieno[2,3-d]pyrimidine-2-ol (4.88 g, 53%).
$^1$H-NMR (400MHz, $CDCl_3$) : 2.54(d, 3H, J=1.2Hz), 7.24(d, 1H, J=1.2Hz), 7.37(dd, 1H, J=4.8Hz, 8.0Hz), 8.74(dd, 1H, J=2.0Hz, 4.8Hz), 8.86(dd, 1H, J=2.0Hz, 8.0Hz).
mp : >300°C

Example 8: Production of 6-methyl-4-[3-(trifluoromethyl)phenyl]thieno[2,3-d]pyrimidine-2-ol (Compound No. 4-3)

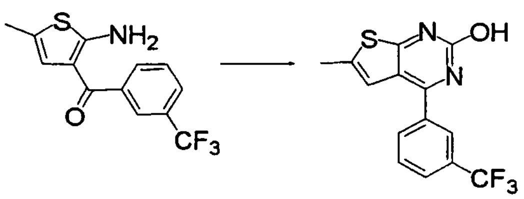

A NMP (5 mL) solution of 2-amino-5-methyl-3-[3-(trifluoromethyl)benzoyl]thiophene (1.50 g, 5.26 mmol) and urea (1.58 g, 26.3 mmol) was heated under stirring at 180°C for 5 hours. After completion of the reaction, the reaction liquid was poured into water and precipitated crystals were collected by filtration. The crystals were washed with isopropanol and then dried to obtain 6-methyl-4-[3-(trifluoromethyl)phenyl]thieno[2,3-d]pyrimidine-2-ol (1.30 g, 80%).
$^1$H-NMR (400MHz, $CDCl_3$) : 2.49(d, 3H, J=1.2Hz), 6.75(d, 1H, J=1.2Hz), 7.78(t, 1H, J=8.0Hz),7.85(d, 1H, J=8.0Hz), 8.02(s, 1H), 8.07(d, 1H, J=8.0Hz).
mp : 251-253°C (dec.)

Example 9: Production of 2-chloro-6-methyl-4-(3-methylthiophen-2-yl)thieno[2,3-d]pyrimidine (Compound No. 5-22) and 2-chloro-4-(5-formyl-3-methylthiophen-2-yl)-6-methylthieno[2,3-d]pyrimidine (Compound No. 5-23)

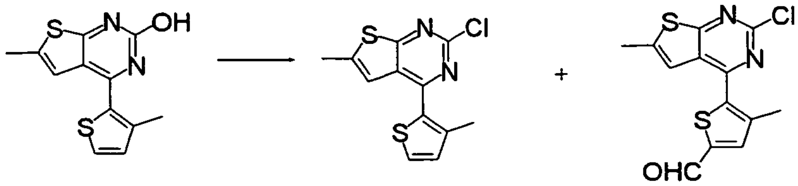

DMF (8 g) was added dropwise to phosphorus oxychloride (40.2 g, 0.263 mol) and then 6-methyl-4-(3-methylthiophen-2-yl)thieno[2,3-d]pyrimidine-2-ol (8.60 g, 0.0329 mol) was added dropwise thereto, followed by heating under stirring at 100°C for 2 hours. After completion of the reaction, the reaction liquid was poured into ice and precipitated crystals were collected by filtration. After the crystals were dissolved in ethyl acetate, the solution was washed with an aqueous sodium bicarbonate solution, water, and brine and then the solvent was removed by evaporation. The residue was purified on a silica gel column (Kiesel gel 60 manufactured by MERCK, 20% AcOEt-Hex) to obtain 2-chloro-6-methyl-4-(3-methylthiophen-2-yl)thieno[2,3-d]pyrimidine (8.10 g, 88%) and 2-chloro-4-(5-formyl-3-methylthiophen-2-yl)-6-methylthieno[2,3-d]pyrimidine (0.86 g, 8%).
2-Chloro-6-methyl-4-(3-methylthiophen-2-yl)thieno[2,3-d]pyrimidine $^1$H-NMR (400MHz, $CDCl_3$) : 2.52(s, 3H), 2.63(d, 3H, J=1.2Hz), 7.03(d, 1H, J=4.8Hz), 7.27(d, 1H, J=1.2Hz), 7.48(d, 1H, J=4.8Hz).
mp : 127°C
2-Chloro-4-(5-formyl-3-methylthiophen-2-yl)-6-methylthieno[2,3-d]pyrimidine $^1$H-NMR (400MHz, $CDCl_3$) : 2.55(s, 3H), 2.65(d, 3H, J=1.2Hz), 7.28(d, 1H, J=1.2Hz), 7.69(s, 1H), 9.97(s, 1H).
mp : 204-206°C (dec.)

Example 10: Production of 2-chloro-4-(3-chlorophenyl)-6-methylthieno[2,3-d]pyrimidine (Compound No. 5-15)

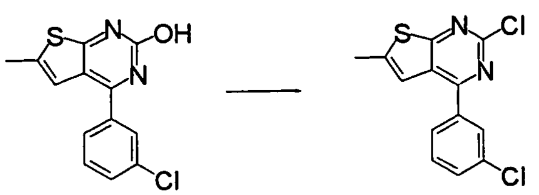

DMF (16 g) was added dropwise to phosphorus oxychloride (42.9 g, 0.280 mol) and then 4-(3-chlorophenyl)-6-methylthieno[2,3-d]pyrimidine-2-ol (15.5 g, 0.0561 mol) was added thereto, followed by heating under stirring at 100°C for 2 hours. After completion of the reaction, the reaction liquid was poured into ice and precipitated crystals were collected by filtration. After the crystals were dissolved in ethyl acetate, the solution was washed with an aqueous sodium bicarbonate solution, water, and brine and then the solvent was removed by evaporation. The residue was purified on a silica gel column (Kiesel gel 60 manufactured by MERCK, 20-30% AcOEt-Hex) to obtain 2-chloro-4-(3-chlorophenyl)-6-methylthieno[2,3-d]pyrimidine (5.73 g, 35%).
$^1$H-NMR (400MHz, $CDCl_3$) : 2.65(d, 3H, J=1.2Hz), 7.19(d, 1H, J=1.2Hz), 7.5(m, 2H), 7.80(dt, 1H, J=1.6Hz, 7.2Hz), 7.92(t, 1H, J=2.0Hz).
mp : 118-120°C

Example 11: Production of 2-chloro-6-methyl-4-[3-(trifluoromethyl)phenyl]thieno[2,3-d]pyrimidine (Compound No. 5-4)

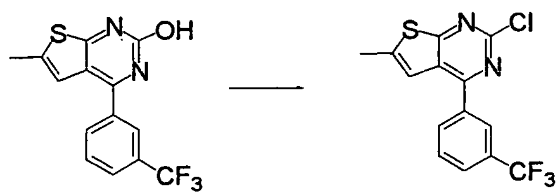

DMF (1.2 g) was added dropwise to phosphorus oxychloride (5.13 g, 33.5 mmol) and then 6-methyl-4-[3-(trifluoromethyl)phenyl]thieno[2,3-d]pyrimidine-2-ol (1.30 g, 4.19 mmol) was added thereto, followed by heating under stirring at 100°C for 3 hours. After completion of the reaction, the reaction liquid was poured into ice and precipitated crystals were collected by filtration. After the crystals were dissolved in ethyl acetate, the solution was washed with water and brine and then the solvent was removed by evaporation. The residue was purified on a silica gel column (Kiesel gel 60 manufactured by MERCK, 20% AcOEt-Hex) and the resulting crystals were washed with hexane to obtain 2-chloro-6-methyl-4-[3-(trifluoromethyl)phenyl]thieno[2,3-d]pyrimidine (1.12 g, 81%).
$^1$H-NMR (400MHz, $CDCl_3$) : 2.66(d, 3H, J=1.2Hz), 7.16(d, 1H, J=1.2Hz), 7.70(t, 1H, J=8.0Hz),7.82(d, 1H, J=8.0Hz), 8.12(d, 1H, J=8.0Hz), 8.19(s, 1H).
mp : 108°C

Example 12: Production of 4-[4-fluoro-3-(trifluoromethyl)phenyl]-6-methyl-2-propoxythieno[2,3-d]pyrimidine (Compound No. 6-29) and 6-methyl-2-propoxy-4-[4-propoxy-3-(trifluoromethyl)phenyl]thieno[2,3-d]pyrimidine (Compound No. 6-32)

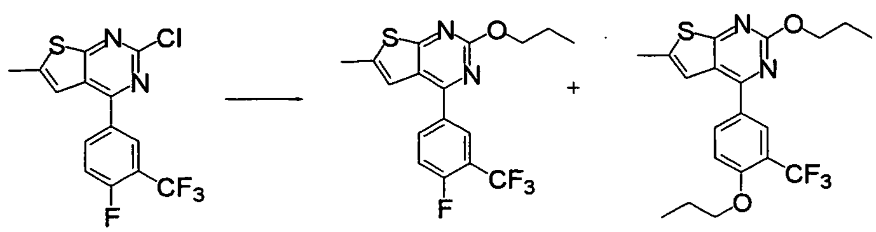

Sodium hydride (0.10 g, 2.50 mmol) was added to a THF (20 mL) solution of propanol (0.15 g, 2.50 mmol) and then a THF (5 mL) solution of 2-chloro-6-methyl-4-[4-fluoro-3-(trifluoromethyl)phenyl]thieno[2,3-d]pyrimidine (0.30 g, 0.866 mmol) was added dropwise thereto at room temperature, followed by heating under reflux for 2 hours. After completion of the reaction, the solvent was removed by evaporation and the obtained residue was dissolved in ethyl acetate. The solution was washed with water and brine and then the solvent was removed by evaporation. The residue was purified on a silica gel column (Kiesel gel 60 manufactured by MERCK, 20% AcOEt-Hex) to obtain 4-[4-fluoro-3-(trifluoromethyl)phenyl]-6-methyl-2-propoxythieno[2,3-d]pyrimidine (0.12 g, 38%) and 6-methyl-2-propoxy-4-[4-propoxy-3-(trifluoromethyl)phenyl]thieno[2,3-d]pyrimidine (0.066 g, 18%).
4-[4-Fluoro-3-(trifluoromethyl)phenyl]-6-methyl-2-propoxythieno[2,3-d]pyrimidine $^1$H-NMR (400MHz, $CDCl_3$) : 1.08(t, 3H, J=7.6Hz), 1.90(sixtet, 2H, J=7.6Hz), 2.59(d, 3H, J=1.2Hz), 4.44(t, 2H, J=7.6Hz), 7.01(d, 1H, J=1.2Hz), 7.36(t, 1H, J=9.6Hz), 8.13(m, 1H), 8.20(d, 1H, J=6.4Hz)
6-Methyl-2-propoxy-4-[4-propoxy-3-(trifluoromethyl)phenyl]thieno[2,3-d]pyrimidine $^1$H-NMR (400MHz, $CDCl_3$) : 1.07 (t, 3H, J=7.6Hz), 1.09(t, 3H, J=7.6Hz), 1.90(sixtet, 2H, J=7.6Hz), 2.58(d, 3H, J=1.2Hz), 4.11(t, 2H, J=7.2Hz), 4.43(t, 2H, J=7.2Hz), 7.06(d, 1H, J=1.2Hz), 7.11(t, 1H, J=8.8Hz), 8.10(dd, 1H, J=2.0Hz, 8.8Hz), 8.18(d, 1H, J=2.0Hz)

Example 13: Production of 2-(isopropylsulfonyloxy)-6-methyl-4-[3-(trifluoromethoxy)phenyl]thieno[2,3-d]pyrimidine (Compound No. 6-163)

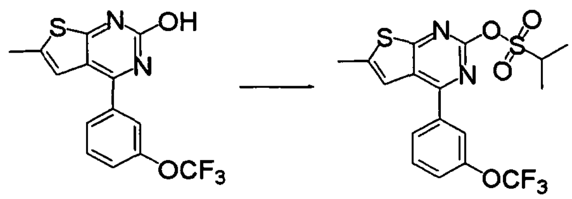

To an acetonitrile (20 mL) solution of 2-hydroxy-6-methyl-4-[3-(trifluoromethyl)phenyl]thieno[2,3-d]pyrimidine (0.30 g, 0.920 mmol) were added isopropylsulfonyl chloride (0.36 g, 2.50 mmol), triethylamine (0.25 g, 2.50 mmol), and trimethylamine hydrochloride (0.01 g) at room temperature and then the whole was stirred at room temperature for 4 hours, followed by heating under stirring at 60°C for 6 hours. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 30% AcOEt-Hex) to obtain 2-(isopropylsulfonyloxy)-6-methyl-4-[3-(trifluoromethoxy)phenyl] thieno[2,3-d]pyrimidine (60.4 mg, 15%).
$^1$H-NMR (400MHz, $CDCl_3$) : 1.63(d, 6H, J=7.2Hz), 2.66(d, 3H, J=1.2Hz), 3.83(7-plet, 1H, J=7.2Hz), 7.22(d, 1H, J=1.2Hz), 7.41(m, 1H), 7.61(t, 1H, J=8.0Hz), 7.81(s, 1H), 7.90(d, 1H, J=8.0Hz).

Example 14: Production of 6-methyl-4-[3-(trifluoromethyl)phenyl]-2-(vinylsulfonyloxy)thieno[2,3-d]pyrimidine (Compound No. 6-150)

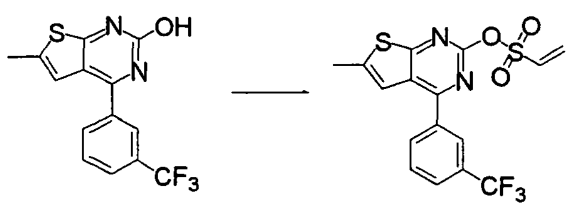

To a methylene chloride (20 mL) solution of 2-hydroxy-6-methyl-4-[3-(trifluoromethyl)phenyl]thieno[2,3-d]pyrimidine (0.30 g, 0.968 mmol) were added 2-chloroethylsulfonyl chloride (0.41 g, 2.50 mmol) and triethylamine (0.25 g, 2.50 mmol) at room temperature and then the whole was stirred at room temperature for 12 hours. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 30% AcOEt-Hex) to obtain 6-methyl-4-[3-(trifluoromethyl) phenyl]-2-(vinylsulfonyloxy)thieno[2,3-d]pyrimidine (0.23 g, 59%).
$^1$H-NMR (400MHz, $CDCl_3$) : 2.67(d, 3H, J=1.2Hz), 6.27(dd, 1H, J=0.8Hz, 10HZ), 6.64(dd, 1H, J=0.8Hz, 16.8HZ), 7.20 (dd, 1H, J=10Hz, 16.8HZ),7.21(d, 1H, J=1.2Hz), 7.71(d, 1H, J=8.0Hz), 7.83(d, 1H, J=8.0Hz), 8.14(d, 1H, J=8.0Hz), 8.19(s, 1H)

Example 15: Production of 2-(chloromethyl)-6-methyl-4-[3-(trifluoromethyl)phenyl]thieno[2,3-d]pyrimidine (Compound No. 6-12)

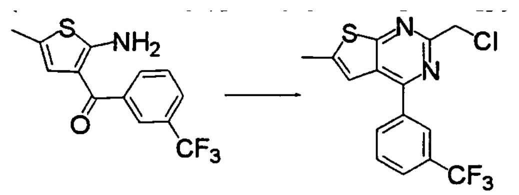

Aluminum chloride (3.65 g, 27.4 mmol) was added to a chloroacetonitrile (15 mL) solution of 2-amino-2-[3-(trifluoromethyl)phenyl]-5-methylthiophene (3.90 g, 13.7 mmol), followed by heating under stirring at 100°C for 2 hours. After completion of the reaction, the reaction liquid was poured into ice-water and extracted with ethyl acetate. The resulting extract solution was washed with water and brine and then the solvent was removed by evaporation. The residue was purified on a silica gel column (Kiesel gel 60 manufactured by MERCK, 10-20% AcOEt-Hex) to obtain 2-(chloromethyl)-6-methyl-4-[3-(trifluoromethyl)phenyl]thieno[2,3-d]pyrimidine (2.92 g, 62%).
$^1$H-NMR (400MHz, $CDCl_3$) :2.67(d, 1H, J=2.0Hz), 4.90(s, 2H), 7.18(d, 1H, J=1.2Hz), 7.64(d, 1H, J=8.0Hz), 7.80(t, 1H, J=8.0Hz), 8.13(d, 1H, J=8.0Hz), 8.20(s, 1H)

Example 16: Production of 6-methyl-2-[(2,2,2-trifluoroethoxy)methyl]-4-[3-(trifluoromethoxy)phenyl]thieno[2,3-d]pyrimidine (Compound No. 6-17)

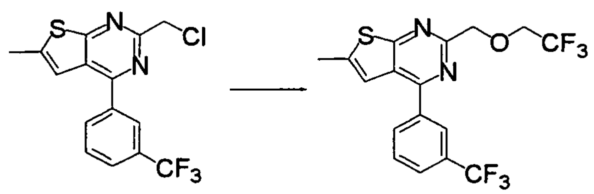

Sodium hydride (0.10 g, 2.50 mmol) was added to a THF (20 mL) solution of 2,2,2-trifluoroethanol (0.25 g, 2.5 mmol) and the whole was stirred at room temperature for 0.5 hour. Then, the mixture was cooled with ice-water and a THF (5 mL) solution of 2-chloromethyl-6-methyl-4-[3-(trifluoromethyl)phenyl]thieno[2,3-d]pyrimidine (0.30 g, 0.875 mmol) was added dropwise thereto. The reaction liquid was warmed to room temperature and then heated under stirring at 60°C for 4 hours. After completion of the reaction, the solvent was removed by evaporation and the obtained residue was dissolved in ethyl acetate. The solution was washed with water and brine and then the solvent was removed by evaporation. The residue was purified on a silica gel column (Kiesel gel 60 manufactured by MERCK, 20% AcOEt-Hex) to obtain 6-methyl-2-[(2,2,2-trifluoroethoxy)methyl]-4-[3-(trifluoromethyl)phenyl]thieno[2,3-d]pyrimidine (0.27 g, 75%).
$^1$H-NMR (400MHz, $CDCl_3$) :2.67(d, 1H, J=2.0Hz), 4.16(q, 2H, J=8.4Hz), 5.05(s, 2H), 7.18(d, 1H, J=1.2Hz), 7.70(d, 1H, J=8.0Hz), 7.80(t, 1H, J=8.0Hz), 8.12(d, 1H, J=8.0Hz), 8.20(s, 1H).
mp : 40-41°C

Example 17: Production of 6-methyl-2-methylthio-4-[4-(trifluoromethyl)phenyl]thieno[2,3-d]pyrimidine (Compound No. 6-171)

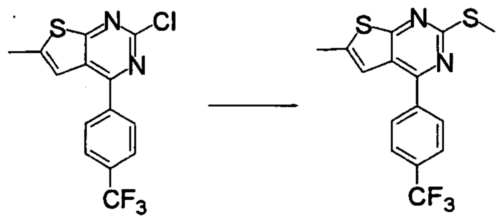

Sodium methanethiolate (0.64 g, 9.13 mmol) was suspended in THF (20 mL) and a THF (5 mL) solution of 2-chloro-6-methyl-4-[4-(trifluoromethyl)phenyl]thieno[2,3-d]pyrimidine (1.50 g, 4.57 mmol) was added dropwise thereto at room temperature, followed by heating under reflux for 7 hours. After completion of the reaction, the reaction liquid was poured into an aqueous sodium bicarbonate solution and extracted with ethyl acetate. The resulting extract solution was washed with water and brine and then the solvent was removed by evaporation. The resulting crystals were washed with hexane to obtain 6-methyl-2-methylthio-4-[4-(trifluoromethyl)phenyl]thieno[2,3-d]pyrimidine (1.24 g, 80%).
$^1$H-NMR (400MHz, $CDCl_3$) : 2.60(d, 3H, J=1.2Hz), 2.68(s, 3H), 7.08(d, 1H, J=1.2Hz), 7.79(d, 2H, J=8.0Hz), 8.03(d, 2H, J=8.0Hz).
mp : 113°C

Example 18: Production of 6-methyl-2-(methylsulfonyl)-4-[4-(trifluoromethyl)phenyl]thieno[2,3-d]pyrimidine (Compound No. 6-173) and 6-methyl-2-(methylsulfinyl)-4-[4-(trifluoromethyl)phenyl]thieno[2,3-d]pyrimidine (Compound No. 6-175)

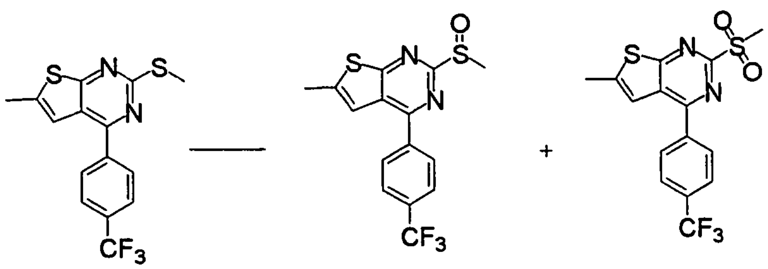

To a methylene chloride (30 mL) solution of 6-methyl-2-(methylthio)-4-[4-(trifluoromethyl)phenyl]thieno[2,3-d] pyrimidine (1.00 g, 2.94 mmol) was added m-chloroperbenzoic acid (0.76 g, 4.41 mmol) at room temperature and then the whole was stirred at room temperature for 7 hours. After completion of the reaction, the reaction liquid was poured into a 1N aqueous sodium hydroxide solution and extracted with ethyl acetate. The resulting extract solution was washed with water and brine and then the solvent was removed by evaporation. The residue was purified on a silica gel column (Kiesel gel 60 manufactured by MERCK, 20% AcOEt-Hex to AcOEt alone) to obtain 6-methyl-2-methylsulfonyl-4-[4-(trifluoromethyl)phenyl]thieno[2,3-d]pyrimidine (0.70 g, 64%) and 6-methyl-2-methylsulfinyl-4-[4-(trifluoromethyl)phenyl]thieno[2,3-d]pyrimidine (0.20 g, 19%).
6-Methyl-2-methylsulfonyl-4-[4-(trifluoromethyl)phenyl]thieno[2,3-d]pyrimidine $^1$H-NMR (400MHz, $CDCl_3$) : 2.74(d, 3H, J=1.2Hz), 3.46(s, 3H), 7.35(d, 1H, J=1.2Hz), 7.85(d, 2H, J=8.0Hz), 8.11(d, 2H, J=8.0Hz).
mp : 153°C
6-Methyl-2-methylsulfinyl-4-[4-(trifluoromethyl)phenyl]thieno[2,3-d]pyrimidine $^1$H-NMR (400MHz, $CDCl_3$) : 2.71(d, 3H, J=1.2Hz), 3.05(s, 3H), 7.28(d, 1H, J=1.2Hz), 7.84(d, 2H, J=8.0Hz), 8.08(d, 2H, J=8.0Hz).
mp : 171°C

Example 19: Production of 6-methyl-2-(methylamino)-4-[4-(trifluoromethyl)phenyl]thieno[2,3-d]pyrimidine (Compound No. 6-168)

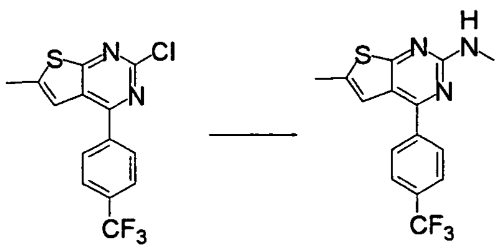

To a methylamine/methanol solution (20 mL) was added dropwise a THF (5 mL) solution of 2-chloro-6-methyl-4-[4-(trifluoromethyl)phenyl]thieno[2,3-d]pyrimidine (0.40 g, 1.22 mmol) at room temperature, followed by stirring for 8 hours. After completion of the reaction, the solvent was removed by evaporation and then the residue was poured into water, followed by extraction with ethyl acetate. The resulting extract solution was washed with water and brine and then the solvent was removed by evaporation. The resulting crystals were washed with methanol to obtain 6-methyl-2-(methylamino)-4-[4-(trifluoromethyl)phenyl]thieno[2,3-d]pyrimidine (0.34 g, 87%).
$^1$H-NMR (400MHz, $CDCl_3$) : 2.51(d, 3H, J=1.2Hz), 3.10(d, 3H, J=~5.2Hz), 5.15(br.s, 1H), 6.89(d, 1H, J=1.2Hz), 7.76 (d, 2H, J=8.0Hz), 7.96(d, 2H, J=8.0Hz).
mp : 233°C

Example 20: Production of 6-methyl-2-[4-(trifluoromethyl)phenyl]-4-[3-(trifluoromethyl)phenyl]thieno[2,3-d]pyrimidine (Compound No. 6-18)

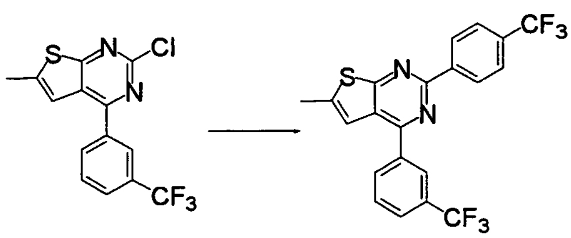

To a toluene (2 mL)-water (1 mL) mixed solution of 2-chloro-6-methyl-4-[3-(trifluoromethyl)phenyl]thieno [2,3-d]pyrimidine (0.30 g, 0.913 mmol) were added 4-(trifluoromethyl)benzeneboronic acid (0.20 g, 1.07 mmol), sodium carbonate (0.22 g, 2.14 mmol), and tetrakis(triphenylphosphine)palladium (0.062 g, 5 mol%), followed by refluxing under a nitrogen stream for 1.5 hours. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 10% AcOEt-Hex) to obtain 6-methyl-2-[4-(trifluoromethyl)phenyl]-4-[3-(trifluoromethyl)phenyl]thieno[2,3-d]pyrimidine (0.22 g, 54%).
$^1$H-NMR (400MHz, $CDCl_3$) :2.70(d, 3H, J=1.2Hz), 7.22(d, 1H, J=1.2Hz), 7.72(d, 1H, J=8.0Hz), 7.77(d, 2H, J=8.4Hz), 7.83(d, 1H, J=8.0Hz), 8.23(d, 1H, J=8.0Hz), 8.29(s, 1H), 8.72(d, 2H, J=8.4Hz).
mp: 134-136°C

Example 21: Production of 6-methyl-4-[3-(trifluoromethyl)phenyl]thieno[2,3-d]pyrimidine-2-carboxylic acid (Compound No. 6-180) and 6-methyl-4-[3-(trifluoromethyl)phenyl]thieno[2,3-d]pyrimidine (Compound No. 6-9)

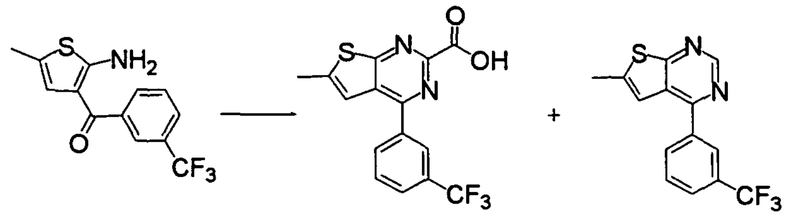

To an ethanol (50 mL) solution of 2-amino-5-methyl-3-[3-(trifluoromethyl)benzoyl]thiophene (4.00 g, 14.0 mmol) were added ammonium acetate (3.24 g, 42.1 mmol) and glyoxylic acid (about 40% aqueous solution, 2.61 g, 14.0 mmol), followed by heating under stirring at 50°C for 24 hours. After completion of the reaction, the solvent was removed by evaporation, the reaction product was poured into water, and precipitated crystals were filtrated. The resulting crystals were washed with ethanol and then dried to obtain 6-methyl-4-[3-(trifluoromethyl)phenyl]thieno[2,3-d] pyrimidine-2-carboxylic acid (0.60 g, 13%).
$^1$H-NMR (400MHz, $CDCl_3$) :2.64(d, 3H, J=1.2Hz), 7.39(d, 1H, J=1.2Hz), 7.84(d, 1H, J=8.0Hz), 7.95(d, 1H, J=8.0Hz), 8.22(s, 1H), 7.26(d, 1H, J=8.0Hz).
mp: 226-227°C (dec.)

Furthermore, the filtrate was washed with water and brine and then the solvent was removed by evaporation. The residue was purified on a silica gel column (Kiesel gel 60 manufactured by MERCK, 10% AcOEt-Hex) to obtain 6-methyl-4-[3-(trifluoromethyl)phenyl]thieno[2,3-d]pyrimidine (1.10 g, 27%).
$^1$H-NMR (400MHz, $CDCl_3$) :2.67(d, 3H, J=1.2Hz), 7.19(d, 1H, J=1.2Hz), 7.70(d, 1H, J=8.0Hz), 7.80(d, 1H, J=8.0Hz), 8.12(d, 1H, J=8.0Hz), 8.21(s, 1H), 9.09(s, 1H).
mp: 70-72°C

Example 22: Production of ethyl 6-methyl-4-[3-(trifluoromethyl)phenyl]thieno[2,3-d]pyrimidine-2-carboxylate (Compound No. 6-181)

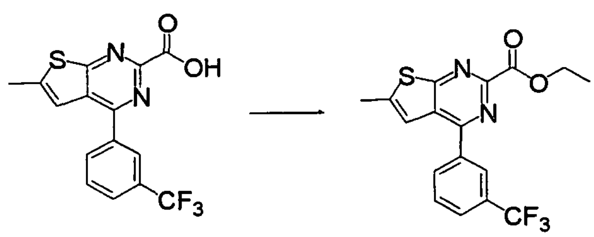

Thionyl chloride (2 mL) was added to 6-methyl-4-[3-(trifluoromethyl)phenyl]thieno[2,3-d]pyrimidine-2-carboxylic acid (0.40 g, 1.18 mmol), followed by heating at 80°C under stirring for 1 hour. After completion of the reaction, the reaction liquid was evaporated and half of the obtained residue was added dropwise to a cooled mixed solution of ethanol (10 mL) and triethylamine (0.5 mL). After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 10% AcOEt-Hex) to obtain ethyl 6-methyl-4-[3-(trifluoromethyl)phenyl]thieno[2,3-d]pyrimidine-2-carboxylate (41.2 mg).
$^1$H-NMR (400MHz, $CDCl_3$) :1.54(t, 3H, J=7.2Hz), 2.72(d, 3H, J=1.2Hz), 4.59(q, 2H, J=7.2Hz), 7.71(t, 1H, J=8.0Hz), 7.82(d, 1H, J=8.0Hz), 8.18(d, 1H, J=8.0Hz), 8.23(s, 1H)

At that time, it was impossible to confirm the proton peak of the thiophene ring since the peak seemed to be included in the chloroform peak.
mp: 134°C

Example 23: Production of 6-methyl-4-[3-(trifluoromethyl)phenyl]thieno[2,3-d]pyrimidine-2-carboxamide (Compound No. 6-182)

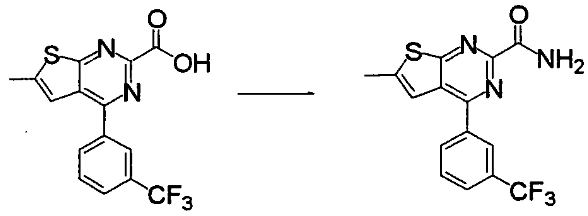

Thionyl chloride (2 mL) was added to 6-methyl-4-[3-(trifluoromethyl)phenyl]thieno[2,3-d]pyrimidine-2-carboxylic acid (0.40 g, 1.18 mmol), followed by heating at 80°C under stirring for 1 hour. After completion of the reaction, the reaction liquid was evaporated and half of the obtained residue was added dropwise to a cooled aqueous ammonia (10 mL). After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 10% AcOEt-Hex) to obtain 6-methyl-4-[3-(trifluoromethyl)phenyl]thieno[2,3-d]pyrimidine-2-carboxamide (82.2 mg).
$^1$H-NMR (400MHz, $CDCl_3$) :2.72(d, 3H, J=1.2Hz), 5.85(br.s, 1H), 7.73(t, 1H, J=8.0Hz), 7.84(d, 1H, J=8.0Hz), 8.15(d, 1H, J=8.0Hz), 8.20(s, 1H)
At that time, it was impossible to confirm the proton peak of the thiophene ring since the peak seemed to be included in the chloroform peak.
mp: 209°C

Example 24: Production of 2-cyano-6-methyl-4-[3-(trifluoromethyl)phenyl]thieno[2,3-d]pyrimidine (Compound No. 6-10)

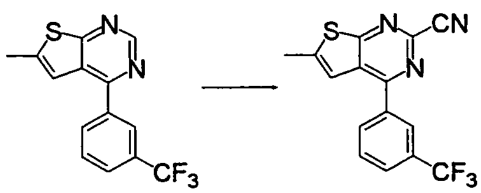

To a methylene chloride (20 mL) solution of 6-methyl-4-[3-(trifluoromethyl)phenyl]thieno[2,3-d]pyrimidine (0.93 g, 3.16 mmol) was added m-chloroperbenzoic acid (0.82 g, 4.74 mmol) and then the whole was stirred at room temperature for 20 hours. After completion of the reaction, the reaction liquid was evaporated and the obtained residue was poured into an aqueous sodium bicarbonate solution, followed by extraction with ethyl acetate. The resulting extract solution was washed with water and brine and then the solvent was removed by evaporation. The residue was purified on a silica gel column (Kiesel gel 60 manufactured by MERCK, 20-50% AcOEt-Hex) to obtain an N-oxide (0.56 g, 57%). Upon confirmation on $^1$H-NMR, the product was found to be a mixture of positional isomers.
Subsequently, trimethylsilyl cyanide (0.54 g, 5.41 mmol) and triethylamine (0.36 g, 3.60 mmol) were added to an acetonitrile (10 mL) solution of the obtained N-oxide (0.56 g, 1.80 mmol), followed by refluxing for 7 hours. After completion of the reaction, the reaction liquid was evaporated and the obtained residue was poured into an aqueous sodium bicarbonate solution, followed by extraction with methylene chloride. The resulting extract solution was washed with water and brine and then the solvent was removed by evaporation. The residue was purified on a silica gel column (Kiesel gel 60 manufactured by MERCK, 10% AcOEt-Hex) to obtain 2-cyano-6-methyl-4-[3-(trifluoromethyl)phenyl]thieno[2,3-d]pyrimidine (0.20 g, 35%).
$^1$H-NMR (400MHz, $CDCl_3$) :2.75(d, 3H, J=1.2Hz), 7.31(d, 1H, J=1.2Hz), 7.74(t, 1H, J=8.0Hz), 7.85(d, 1H, J=8.0Hz), 8.15(d, 1H, J=8.0Hz), 8.21(s, 1H).
mp: 121-122°C

Example 25: Production of 2-butyl-6-methyl-4-[3-(trifluoromethoxy)phenyl]thieno[2,3-d]pyrimidine (Compound No. 6-11)

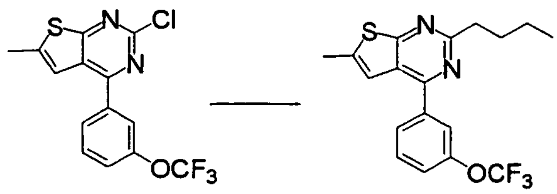

To a xylene (20 mL) solution of 2-chloro-6-methyl-4-[3-(trifluoromethoxy)phenyl]thieno[2,3-d]pyrimidine (0.50 g, 1.45 mmol) were added tetra-n-butyltin (0.55 g, 1.60 mmol) and bis(triphenylphosphine)palladium dichloride (catalyst amount 0.05 g), followed by heating under stirring at 110°C under a nitrogen stream for 14 hours. After completion of the reaction, the reaction product was poured into water. Insoluble matter was removed by filtration through Celite (trade name) and the filtrate was dissolved in ethyl acetate. The solution was washed with water and brine and then the solvent was removed by evaporation. The residue was purified on a silica gel column (Kiesel gel 60 manufactured by MERCK, 10% AcOEt-Hex) to obtain 2-n-butyl-6-methyl-4-[3-(trifluoromethoxy)phenyl]thieno[2,3-d]pyrimidine (0.30 g, 59%).
$^1$H-NMR (400MHz, $CDCl_3$) : 0.98(t, 3H, J=7.2Hz), 1.47(sextet, 2H, J=7.2Hz), 1.91(m, 2H), 2.63(d, 3H, J=1.2Hz), 3.09 (t, 2H, J=7.2Hz), 7.12(d, 1H, J=1.2Hz), 7.37(dt, 1H, J=8.0Hz, 1.2Hz), 7.58(t, 1H, J=8.0Hz), 7.78(s, 1H), 7.86(dt, 1H, J=8.0Hz, 1.2Hz).

Example 26: Production of 6-methyl-4-[3-(trifluoromethyl)phenoxy]-2-(3,3,3-trifluoropropyl)thieno[2,3-d]pyrimidine (Compound No. 6-130)

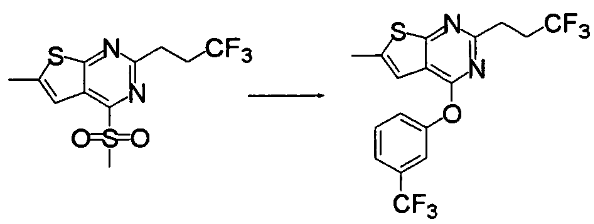

To a DMF (5 mL) solution of 6-methyl-4-(3-methylsulfonyl)-2-(3,3,3-trifluoropropyl)thieno[2,3-d]pyrimidine (0.30 g, 0.925 mmol) were added 3-(trifluoromethyl)phenol (0.15 g, 0.925 mmol) and potassium carbonate (0.19 g, 1.39 mmol), followed by stirring at room temperature for 1 day. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 10% AcOEt-Hex) to obtain 6-methyl-4-[3-(trifluoromethyl)phenoxy]-2-(3,3,3-trifluoropropyl)thieno [2,3-d]pyrimidine (0.26 g, 68%).
$^1$H-NMR (400MHz, $CDCl_3$) :2.5-2.6(m, 2H), 2.64(d, 3H, J=1.2Hz), 3.09(m, 2H), 7.12(q, 1H, J=1.2Hz), 7.4-7.6(m, 4H).
mp: 85°C

Example 27: Production of 6-methyl-2-(2,2,2-trifluoroethoxy)-4-[3-(trifluoromethyl)phenoxy]thieno[2,3-d]pyrimidine (Compound No. 6-129)

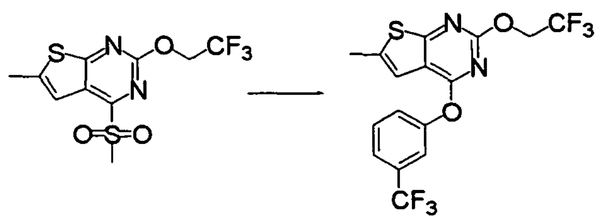

To a DMF (20 mL) solution of 6-methyl-4-(methylsulfonyl)-2-(2,2,2-trifluoroethoxy)thieno[2,3-d]pyrimidine (0.50 g, 1.53 mmol) and potassium carbonate (0.23 g, 1.68 mmol) was added 3-(trifluoromethyl)phenol (0.27 g, 1.68 mmol), followed by stirring at room temperature for 1.5 hours. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 20% AcOEt-Hex) to obtain 6-methyl-2-(2,2,2-trifluoroethoxy)-4-[3-(trifluoromethyl)phenoxy]thieno [2,3-d]pyrimidine (0.58 g, 94%).
$^1$H-NMR (400MHz, $CDCl_3$) : 2.60(d, 3H, J=1.2Hz), 4.66(q, 2H, J=8.4Hz), 7.07(q, 1H, J=1.2Hz), 7.44(m, 1H), 7.51(m, 1H), 7.57(m, 2H).
mp: 124-125°C

Example 28: Production of 5-methyl-2-ureido-3-[3-(trifluoromethyl)benzoyl]thiophene

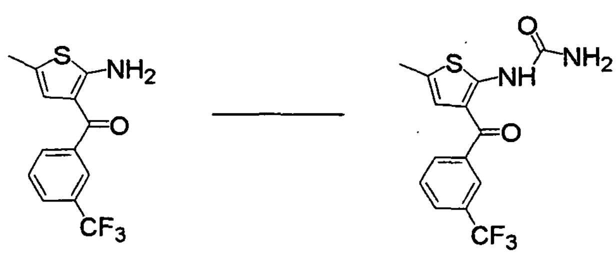

To an acetic acid (25 mL) solution of2-amino-5-methyl-3-[3-(trifluoromethyl)benzoyl]thiophene (2.50 g, 8.76 mmol) was slowly added dropwise a water (60 mL) solution of sodium cyanate (1.03 g, 15.8 mmol). After completion of the reaction, the resulting crystals were filtrated off, washed with water, and then dried to obtain 5-methyl-2-ureido-3-[3-(trifluoromethyl)benzoyl]thiophene (2.56 g, 89%).
$^1$H-NMR (400MHz, $CDCl_3$) : 2.26(d, 3H, J=1.2Hz), 6.41(d, 1H, J=1.2Hz), 6.95(br.s, 2H), 7.57(t, 1H, J=8.0Hz), 7.73(d, 1H, J=8.0Hz), 7.83(d, 1H, J=8.0Hz), 7.91(s, 1H). mp: 155°C (dec.)

Example 29: Production of 2-chloro-6-methyl-4-[3-(trifluoromethyl)phenyl]thieno[3,2-d]pyrimidine (Compound No. 5-34) 29-1) Production of methyl 5-methyl-3-ureidothiophene-2-carboxylate

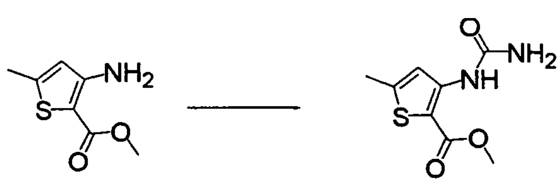

To an acetic acid (40 mL) solution of methyl 2-amino-5-methyl-thiophene-2-carboxylate (5.00 g, 29.2 mmol, synthesized with reference to JP-A-5-117263) was slowly added dropwise a water (20 mL) solution of sodium cyanate (5.78 g, 87.6 mmol). After completion of the reaction, the resulting crystals were filtrated off, washed with water, and then dried to obtain methyl 5-methyl-3-ureidothiophene-2-carboxylate (4.54 g, 73%).
$^1$H-NMR (400MHz, $CDCl_3$) : 2.47(d, 3H, J=1.2Hz), 3.84(s, 3H), 4.68(br.s, 2H), 7.71(d, 1H, J=1.2Hz), 9.50(br.s, 1H).

29-2) Production of 2,4-dihydroxy-6-methylthieno[3,2-d]pyrimidine (Compound No. 4-1)

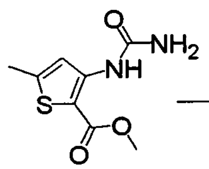

To an ethanol (50 mL) solution of methyl 5-methyl-3-ureidothiophene-2-carboxylate (4.54 g, 19.2 mmol) was added potassium hydroxide (2.54 g, 43.8 mmol), followed by heating under reflux at 80°C for 3 hours. After completion of the reaction, the resulting crystals were filtrated off, washed with ethanol, and then a solution obtained by dissolving the resulting crystals in water was acidified with concentrated hydrochloric acid. The precipitated crystals were filtrated off, washed with water, and then dried to obtain 2,4-dihydroxy-6-methylthieno[3,2-d]pyrimidine (2.72 g, 70%).
mp: >300°C

29-3) Production of 2,4-dichloro-6-methylthieno[3,2-d]pyrimidine

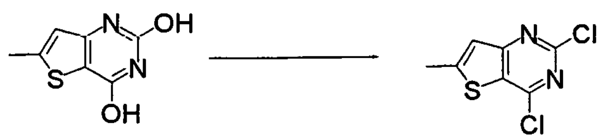

Phosphorus oxychloride (6.85 g, 44.8 mmol) was added to a DMF (1 mL) solution of 2,4-dihydroxy-6-methylthieno[3,2-d]pyrimidine (2.72 g, 14.9 mmol), followed by heating under reflux at 100°C for 2 hours. After completion of the reaction, the reaction liquid was poured into ice-water and precipitated crystals were collected by filtration. After the crystals were dissolved in ethyl acetate, the solution was washed with water and brine and then the solvent was removed by evaporation to obtain 2,4-dichloro-6-methylthieno[3,2-d]pyrimidine (3.21 g, 98%).
$^1$H-NMR (400MHz, $CDCl_3$) : 2.73(d, 3H, J=1.2Hz), 7.20(q, 1H, J=1.2Hz).
mp: 152-153°C

29-4) Production of 2-chloro-6-methyl-4-[3-(trifluoromethyl)phenyl]thieno[3,2-d]pyrimidine (Compound No. 5-34)

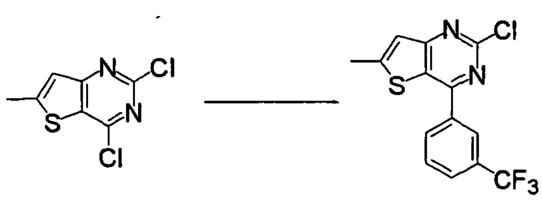

To a toluene (10 mL)-water (1 mL) mixed solution of 2,4-dichloro-6-methylthieno[3,2-d]pyrimidine (1.00 g, 4.56 mmol) were added 3-(trifluoromethyl)benzeneboronic acid (0.87 g, 4.56 mmol), sodium carbonate (0.97 g, 9.13 mmol), and tetrakis(triphenylphosphine)palladium (0.10 g, 5 mol%), followed by heating under reflux under a nitrogen stream for 2 hours. After completion of the reaction, the reaction liquid was poured into water and extracted with ethyl

acetate. The resulting extract solution was washed with water and brine and then the solvent was removed by evaporation. The residue was purified on a silica gel column (Kiesel gel 60 manufactured by MERCK, 10% AcOEt-Hex) to obtain 2-chloro-6-methyl-4-[3-(trifluoromethyl)phenyl]thieno[3,2-d]pyrimidine (1.33 g, 89%).
$^1$H-NMR (400MHz, $CDCl_3$) : 2.75(d, 3H, J=1.2Hz), 7.26(d, 1H, J=1.2Hz), 7.72(t, 1H, J=8.0Hz), 7.85(d, 1H, J=8.0Hz), 8.36(d, 1H, J=8.0Hz), 8.43(s, 1H).
mp : 174-176°C (dec.)

Example 30: Production of 2-isopropoxy-6-methyl-4-[3-(trifluoromethyl)phenyl]thieno[3,2-d]pyrimidine (Compound No. 6-231)

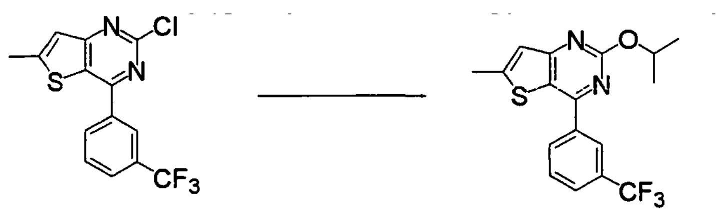

To a THF (10 mL) solution of isopropanol (0.15 g, 2.50 mmol) was added sodium hydride (0.10 g, 2.50 mmol), followed by stirring at room temperature for 0.5 hour. Then, the mixture was cooled with ice-water and a THF (5 mL) solution of 2-chloro-6-methyl-4-[3-(trifluoromethyl)phenyl]thieno[3,2-d]pyrimidine (0.40 g, 1.22 mmol) was added dropwise thereto. The reaction liquid was warmed to room temperature and then heated under stirring at 60°C for 2 hours. After completion of the reaction, the solvent was removed by evaporation and the obtained residue was dissolved in ethyl acetate. The solution was washed with water and brine and then the solvent was removed by evaporation. The residue was purified on a silica gel column (Kiesel gel 60 manufactured by MERCK, 20% AcOEt-Hex) to obtain 2-isopropoxy-6-methyl-4-[3-(trifluoromethyl)phenyl]thieno[3,2-d]pyrimidine (0.16 g, 37%).
$^1$H-NMR (400MHz, $CDCl_3$) :1.47(d, 6H, J=6.0Hz), 2.68(d, 3H, J=1.2Hz), 5.45(7-plet, 1H, J=6.0Hz), 7.11(d, 1H, J=1.2Hz), 7.67(t, 1H, J=8.0Hz), 7.79(d, 1H, J=8.0Hz), 8.35(d, 1H, J=8.0Hz), 8.44(s, 1H).
mp: 106°C

Production Examples of a part of intermediates are shown below as Reference Examples.

Reference Example 1: Production of ethyl 3-(difluoromethylthio)benzoate

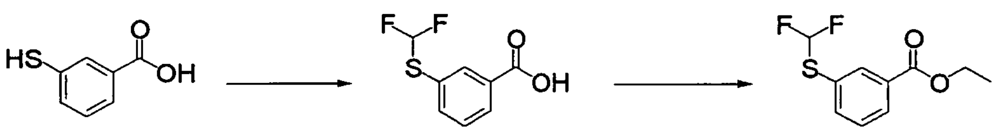

To a dioxane (150 mL) solution of 3-mercaptobenzoic acid (25.0 g, 0.162 mol) were added water (50 mL) and sodium hydroxide (40 g, 1.0 mol), followed by heating under stirring at 60°C. Therein was bubbled Flon 22 (difluorochloromethane) gas for 9 hours. Fron 22 was bubbled in at such a rate that it was gently refluxed by means of a gas trap cooled with dry ice-acetone, and during the time, each 10 g of sodium hydroxide was further added twice. After completion of the reaction, dioxane was removed by evaporation and then the residue was acidified by adding concentrated hydrochloric acid. The resulting residue was dissolved in ethyl acetate and insoluble matter was filtrated through Celite (trade name). Thereafter, the filtrate was washed with water and brine and then the solvent was removed by evaporation. Based on NMR inspection of the residue, the ratio of 3-(difluoromethylthio)benzoic acid to the starting material was found to be about 9:1.

Subsequently, thionyl chloride (28.4 g, 0.239 mol) and toluene (30 mL) were added to crude 3-(difluoromethylthio)benzoic acid (32.5 g) obtained above, followed by heating under stirring at 100°C for 1 hour. After completion of the reaction, the solvent was removed by evaporation to obtain a crude acid chloride.

Then, the acid chloride obtained above was added to a mixed solution of ethanol (100 mL) and triethylamine (24.1 g, 0.239 mol) cooled with ice-water, followed by stirring at room temperature for 3 hours. After completion of the reaction, the solvent was removed by evaporation and the obtained residue was dissolved in ethyl acetate. The solution

was washed with water and brine and then the solvent was removed by evaporation. The residue was purified on a silica gel column (Kiesel gel 60 manufactured by MERCK, 2% AcOEt-Hex) to obtain ethyl 3-(difluoromethylthio)benzoate (30.7 g, 84%).
$^1$H-NMR (400MHz, $CDCl_3$) : 1.41(t, 3H, J=7.2Hz), 4.40(q, 2H, J=7.2Hz), 6.86(t, 1H, J=56.8Hz), 7.48(t, 1H, J=8.0Hz), 7.77(dt, 1H, J=8.0Hz, 1.6Hz), 8.10(dt, 1H, J=8.0Hz, 1.6Hz), 8.25(t, 1H, J=1.6Hz).

Reference Example 2: Production of ethyl 4-chloro-3-ethyl-1-methylpyrazole-5-carboxylate

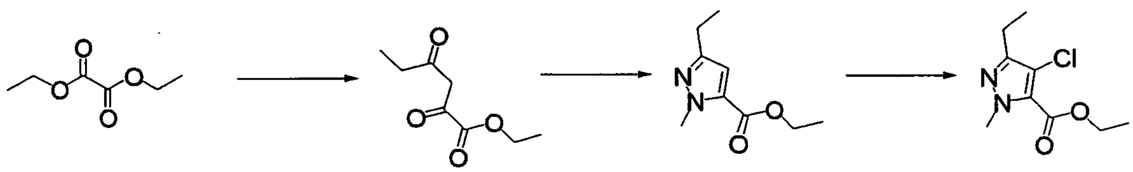

A toluene (150 mL) solution of sodium ethoxide (22.7 g, ca. 90%, 0.30 mol) cooled to -20°C was vigorously stirred and a mixed solution of methyl ethyl ketone (21.6 g, 0.30 mol) and diethyl oxalate (43.8 g, 0.30 mol) was added dropwise thereto. After completion of the reaction, the reaction liquid was neutralized with concentrated hydrochloric acid and washed with water and then the solvent was removed to obtain a crude ketoester compound (53.0 g).
Subsequently, the crude ketoester compound obtained above was added dropwise to a toluene (200 mL) solution of methylhydrazine (15.5 g, 0.34 mol) cooled to -20°C. After completion of the reaction, the reaction liquid was washed with water and the solvent was removed by evaporation to obtain crude ethyl 3-ethyl-1-methylpyrazole-5-carboxylate (38.4 g).
Furthermore, chlorine (16.4 g, 0.21 mmol) separately weighed was bubbled into a methanol (150 mL) solution of crude ethyl 3-ethyl-1-methylpyrazole-5-carboxylate obtained above, the chlorine being spontaneously vaporized. After completion of the reaction, the solvent was removed by evaporation and the residue was purified on a silica gel column (Kiesel gel 60 manufactured by MERCK, 10% AcOEt-Hex) to obtain ethyl 4-chloro-3-ethyl-1-methylpyrazole-5-carboxylate (41.0 g).

Reference Example 3: Production of 2-amino-5-methylthiophene-3-carboxamide

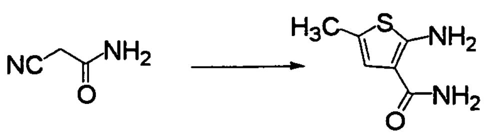

To a DMF (100 mL) solution of cyanoacetamide (42.0 g, 0.5 mol), sulfur (16.0 g, 0.5 mol), and triethylamine (50.5 g, 0.5 mol) was slowly added dropwise an ethanol (15 mL) solution of propionaldehyde (31.9 g, 0.55 mol) at room temperature (inner temperature rose to 70°C), followed by heating under stirring at 60°C for 1.5 hours. After completion of the reaction, the reaction mixture was poured into water and extracted with ethyl acetate. The resulting extract solution was washed with water and brine and then the solvent was removed by evaporation. The residue was washed with methylene chloride to obtain an objective product (42.4 g). The filtrate was purified on a silica gel column (Kiesel gel 60 manufactured by MERCK, 20% AcOEt-Hex) to obtain 2-amino-5-methylthiophene-3-carboxamide (52.6 g, 67%).
$^1$H-NMR (400MHz, $CDCl_3$) : 2.27(d, 3H, J=1.2Hz), 5.32(br s, 2H), 6.01(br.s, 2H), 6.33(q, 1H, J=1.2Hz).
mp: 140-141°C

Reference Example 4: Production of 5-methyl-2-[(4,4,4-trifluorobutyryl)amino]thiophene-3-carboxamide

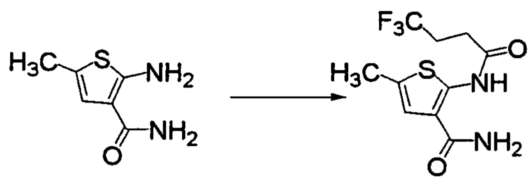

A triethylamine (9.70 g, 96.0 mmol) and ethyl acetate (100 mL) solution of 2-amino-5-methylthiophene-3-carboxamide (10.0 g, 64.0 mmol) was cooled with ice-water, and 4,4,4-trifluorobutyryl chloride (10.3 g, 64.0 mmol) separately prepared was slowly added dropwise thereto. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 20-40% AcOEt-Hex) to obtain 5-methyl-2-[(4,4,4-trifluorobutyryl)amino]thiophene-3-carboxamide (6.58 g, 37%) in total.
$^1$H-NMR (400MHz, $CDCl_3$) : 2.40(d, 3H, J=1.2Hz), 2.5-2.6(m, 2H), 2.74(m, 2H), 5.64(br, 2H), 6.57(d, 1H, J=1.2Hz).
mp: 151-153°C

Reference Example 5: Production of 4-hydroxy-6-methyl-2-(3,3,3-trifluoropropyl)thieno[2,3-d]pyrimidine

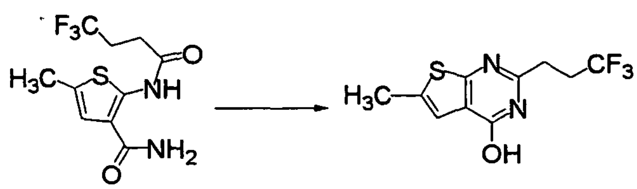

Potassium hydroxide (3.92 g, 67.7 mmol) was added to an ethanol (50 mL) solution of 5-methyl-2-[(4,4,4-trifluorobutyryl)amino]thiophene-3-carboxamide (6.33 g, 22.6 mmol), followed by heating under reflux at 80°C for 7 hours. After completion of the reaction, the reaction liquid was evaporated under reduced pressure. The residue was poured into water and then insoluble matter was extracted with ethyl acetate. The resulting aqueous layer was acidified with concentrated hydrochloric acid and precipitated crystals were filtrated off. Then, the resulting crystals were washed with water and dried to obtain 4-hydroxy-6-methyl-2-(3,3,3-trifluoropropyl)thieno[2,3-d]pyrimidine (4.78 g, 81%).
$^1$H-NMR (400MHz, $CDCl_3$) : 2.55(d, 3H, J=1.2Hz), 2.74(m, 2H), 3.04(m, 2H), 7.12(d, 1H, J=1.2Hz), 12.23(br.s, 1H).
mp: 267-269°C (dec.)

Reference Example 6: Production of 4-chloro-6-methyl-2-(3,3,3-trifluoropropyl)thieno[2,3-d]pyrimidine

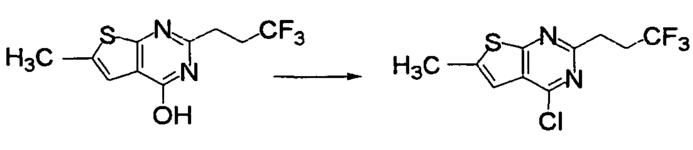

Phosphorus oxychloride (16.1 g, 0.105 mol) was added to a DMF (6 mL) solution of 4-hydroxy-6-methyl-2-(3,3,3-trifluoropropyl)thieno[2,3-d]pyrimidine (5.53 g, 21.1 mmol), followed by heating under reflux at 100°C for 2 hours. After completion of the reaction, the reaction liquid was poured into ice-water and precipitated crystals were collected by filtration. After the resulting crystals were dissolved in ethyl acetate, the solution was washed with water and brine and then the solvent was removed by evaporation. The residue was purified on a silica gel column (Kiesel gel 60 manufactured by MERCK, 10% AcOEt-Hex) and the resulting crystals were washed with hexane to obtain

4-chloro-6-methyl-2-(3,3,3-trifluoropropyl)thieno[2,3-d]pyrimidine (2.10 g, 35%).

Reference Example 7: Production of 6-methyl-4-(methylthio)-2-(3,3,3-trifluoropropyl)thieno[2,3-d]pyrimidine

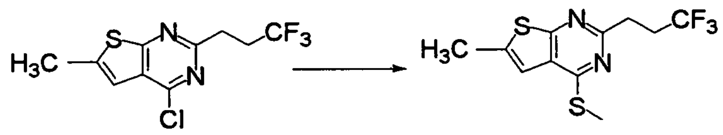

To a THF (30 mL) solution of 4-chloro-6-methyl-2-(3,3,3-trifluoropropyl)thieno[2,3-d]pyrimidine (2.10 g, 7.48 mmol) was added a 15% aqueous sodium methyl mercaptan solution (5.23 g, 11.2 mmol), followed by heating under reflux for 3 hours. After completion of the reaction, the reaction liquid was poured into water and extracted with ethyl acetate. The resulting extract solution was washed with water and brine and then the solvent was removed by evaporation. The residue was purified on a silica gel column (Kiesel gel 60 manufactured by MERCK, 10% AcOEt-Hex) to obtain 6-methyl-4-(methylthio)-2-(3,3,3-trifluoropropyl)thieno[2,3-d]pyrimidine (1.80 g, 82%).
$^1$H-NMR (400MHz, $CDCl_3$) : 2.59(d, 3H, J=1.2Hz), 2.67(s, 3H), 2.7-2.8(m, 2H), 3.23(m, 2H), 6.93(q, 1H, J=1.2Hz).
mp: 81-83°C

Reference Example 8: Production of 6-methyl-4-methylsulfonyl-2-(3,3,3-trifluoropropyl)thieno[2,3-d]pyrimidine

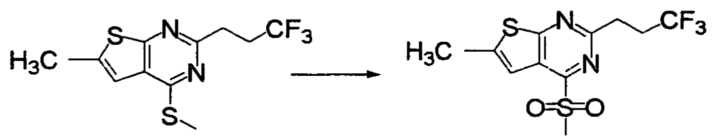

To a methylene chloride (20 mL) solution of 6-methyl-4-methylthio-2-(3,3,3-trifluoropropyl)thieno[2,3-d]pyrimidine (1.60 g, 5.47 mmol) was added m-chloroperbenzoic acid (2.83 g, 16.4 mmol), followed by stirring at room temperature for 1 day. After completion of the reaction, precipitated crystals were filtrated off and the filtrate was poured into water, followed by extraction with ethyl acetate. The resulting extract solution was washed with water and brine and then the solvent was removed by evaporation. The residue was purified on a silica gel column (Kiesel gel 60 manufactured by MERCK, 20% AcOEt-Hex) to obtain 6-methyl-4-(methylsulfonyl)-2-(3,3,3-trifluoropropyl)thieno[2,3-d]pyrimidine (1.17 g, 65%).
$^1$H-NMR (400MHz, $CDCl_3$) : 2.70(d, 3H, J=1.2Hz), 2.7-2.8(m, 2H), 3.38(s, 3H), 3.38(m, 2H), 7.65(q, 1H, J=1.2Hz).
mp: 60-61°C

Reference Example 9: Production of ethyl 2-amino-5-methylthiophene-3-carboxylate

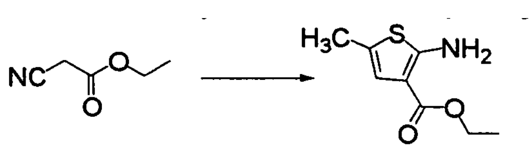

To a DMF (100 mL) solution of ethyl cyanoacetate (56.6 g, 0.5 mol), sulfur (16.0 g, 0.5 mol), and triethylamine (50.5 g, 0.5 mol) was slowly added dropwise an ethanol (15 mL) solution of propionaldehyde (31.9 g, 0.55 mol) at room temperature (inner temperature rose to 60°C), followed by heating under stirring at 60°C. After completion of the reaction, the reaction mixture was poured into water and extracted with ethyl acetate. The resulting extract solution was washed with water and brine and then the solvent was removed by evaporation. The residue was purified on a silica gel column (Kiesel gel 60 manufactured by MERCK, 20% AcOEt-Hex) to obtain ethyl 2-amino-5-methylthiophene-3-carboxylate (81.8 g, 89%).

$^1$H-NMR (400MHz, $CDCl_3$) : 1.33(t, 3H, J=7.2Hz), 2.26(d, 3H, J=1.2Hz), 4.25(q, 2H, J=7.2Hz), 5.76(br.s, 2H), 6.61(q, 1H, J=1.2Hz).

Reference Example 10: Production of ethyl 5-methyl-2-ureidothiophene-3-carboxylate

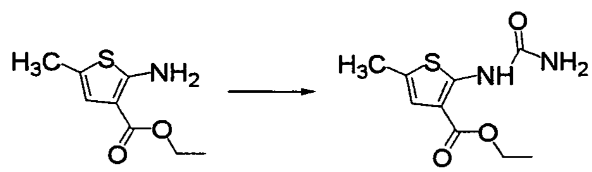

To an acetic acid (80 mL) solution of ethyl 2-amino-5-methylthiophene-3-carboxylate (10.0 g, 54.0 mmol) was slowly added dropwise a water (60 mL) solution of sodium cyanate (7.01 g, 0.108 mol). After completion of the reaction, the resulting crystals were filtrated off, washed with water, and then dried to obtain ethyl 5-methyl-2-ureidothiophene-3-carboxylate (7.60 g, 62%).
$^1$H-NMR (400MHz, $CDCl_3$) : 1.36(t, 3H, J=7.2Hz), 2.35(d, 3H, J=1.2Hz), 4.29(q, 2H, J=7.2Hz), 4.81(br.s, 2H), 6.78(d, 1H, J=1.2Hz), 10.36(br.s, 1H).
mp: 198-199°C (dec.)

Reference Example 11: Production of 2,4-dihydroxy-6-methylthieno[2,3-d]pyrimidine

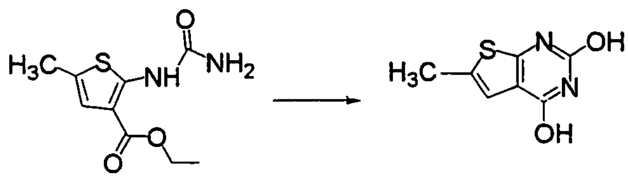

To an ethanol (100 mL) solution of ethyl 5-methyl-2-ureidothiophene-3-carboxylate (7.60 g, 33.3 mmol) was added potassium hydroxide (5.80 g, 0.10 mol), followed by heating under reflux at 80°C for 3 hours. After completion of the reaction, the resulting crystals were filtrated off, washed with ethanol, and then a solution obtained by dissolving the obtained crystals in water was acidified with concentrated hydrochloric acid. Precipitated crystals were filtrated off, washed with water, and then dried to obtain 2,4-dihydroxy-6-methylthieno[2,3-d]pyrimidine (3.60 g, 59%).
$^1$H-NMR (400MHz, $CDCl_3$) : 2.37(d, 3H, J=1.2Hz), 6.82(d, 1H, J=1.2Hz), 11.5(br.s, 1H), 11.7(br.s, 1H).
mp: >300°C

Reference Example 12: Production of 2,4-dichloro-6-methylthieno[2,3-d]pyrimidine

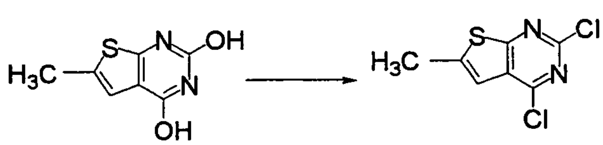

Phosphorus oxychloride (28.1 g, 0.184 mol) was added to a DMF (10 mL) solution of 2,4-dihydroxy-6-methylthieno[2,3-d]pyrimidine (6.70 g, 36.8 mmol), followed by heating under reflux at 100°C for 2.5 hours. After completion of the reaction, the reaction liquid was poured into ice-water and precipitated crystals were collected by filtration. After the resulting crystals were dissolved in ethyl acetate, the solution was washed with water and brine and then the solvent was removed by evaporation to obtain 2,4-dichloro-6-methylthieno[2,3-d]pyrimidine (6.40 g, 79%).

$^1$H-NMR (400MHz, $CDCl_3$) : 2.65(d, 3H, J=1.2Hz), 7.07(q, 1H, J=1.2Hz).
mp: 145-146°C

Reference Example 13: Production of 2-chloro-6-methyl-4-(methylthio)thieno[2,3-d]pyrimidine

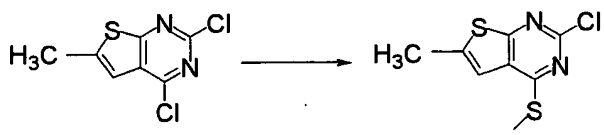

To a THE (50 mL) solution of 2,4-dichloro-6-methylthieno[2,3-d]pyrimidine (3.80 g, 17.3 mmol) was added a 15% aqueous sodium methyl mercaptan solution (9.71 g, 20.8 mmol), followed by heating under reflux for 4 hours. After completion of the reaction, the precipitated salt was filtrated off and the filtrate was poured into water and extracted with ethyl acetate. The resulting extract solution was washed with water and brine and then the solvent was removed by evaporation. A part of the product was purified on a silica gel column (Kiesel gel 60 manufactured by MERCK, 20% AcOEt-Hex) to obtain 2-chloro-6-methyl-4-(methylthio)thieno[2,3-d]pyrimidine. The remainder was used in the next reaction without purification.
$^1$H-NMR (400MHz, $CDCl_3$) : 2.60(d, 3H, J=1.2Hz), 2.70(s, 3H), 6.94(q, 1H, J=1.2Hz).
mp: 111-112°C

Reference Example 14: Production of 6-methyl-4-(methylthio)-2-(2,2,2-trifluoroethoxy)thieno[2,3-d]pyrimidine

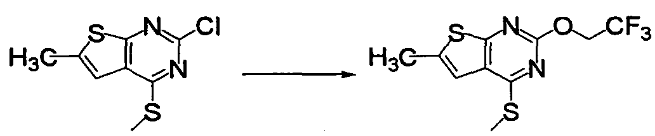

Sodium hydride (0.87 g, 21.8 mmol) was added to a THF (50 mL) solution of 2,2,2-trifluoroethanol (2.18 g, 21.8 mmol) and the whole was stirred at room temperature for 0.5 hour. Thereto was added crude 2-chloro-6-methyl-4-(methylthio)thieno[2,3-d]pyrimidine (3.35 g, 14.5 mmol) obtained in Reference Example 13, followed by heating under stirring at 60°C for 6 hours. After completion of the reaction, the reaction liquid was poured into water and extracted with ethyl acetate. The resulting extract solution was washed with water and brine and then the solvent was removed by evaporation. A crystallized crude product was washed with hexane to obtain 6-methyl-4-(methylthio)-2-(2,2,2-trifluoroethoxy)thieno[2,3-d]pyrimidine.
$^1$H-NMR (400MHz, $CDCl_3$) : 2.56(q, 3H, J=1.2Hz), 2.68(s, 3H), 4.86(q, 2H, J=8.4Hz), 6.89(q, 1H, J=1.2Hz).
mp: 97°C

Reference Example 15: Production of 6-methyl-4-(methylsulfonyl)-2-(2,2,2-trifluoroethoxy)thieno[2,3-d]pyrimidine

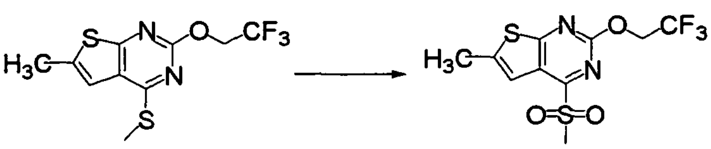

To a methylene chloride (50 mL) solution of crude 6-methyl-4-(methylthio)-2-(2,2,2-trifluoroethoxy)thieno [2,3-d]pyrimidine (3.35 g, 14.5 mmol) obtained in the above Reference Example 14 was added m-chloroperbenzoic acid (2.82 g, 16.3 mmol), followed by stirring at room temperature for 1 day. After completion of the reaction, precipitated crystals were filtrated off and the filtrate was poured into water, followed by extraction with ethyl acetate. The resulting

extract solution was washed with water and brine and then the solvent was removed by evaporation. The residue was purified on a silica gel column (Kiesel gel 60 manufactured by MERCK, 20% AcOEt-Hex) to obtain 6-methyl-4-(methylsulfonyl)-2-(2,2,2-trifluoroethoxy)thieno[2,3-d]pyrimidine (1.18 g).

$^1$H-NMR (400MHz, $CDCl_3$) : 2.65(d, 3H, J=1.2Hz), 3.39(s, 3H), 4.90(q, 2H, J=8.4Hz), 7.59(q, 1H, J=1.2Hz).

mp: 138-140°C

Tables 1 to 6 illustrate the compounds of the present invention obtainable in a similar manner to any of the above Reference Examples and Examples, but the invention is not limited thereto. In the following Tables, "Me" means methyl, "Et" ethyl, "Pr" propyl, "Bu" butyl, "Pen" pentyl, "Hex" hexyl, "Ph" phenyl, "Py" pyridyl, "Ac" acetyl, "Ts" p-toluenesulfonyl, "n-" normal, "i-" iso, "t-" tertiary, and "c-" an alicyclic hydrocarbon group. Moreover, in the $X^1$ column of Table 6, "-" means a single bond.

Formula (XXXVI)

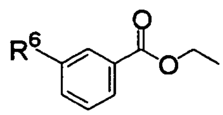

(XXXVI)

Table 1

| (3-Substituted benzoic acid ethyl esters) | | |
|---|---|---|
| No. | $R^6$ | $^1$H-NMR (CDCl3), δ (ppm) |
| 1-1 | $SCHF_2$ | 1.41(t, 3H, J=7.2Hz), 4.40(q, 2H, J=7.2Hz), 6.86(t, 1H, J=56.8Hz), 7.48(t, 1H, J=8.0Hz), 7.77(dt, 1H, J=8.0Hz, 1.6Hz), 8.10(dt, 1H, J=8.0Hz, 1.6Hz), 8.25(t, 1H, J=1.6Hz). |
| 1-2 | $OCF_3$ | 1.41(t, 3H, J=7.2Hz), 4.40(q, 2H, J=7.2Hz), 7.40(br. d, 1H, J=8.0Hz), 7.48(t, 1H, J=8.0Hz), 7.89 (m, 1H), 7.99(dt, 1H, J=8.0Hz, 1.2Hz). |

Formula (IX)

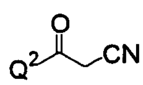

(IX)

Table 2 (Acylacetonitrile derivatives)

| No. | $Q^2$ | m.p. (°C) or $^1$H-NMR ($CDCl_3$), δ (ppm) |
|---|---|---|
| 2-1 | $2\text{-}CF_3C_6H_4$ | 71-72 |
| 2-2 | $3\text{-}CF_3C_6H_4$ | 63 |
| 2-3 | $4\text{-}CF_3C_6H_4$ | 44-45 |
| 2-4 | $3,5\text{-}(CF_3)_2C_6H_3$ | 75-77 |
| 2-5 | $3\text{-}CF_3OC_6H_4$ | 65-66 |
| 2-6 | $3\text{-}CHF_2OC_6H_4$ | 52-54 |
| 2-7 | $3\text{-}CF_3SC_6H_4$ | 77-79 |
| 2-8 | $3\text{-}CHF_2SC_6H_4$ | 62-63 |
| 2-9 | $3\text{-}CF_3\text{-}4\text{-}FC_6H_3$ | 4.13(s, 2H), 7.40(t, 1H, J=7.6Hz), 8.17(m, 1H), 8.21(d, 1H, J=7.6Hz). |
| 2-10 | $3\text{-}ClC_6H_4$ | 83-85(dec.) |
| 2-11 | $4\text{-}ClC_6H_4$ | 127-130 |
| 2-12 | $3\text{-}BrC_6H_4$ | 89-90 |
| 2-13 | $2\text{-}MeC_6H_4$ | 80-82 |
| 2-14 | $2,6\text{-}Me_2C_6H_3$ | 2.28(s, 6H), 3.76(s, 2H), 7.07(d, 2H, J=7.2Hz), 7.23(d, 1H, J=7.2Hz). |
| 2-15 | $2\text{-}MeOC_6H_4$ | 86-87 |
| 2-16 | $3\text{-}MeOC_6H_4$ | 87-88 |
| 2-17 | 3-Me-thiophen-2-yl | 112 |
| 2-18 | 2-Cl-3-py | 114-115 |
| 2-19 | 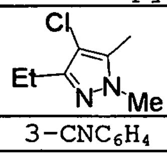 | 96 |
| 2-20 | $3\text{-}CNC_6H_4$ | 128-130 |

Formula (XI)

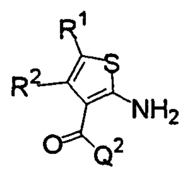 (XI)

Table 3 (2-Aminothiophene derivatives)

| No. | $R^1$ | $R^2$ | $Q^2$ | m.p. (°C) or $^1$H-NMR ($CDCl_3$), δ (ppm) |
|---|---|---|---|---|
| 3-1 | Me | H | 2-$CF_3C_6H_4$ | 136-137 |
| 3-2 | Me | H | 3-$CF_3C_6H_4$ | 158 |
| 3-3 | Et | H | 3-$CF_3C_6H_4$ | 1.21(t, 3H, J=7.6Hz), 2.60(q, 2H, J=7.6Hz), 6.42(s, 1H), 6.96(br.s, 2H), 7.50(t, 1H, J=8.0Hz), 7.73(d, 1H, J=8.0Hz), 7.83(d, 1H, J=8.0Hz), 7.92(s, 1H). |
| 3-4 | H | H | 3-$CF_3C_6H_4$ | 143-145 |
| 3-5 | Me | H | 4-$CF_3C_6H_4$ | 156 |
| 3-6 | Me | H | 3,5-$(CF_3)_2C_6H_3$ | 70 |
| 3-7 | Me | H | 3-$CF_3OC_6H_4$ | 113-114 |
| 3-8 | Me | H | 3-$CHF_2OC_6H_4$ | 2.25(d, 3H, J=1.2Hz), 6.46(d, 1H, J=1.2Hz), 6.55(t, 1H, J=73.2Hz), 6.91(br.s, 2H),7.23(d, 1H, J=8.4Hz), 7.41(s, 1H), 7.44(t, 1H, J=8.0Hz), 7.50(dt, 1H, J=8.0Hz, 1.2Hz). |
| 3-9 | Me | H | 3-$CF_3SC_6H_4$ | 73-75(dec.) |
| 3-10 | Me | H | 3-$CHF_2SC_6H_4$ | 2.25(d, 3H, J=1.2Hz), 6.45(d, 1H, J=1.2Hz), 6.87(t, 1H, J=56.8Hz), 6.93(br.s, 2H), 7.47(t, 1H, J=8.0Hz), 7.70(m, 2H), 7.87(t, 1H, J=1.6Hz). |
| 3-11 | Me | H | 3-$CF_3$-4-$FC_6H_3$ | 115-116 |
| 3-12 | Me | H | 2-$ClC_6H_4$ | 161-162 |
| 3-13 | Me | H | 3-$ClC_6H_4$ | 112 |
| 3-14 | Me | H | 3-$BrC_6H_4$ | 118 |
| 3-15 | Me | H | 2,5-$Cl_2C_6H_3$ | 193 |
| 3-16 | Me | H | 2-$MeC_6H_4$ | 149-151(dec.) |
| 3-17 | Me | H | 2,6-$Me_2C_6H_3$ | 131-132 |
| 3-18 | Me | H | 2-$MeOC_6H_4$ | 171 |
| 3-19 | Me | H | 3-$MeOC_6H_4$ | 2.24(d, 3H, J=1.2Hz), 3.85(s, 3H), 6.52(d, 1H, J=1.2Hz), 6.86(br.s, 2H), 7.0-7.2(m, 3H), 7.34(t, 1H, J=8.0Hz). |

Table 3 (Continued)

| No. | $R^1$ | $R^2$ | $Q^2$ | m.p.(°C) or $^1H$-NMR ($CDCl_3$), δ (ppm) |
|---|---|---|---|---|
| 3-20 | Me | H | t-Bu | 114 |
| 3-21 | Me | H | 3-Me-thiophen-2-yl | 116-118 |
| 3-22 | Me | H | 2-Me-3-py | 2.19(d, 3H, J=1.2Hz), 2.56(s, 3H), 6.04(d, 1H, J=1.2Hz), 7.18(dd, 1H, J=4.8Hz, 7.6Hz), 7.57(dd, 1H, J=4.8Hz, 7.6Hz), 8.56(dd, 1H, J=2.0Hz, 4.8Hz). |
| 3-23 | Me | H | 2-Cl-3-py | 120-122 |
| 3-24 | Me | H | 2-furyl | 111-112 |
| 3-25 | Me | H | 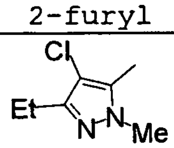 | 151-153 |
| 3-26 | Me | H | $3-CNC_6H_4$ | 2.26(d, 3H, J=1.2Hz), 6.38(d, 1H, J=1.2Hz), 7.00(br.s, 2H), 7.57(t, 1H, J=8.0Hz), 7.76(dt, 1H, J=8.0Hz, 1.2Hz), 7.78(dt, 1H, J=8.0Hz, 1.2Hz), 7.93(t, 1H, J=1.2Hz). |

Formula (XII)

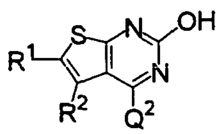 (XII)

Table 4 (2-Hydroxythieno[2,3-d]pyrimidine derivatives)

| No. | $R^1$ | $R^2$ | $Q^2$ | m.p.(°C) or $^1$H-NMR ($CDCl_3$), δ (ppm) |
|---|---|---|---|---|
| 4-1 | Me | H | OH | >300 |
| 4-2 | Me | H | 2-$CF_3C_6H_4$ | 274(dec.) |
| 4-3 | Me | H | 3-$CF_3C_6H_4$ | 251-253(dec.) |
| 4-4 | Et | H | 3-$CF_3C_6H_4$ | 1.33(t, 3H, J=7.6Hz), 2.83(dq, 2H, J=7.6Hz, 1.2Hz), 6.76(s, 1H), 7.78(t, 1H, J=8.0Hz), 7.85(d, 1H, J=8.0Hz), 8.03(s, 1H), 8.07(d, 1H, J=8.0Hz). |
| 4-5 | H | H | 3-$CF_3C_6H_4$ | 275-277(dec.) |
| 4-6 | Me | H | 4-$CF_3C_6H_4$ | 284(dec.) |
| 4-7 | Me | H | 3,5-$(CF_3)_2C_6H_3$ | 241-242 |
| 4-8 | Me | H | 3-$CF_3OC_6H_4$ | 210(dec.) |
| 4-9 | Me | H | 3-$CHF_2OC_6H_4$ | 256-258(dec.) |
| 4-10 | Me | H | 3-$CF_3SC_6H_4$ | 247(dec.) |
| 4-11 | Me | H | 3-$CHF_2SC_6H_4$ | 240-242 |
| 4-12 | Me | H | 3-$CF_3$-4-$FC_6H_3$ | 230(dec.) |
| 4-13 | Me | H | 2-$ClC_6H_4$ | 280-281(dec.) |
| 4-14 | Me | H | 3-$ClC_6H_4$ | >300 |
| 4-15 | Me | H | 3-$BrC_6H_4$ | 263-265(dec.) |
| 4-16 | Me | H | 2,5-$Cl_2C_6H_3$ | >300 |
| 4-17 | Me | H | 2-$MeC_6H_4$ | >300 |
| 4-18 | Me | H | 2-$MeOC_6H_4$ | 286(dec.) |
| 4-19 | Me | H | 3-$MeOC_6H_4$ | 264-266(dec.) |
| 4-20 | Me | H | t-Bu | 185-186(dec.) |
| 4-21 | Me | H | 3-Me-thiophen-2-yl | 251-253(dec.) |
| 4-22 | Me | H | 2-Cl-3-py | >300 |
| 4-23 | Me | H | 2-furyl | 2.52(d, 3H, J=1.2Hz), 6.72(dd, 1H, J=2.4Hz, 1.2Hz), 7.12(d, 1H, J=1.6Hz), 7.35(dd, 1H, J=2.4Hz, 1.6Hz), 7.77(m, 1H). |
| 4-24 | Me | H | 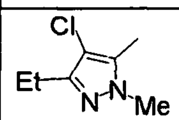 | 221-222 |

Formula (I-24)

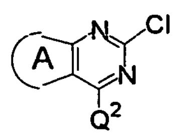 (I-24)

Table 5 (2-Chlorothienopyrimidine derivatives)

| No. | A | $R^1$ | $R^2$ | $Q^2$ | m.p. (°C) |
|---|---|---|---|---|---|
| 5-1 | A1 | Me | H | Cl | 145-146 |
| 5-2 | A1 | Me | H | SMe | 111-112 |
| 5-3 | A1 | Me | H | $2\text{-}CF_3C_6H_4$ | 82-83 |
| 5-4 | A1 | Me | H | $3\text{-}CF_3C_6H_4$ | 108 |
| 5-5 | A1 | Et | H | $3\text{-}CF_3C_6H_4$ | 63 |
| 5-6 | A1 | H | H | $3\text{-}CF_3C_6H_4$ | 126 |
| 5-7 | A1 | Me | H | $4\text{-}CF_3C_6H_4$ | 120 |
| 5-8 | A1 | Me | H | $3,5\text{-}(CF_3)_2C_6H_3$ | 135-136 |
| 5-9 | A1 | Me | H | $3\text{-}CF_3OC_6H_4$ | 97 |
| 5-10 | A1 | Me | H | $3\text{-}CHF_2OC_6H_4$ | 116 |
| 5-11 | A1 | Me | H | $3\text{-}CF_3SC_6H_4$ | 132-133 |
| 5-12 | A1 | Me | H | $3\text{-}CHF_2SC_6H_4$ | 94-95 |
| 5-13 | A1 | Me | H | $3\text{-}CF_3\text{-}4\text{-}FC_6H_3$ | 72-73 |
| 5-14 | A1 | Me | H | $2\text{-}ClC_6H_4$ | 104-105 |
| 5-15 | A1 | Me | H | $3\text{-}ClC_6H_4$ | 118-120 |
| 5-16 | A1 | Me | H | $3\text{-}BrC_6H_4$ | 126 |
| 5-17 | A1 | Me | H | $2,5\text{-}Cl_2C_6H_3$ | 143-145 |
| 5-18 | A1 | Me | H | $2\text{-}MeC_6H_4$ | 117-118 |
| 5-19 | A1 | Me | H | $2\text{-}MeOC_6H_4$ | 179-180 |
| 5-20 | A1 | Me | H | $3\text{-}MeOC_6H_4$ | 138-139 |
| 5-21 | A1 | Me | H | t-Bu | 118-119 |
| 5-22 | A1 | Me | H | 3-Me-thiophen-2-yl | 127 |
| 5-23 | A1 | Me | H | 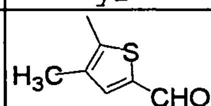 | 204-206(dec.) |
| 5-24 | A1 | Me | H | 2-Cl-3-py | >300 |
| 5-25 | A1 | Me | H | 2-furyl | 135 |
| 5-26 | A1 | Me | H | 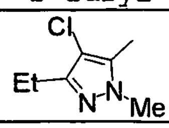 | 159-160 |

Table 5 (Continued)

| No. | A | $R^1$ | $R^2$ | $Q^2$ | m.p. (°C) |
|---|---|---|---|---|---|
| 5-27 | A1 | Me | H | $3-CNC_6H_4$ | 189-190 |
| 5-28 | A1 | Me | H | $4-MeC_6H_4$ | 119-120 |
| 5-29 | A1 | Me | H | $3-MeC_6H_4$ | 104-106 |
| 5-30 | A1 | Me | H | $2,3-Me_2C_6H_3$ | 129-131 |
| 5-31 | A1 | Me | H | $2-CF_3OC_6H_4$ | 86 |
| 5-32 | A2 | H | H | $3-CF_3C_6H_4$ | 146-148 |
| 5-33 | A2 | H | H | $3-CF_3OC_6H_4$ | 89-90 |
| 5-34 | A2 | Me | H | $3-CF_3C_6H_4$ | 174-176(dec.) |
| 5-35 | A2 | Me | H | $3-CF_3OC_6H_4$ | 107-109 |
| 5-36 | A2 | H | Me | $3-CF_3C_6H_4$ | 119-120 |
| 5-37 | A2 | H | Me | $3-CF_3OC_6H_4$ | 95-96 |

Formula (I-25)

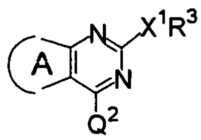

(I-25)

Table 6 (Substituted thienopyrimidine derivatives)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-1 | A1 | H | H | O | n-Pr | $3-CF_3C_6H_4$ | 106 |
| 6-2 | A1 | H | H | O | i-Pr | $3-CF_3C_6H_4$ | 86-87 |
| 6-3 | A1 | H | H | O | c-Pen | $3-CF_3C_6H_4$ | 68-70 |
| 6-4 | A1 | H | H | O | 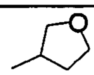 | $3-CF_3C_6H_4$ | 88-90 |
| 6-5 | A1 | H | H | O | 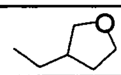 | $3-CF_3C_6H_4$ | 105 |
| 6-6 | A1 | H | H | O | Me | $3-CF_3C_6H_4$ | 145-146 |
| 6-7 | A1 | H | H | O | $CH(Me)CH_2OMe$ | $3-CF_3C_6H_4$ | 67-68 |
| 6-8 | A1 | Et | H | O | n-Pr | $3-CF_3C_6H_4$ | 1.09(t, 3H, J=7.2Hz), 1.37(t, 3H, J=7.2Hz), 1.90(sixtet, 1H, J=7.2Hz), 2.93(dq, 2H, J=1.2Hz, 7.2Hz), 4.45(t, 2H, J=7.2Hz), 7.05(t, 1H, J=1.2Hz), 7.66(t, 1H, J=7.6Hz), 7.78(d, 1H, J=7.6Hz), 8.11(d, 1H, J=7.6Hz), 8.20(s, 1H). |
| 6-9 | A1 | Me | H | - | H | $3-CF_3C_6H_4$ | 70-72 |
| 6-10 | A1 | Me | H | - | CN | $3-CF_3C_6H_4$ | 121-122 |
| 6-11 | A1 | Me | H | - | n-Bu | $3-CF_3OC_6H_4$ | 0.98(t, 3H, J=7.2Hz), 1.47(sextet, 2H, J=7.2Hz), 1.91(m, 2H), 2.63(d, 3H,J=1.2Hz), 3.09(t, 2H,J=7.2Hz), 7.12(d, 1H,J=1.2Hz), 7.37(dt, 1H, J=8.0Hz, 1.2Hz), 7.58(t, 1H, J=8.0Hz), 7.78(s, 1H), 7.86(dt, 1H, J=8.0Hz, 1.2Hz). |
| 6-12 | A1 | Me | H | - | $CH_2Cl$ | $3-CF_3C_6H_4$ | 2.67(d, 1H, J=2.0Hz), 4.90(s, 2H), 7.18(d, 1H, J=1.2Hz), 7.64(d, 1H, J=8.0Hz), 7.80(t, 1H, J=8.0Hz), 8.13(d, 1H, J=8.0Hz), 8.20(s, 1H). |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-13 | A1 | Me | H | - | $CH_2OMe$ | $3-CF_3C_6H_4$ | 2.67(d, 1H, J=2.0Hz), 3.61(s, 3H), 4.86(s, 2H), 7.15(d, 1H, J=1.2Hz), 7.68(d, 1H, J=8.0Hz), 7.79(t, 1H, J=8.0Hz), 8.11(d, 1H, J=8.0Hz), 8.19(s, 1H). |
| 6-14 | A1 | Me | H | - | $CH_2OEt$ | $3-CF_3C_6H_4$ | 1.35(t, 3H, J=7.2Hz), 2.65(d, 3H, J=1.2Hz), 3.78(q, 2H, J=7.2Hz), 4.80(s, 2H), 7.14(d, 1H, J=1.2Hz), 7.68(d, 1H, J=8.0Hz), 7.78(t, 1H, J=8.0Hz), 8.11(d, 1H, J=8.0Hz), 8.19(s, 1H). |
| 6-15 | A1 | Me | H | - | $CH_2O$-n-Pr | $3-CF_3C_6H_4$ | 0.98(t, 3H, J=7.6Hz), 1.75(sextet, 2H, J=7.6Hz), 2.65(d, 3H, J=1.2Hz), 3.66(t, 2H, J=7.6Hz), 4.89(s, 2H), 7.15(d, 1H, J=1.2Hz), 7.68(d, 1H, J=8.0Hz), 7.78(t, 1H, J=8.0Hz), 8.11(d, 1H, J=8.0Hz), 8.20(s, 1H). |
| 6-16 | A1 | Me | H | - | $CH_2O$-i-Pr | $3-CF_3C_6H_4$ | 1.31(d, 6H, J=6.0Hz), 2.65(d, 3H, J=1.2Hz), 3.91(7-plet, 1H, J=6.0Hz), 4.89(s, 2H), 7.14(d, 1H, J=1.2Hz), 7.68(d, 1H, J=8.0Hz), 7.78(t, 1H, J=8.0Hz), 8.11(d, 1H, J=8.0Hz), 8.20(s, 1H). |
| 6-17 | A1 | Me | H | - | $CH_2OCH_2CF_3$ | $3-CF_3C_6H_4$ | 40-41 |
| 6-18 | A1 | Me | H | - | $4-CF_3C_6H_4$ | $3-CF_3C_6H_4$ | 134-136 |
| 6-19 | A1 | Me | H | O | $3-CF_3C_6H_4$ | $3-ClC_6H_4$ | 101-102 |
| 6-20 | A1 | Me | H | O | $3-CF_3C_6H_4$ | $4-CF_3C_6H_4$ | 89-91 |
| 6-21 | A1 | Me | H | O | $3-CF_3C_6H_4$ | t-Bu | 1.45(s, 9H), 2.58(d, 3H, J=1.2Hz), 7.19(d, 1H, J=1.2Hz), 7.4-7.6(m, 4H). |
| 6-22 | A1 | Me | H | O | $3-CF_3OC_6H_4$ | $4-CF_3C_6H_4$ | 67-68 |
| 6-23 | A1 | Me | H | O | Me | $3-CF_3C_6H_4$ | 77 |
| 6-24 | A1 | Me | H | O | Et | $3-CF_3C_6H_4$ | 98 |
| 6-25 | A1 | Me | H | O | n-Pr | $3-CF_3C_6H_4$ | 78 |
| 6-26 | A1 | Me | H | O | i-Pr | $3-CF_3C_6H_4$ | 118-119 |
| 6-27 | A1 | Me | H | O | t-Bu | $3-CF_3C_6H_4$ | 132-133 |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-28 | A1 | Me | H | O | Et | 3-$CF_3$-4-$FC_6H_3$ | 1.49(t, 3H, J=7.2Hz), 2.59(d, 3H, J=1.2Hz), 4.54(q, 2H, J=7.2Hz), 7.02(d, 1H, J=1.2Hz), 7.36(t, 1H, J=9.6Hz), 8.14(m, 1H), 8.21(dd, 1H, J=1.6Hz, 6.8Hz). |
| 6-29 | A1 | Me | H | O | n-Pr | 3-$CF_3$-4-$FC_6H_3$ | 1.08(t, 3H, J=7.6Hz), 1.90(sixtet, 2H, J=7.6Hz), 2.59(d, 3H, J=1.2Hz), 4.44(t, 2H, J=7.6Hz), 7.01(d, 1H, J=1.2Hz), 7.36(t, 1H, J=9.6Hz), 8.13(m, 1H), 8.20(d, 1H, J=6.4Hz). |
| 6-30 | A1 | Me | H | O | i-Pr | 3-$CF_3$-4-$FC_6H_3$ | 125-126 |
| 6-31 | A1 | Me | H | O | n-Pr | 3,5-$(CF_3)_2C_6H_3$ | 146-147 |
| 6-32 | A1 | Me | H | O | n-Pr | 3-$CF_3$-4-n-$PrOC_6H_3$ | 1.07(t, 3H, J=7.6Hz), 1.09(t, 3H, J=7.6Hz), 1.90(sixtet, 2H, J=7.6Hz), 2.58(d, 3H, J=1.2Hz), 4.11(t, 2H, J=7.2Hz), 4.43(t, 2H, J=7.2Hz), 7.06(d, 1H, J=1.2Hz), 7.11(t, 1H, J=8.8Hz), 8.10(dd, 1H, J=2.0Hz, 8.8Hz), 8.18(d, 1H, J=2.0Hz). |
| 6-33 | A1 | Me | H | O | Me | 3-$CF_3OC_6H_4$ | 65-66 |
| 6-34 | A1 | Me | H | O | Et | 3-$CF_3OC_6H_4$ | 72-74 |
| 6-35 | A1 | Me | H | O | n-Pr | 3-$CF_3OC_6H_4$ | 58-60 |
| 6-36 | A1 | Me | H | O | i-Pr | 3-$CF_3OC_6H_4$ | 93 |
| 6-37 | A1 | Me | H | O | n-Bu | 3-$CF_3OC_6H_4$ | 0.99(t, 3H, J=7.6Hz), 1.54(m, 2H), 1.86(m, 2H), 2.58(d, 3H, J=1.2Hz), 4.48(t, 2H, J=7.6H), 7.06(q, 1H, J=1.2Hz), 7.37(m, 1H), 7.56(t, 1H, J=8.0Hz), 7.79(s, 1H), 7.87(dt, 1H, J=8.0Hz, 1.2Hz). |
| 6-38 | A1 | Me | H | O | Et | 3-$CF_3SC_6H_4$ | 98-100 |
| 6-39 | A1 | Me | H | O | n-Pr | 3-$CF_3SC_6H_4$ | 77 |
| 6-40 | A1 | Me | H | O | n-Pr | 3-$CHF_2OC_6H_4$ | 60-63 |
| 6-41 | A1 | Me | H | O | i-Pr | 3-$CHF_2OC_6H_4$ | 56 |

Table 6 (Continued)

| No. | A | $R^1$ | $R^2$ | $X^1$ | $R^3$ | $Q^2$ | m.p. (°C) or $^1H$-NMR ($CDCl_3$), δ (ppm) |
|---|---|---|---|---|---|---|---|
| 6-42 | A1 | Me | H | O | n-Pr | 3-$CHF_2SC_6H_4$ | 1.08(t, 3H, J=7.2Hz), 1.90(quant, 2H, J=7.2Hz), 2.57(d, 1H, J=1.2Hz),4.44(t, 2H, J=7.2Hz), 6.90(t, 1H, J=56.4Hz),7.07(d, 1H, J=1.2Hz), 7.56(d, 1H, J=8.0Hz), 7.73(dt, 1H, J=8.0Hz, 1.6Hz), 7.99(dt, 1H, J=8.0Hz, 1.6Hz), 8.15(t, 1H, J=1.6Hz). |
| 6-43 | A1 | Me | H | O | Et | 2-$CF_3C_6H_4$ | 83-85 |
| 6-44 | A1 | Me | H | O | Et | 4-$CF_3C_6H_4$ | 139 |
| 6-45 | A1 | Me | H | O | Et | 2-$MeOC_6H_4$ | 87-88 |
| 6-46 | A1 | Me | H | O | Et | 2-$MeC_6H_4$ | 1.46(t, 3H, J=7.2Hz), 2.30(s, 3H), 2.50(d, 3H, J=1.2Hz), 4.50(q, 2H, J=7.2Hz), 6.64(d, 1H, J=1.2Hz), 7.4(m, 4H). |
| 6-47 | A1 | Me | H | O | $C_5H_{11}$ | 2-$MeC_6H_4$ | 0.92(t, 3H, J=7.2Hz), 1.3-1.5(m, 4H), 1.85(m, 2H),2.30(s, 3H),2.50(d, 3H, J=1.2Hz), 4.43(t, 2H, J=7.2Hz), 6.63(d, 1H, J=1.2Hz), 7.3-7.4(m, 4H). |
| 6-48 | A1 | Me | H | O | c-Hex | 2-$MeC_6H_4$ | 125-126 |
| 6-49 | A1 | Me | H | O | CH(Me)$CO_2$Et | 2-$MeC_6H_4$ | 1.19(t,3H,J=7.2Hz), 1.68(d, 3H, J=7.2Hz), 2.28(s, 3H), 2.50(d, 3H, J=1.2Hz), 4.15(m, 2H), 5.36(q, 2H, J=7.2Hz), 6.66(d, 1H, J=1.2Hz), 7.3(m, 4H). |
| 6-50 | A1 | Me | H | O | Et | 3-Me-thiophen-2-yl | 54 |
| 6-51 | A1 | Me | H | O | Et | t-Bu | 74-76 |
| 6-52 | A1 | Me | H | O | n-Pr | 3-$ClC_6H_4$ | 103 |
| 6-53 | A1 | Me | H | O | $CH_2C(Me)_3$ | 3-$ClC_6H_4$ | 106-107 |
| 6-54 | A1 | Me | H | O | c-Pen | 3-$ClC_6H_4$ | 123 |
| 6-55 | A1 | Me | H | O | n-Pr | 2,5-$Cl_2C_6H_3$ | 101-103 |
| 6-56 | A1 | Me | H | O | n-Pr | 2-$ClC_6H_4$ | 61-63 |
| 6-57 | A1 | Me | H | O | $CH_2C(Me)_3$ | 2-$ClC_6H_4$ | 167-168 |
| 6-58 | A1 | Me | H | O | c-Pen | 2-$ClC_6H_4$ | 1.6(m, 2H), 1.8-2.0(m, 6H), 2.51(d, 3H, J=1.2Hz), 5.53(7-plet, 1H, J=3.2Hz), 6.63(d, 1H, J=1.2Hz), 7.3-7.6(m, 4H). |
| 6-59 | A1 | Me | H | O | n-Pr | 3-$BrC_6H_4$ | 98 |
| 6-60 | A1 | Me | H | O | $CH_2C(Me)_3$ | 3-$BrC_6H_4$ | 86-88 |
| 6-61 | A1 | Me | H | O | c-Pen | 3-$BrC_6H_4$ | 110-111 |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-62 | A1 | Me | H | O | n-Pr | 3-$MeOC_6H_4$ | 1.08(t, 3H, J=7.6Hz), 1.89(sixtet, 2H, J=7.6Hz), 2.56(d, 3H, J=1.2Hz), 3.89(s, 3H), 4,44(t, 2H, J=7.6Hz), 7.05(m, 1H), 7.10(q, 1H, J=1.2Hz), 7.4-7.5(m, 3H). |
| 6-63 | A1 | Me | H | O | $CH_2C(Me)_3$ | 3-$MeOC_6H_4$ | 1.10(s, 9H), 2.55(d, 3H, J=1.2Hz), 3.90(s, 3H), 4.17(s, 2H), 7.05(m, 1H), 7.08(q, 1H, J=1.2Hz), 7.4-7.5(m, 3H). |
| 6-64 | A1 | Me | H | O | c-Pen | 3-$MeOC_6H_4$ | 1.6-2.1(m, 8H), 2.55(d, 3H, J=1.2Hz), 3.89(s, 3H), 5.56(7-plet, 1H, J=2.8Hz), 7.05(m, 1H,), 7.10(d, 1H, J=1.2Hz), 7.4-7.5(m, 3H). |
| 6-65 | A1 | Me | H | O | n-Pr | 2-furyl | 84 |
| 6-66 | A1 | Me | H | O | $CH(Me)CO_2Et$ | 2-furyl | 85 |
| 6-67 | A1 | Me | H | O | n-Pr | 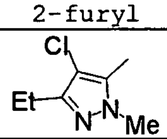 | 90-91 |
| 6-68 | A1 | Me | H | O | n-Pr | 2-Cl-3-py | 126 |
| 6-69 | A1 | Me | H | O | $CH_2C(Me)_3$ | 3-$CF_3C_6H_4$ | 102-104 |
| 6-70 | A1 | Me | H | O | $CH(Me)C_3H_7$ | 3-$CF_3C_6H_4$ | 83-84 |
| 6-71 | A1 | Me | H | O | $CH_2CH(Me)C_2H_5$ | 3-$CF_3C_6H_4$ | 63-65 |
| 6-72 | A1 | Me | H | O | $CH(Me)CH(Me)_2$ | 3-$CF_3C_6H_4$ | 90-91 |
| 6-73 | A1 | Me | H | O | $CH(Me)C(Me)_3$ | 3-$CF_3C_6H_4$ | 115-117 |
| 6-74 | A1 | Me | H | O | $(CH_2)_5Me$ | 3-$CF_3C_6H_4$ | 0.90(t, 3H, J=7.2Hz), 1.35(m, 4H), 1.5(m, 2H), 1.87(fiftet, 2H, J=7.2Hz), 2.58(d, 3H, J=1.2Hz),4.47(t, 2H, J=7.2Hz), 7.04(d, 1H, J=1.2Hz),7.66(t, 1H, J=8.0Hz), 7.77 (d, 1H, J=8.0Hz), 8.10(d, 1H, J=8.0Hz), 8.19(s, 1H). |
| 6-75 | A1 | Me | H | O | $CH_2CH(Et)_2$ | 3-$CF_3C_6H_4$ | 58-59 |
| 6-76 | A1 | Me | H | O | $CH_2CH(Me)C_3H_7$ | 3-$CF_3C_6H_4$ | 0.93(t,3H,J=7.2Hz),1.07(d,3H, J=6.8Hz), 1.2-1.5(m, 4H), 2.05(m, 1H), 2.58(d, 3H, J=1.2Hz), 4.25(dd, 1H, J=5.6Hz, 10Hz), 4.36(dd, 1H, J=5.6Hz, 10Hz), 7.03(d, 1H, J=1.2Hz),7.66(t, 1H, J=8.0Hz), 7.77(d,1 H, J=8.0Hz), 8.10(d, 1H, J=8.0Hz), 8.18(s, 1H). |

Table 6 (Continued)

| No. | A | $R^1$ | $R^2$ | $X^1$ | $R^3$ | $Q^2$ | m.p. (°C) or $^1H$-NMR ($CDCl_3$), δ (ppm) |
|---|---|---|---|---|---|---|---|
| 6-77 | A1 | Me | H | O | CH(Me)$CH_2$ CH$(Me)_2$ | 3-$CF_3C_6H_4$ | 111-112 |
| 6-78 | A1 | Me | H | O | C$(Et)_2$Me | 3-$CF_3C_6H_4$ | 0.94(t, 3H, J=7.6Hz), 1.60(s, 3H), 2.0(m,2H),2.2(m,2H),2.57(d,3H,J=1.2Hz), 7.02(d,1H,J=1.2Hz),7.66 (t,1H, J=8.0Hz), 7.76(d,1H,J=8.0Hz),8.08 (d,1H, J=8.0Hz), 8.16(s, 1H). |
| 6-79 | A1 | Me | H | O | $CH_2C(NH_2)$ (Me)$CH_2OH$ | 3-$CF_3C_6H_4$ | 116-117 |
| 6-80 | A1 | Me | H | O | cyclo-Pen | 3-$CF_3C_6H_4$ | 130-131 |
| 6-81 | A1 | Me | H | O | $CH_2$-c-Hex | 3-$CF_3C_6H_4$ | 102-105 |
| 6-82 | A1 | Me | H | O | 4-t-Bu-c-Hex | 3-$CF_3C_6H_4$ | 0.89(s, 9H), 1.0-1.3(m, 4H), 1.5(m, 1H), 1.9(m,2H),2.3(m,2H),2.58(d,3H,J=1.2Hz), 5.0(m, 1H), 7.03(d, 1H, J=1.2Hz), 7.66(t,1H,J=8.0Hz),7.77 (d,1H, J=8.0Hz), 8.10(d, 1H, J=8.0Hz), 8.18(s, 1H). |
| 6-83 | A1 | Me | H | O | c-Pen | 3-$CF_3$-4-$FC_6H_3$ | 1.6-2.1(m, 8H), 2.58(d, 3H, J=1.2Hz), 5.55(7-plet,1H,J=2.8Hz), 7.00(d,1H,J=1.2Hz),7.36 (t,1H, J=9.2Hz), 8.12(m,1H),8.19(dd,1H,J=6.8Hz, 2.0Hz). |
| 6-84 | A1 | Me | H | O | $CH_2C(Me)_3$ | 3-$CF_3OC_6H_4$ | 76 |
| 6-85 | A1 | Me | H | O | c-Pen | 3-$CF_3OC_6H_4$ | 1.8-2.1(m, 8H), 2.57(d, 3H, J=1.2Hz), 5.55(7-plet,1H,J=2.8Hz), 7.05(d,1H,J=1.2Hz),7.36 (t1H, J=8.0Hz), 7.55(t, 1H, J=8.0Hz), 7.79(br.s, 1H), 7.86(d, 1H, J=8.0Hz). |
| 6-86 | A1 | Me | H | O | c-Pen | 3-$CF_3SC_6H_4$ | 85 |
| 6-87 | A1 | Me | H | O | propargyl | 3-$CF_3C_6H_4$ | 91-93 |
| 6-88 | A1 | Me | H | O | allyl | 3-$CF_3C_6H_4$ | 58-60 |
| 6-89 | A1 | Me | H | O | methallyl | 3-$CF_3C_6H_4$ | 62-63 |

Table 6 (Continued)

| No. | A | $R^1$ | $R^2$ | $X^1$ | $R^3$ | $Q^2$ | m.p. (°C) or $^1$H-NMR ($CDCl_3$), δ(ppm) |
|---|---|---|---|---|---|---|---|
| 6-90 | A1 | Me | H | O | 1-butyn-3-yl | 3-$CF_3C_6H_4$ | 1.74(d, 3H, J=6.8Hz), 2.44(d, 1H, J=2.4Hz), 2.60(d, 1H, J=1.2Hz), 5.85(dq, 1H, J=2.4Hz, 6.8Hz), 7.08(d, 1H, J=1.2Hz),7.67(t, 1H, J=8.0Hz), 7.78(d, 1H, J=8.0Hz),8.14(d, 1H, J=8.0Hz), 8.23(s, 1H). |
| 6-91 | A1 | Me | H | O | trans-crotyl | 3-$CF_3C_6H_4$ | 77-78 |
| 6-92 | A1 | Me | H | O | 3-buten-1-yl | 3-$CF_3C_6H_4$ | 2.58(d, 1H, J=1.2Hz), 2.64(qd, 2H, J=6.8Hz, 1.6Hz), 4.50(t, 2H, J=6.8Hz), 5.11(dd, 1H, J=1.6Hz, 10.4Hz), 5.20(dd, 1H, J=1.6Hz, 17.2Hz), 5.96(dd, 1H, J=10.4Hz, 17.2Hz), 7.05(d, 1H,J=1.2Hz), 7.66(t, 1H, J=8.0Hz), 7.77(d, 1H, J=8.0Hz),8.11(d, 1H, J=8.0Hz), 8.19(s, 1H). |
| 6-93 | A1 | Me | H | O | 2-butyn-1-yl | 3-$CF_3C_6H_4$ | 96 |
| 6-94 | A1 | Me | H | O | 1-butyn-3-yl | 3-$CF_3OC_6H_4$ | 1.74(d, 3H, J=6.8Hz),2.43(d, 1H, J=2.0Hz), 2.59(d, 1H, J=1.2Hz), 5.84(dq, 1H, J=2.0Hz, 6.8Hz), 7.09(d, 1H, J=1.2Hz),7.37(d, 1H, J=8.0Hz), 7.57(t, 1H, J=8.0Hz), 7.84(s, 1H), 7.90(dt, 1H, J=8.0Hz, 1.2Hz). |
| 6-95 | A1 | Me | H | O | 1-butyn-3-yl | 3-$CF_3SC_6H_4$ | 98-99 |
| 6-96 | A1 | Me | H | O | $C_2H_4F$ | 3-$CF_3C_6H_4$ | 66-67 |
| 6-97 | A1 | Me | H | O | $CH_2CF_3$ | 3-$CF_3C_6H_4$ | 100-101 |
| 6-98 | A1 | Me | H | O | $CH_2CCl_3$ | 3-$CF_3C_6H_4$ | 120 |
| 6-99 | A1 | Me | H | O | $CH_2CHF_2$ | 3-$CF_3C_6H_4$ | 98-99 |
| 6-100 | A1 | Me | H | O | $CH(Me)CF_3$ | 3-$CF_3C_6H_4$ | 95-97 |
| 6-101 | A1 | Me | H | O | $CH_2CF_2CF_3$ | 3-$CF_3C_6H_4$ | 43-44 |
| 6-102 | A1 | Me | H | O | $CH(CF_3)_2$ | 3-$CF_3C_6H_4$ | 2.64(d, 3H, J=1.2Hz), 6.52(fiftet, 1H, J=6.0Hz), 7.13(d, 1H, J=1.2Hz), 7.71(t, 1H, J=8.0Hz), 7.83(d, 1H, J=8.0Hz), 8.11(d, 1H, J=8.0Hz), 8.17(s, 1H). |
| 6-103 | A1 | Me | H | O | $CH_2CF_2CHF_2$ | 3-$CF_3C_6H_4$ | 100-101 |
| 6-104 | A1 | Me | H | O | $C_3H_6Br$ | 3-$CF_3C_6H_4$ | 128-129 |
| 6-105 | A1 | Me | H | O | $C_3H_6Cl$ | 3-$CF_3C_6H_4$ | 2.68(d, 3H, J=1.2Hz), 3.62(s, 3H), 7.21(d, 2H, J=1.2Hz),7.72(t, 1H, J=8.0Hz), 7.83(d, 1H, J=8.0Hz),8.14(d, 1H, J=8.0Hz), 8.19(s, 1H). |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-106 | A1 | Me | H | O | $CH(Me)CF_2CF_3$ | $3-CF_3C_6H_4$ | 86-88 |
| 6-107 | A1 | Me | H | O | $C_2H_4F$ | $3-CF_3OC_6H_4$ | 62-64 |
| 6-108 | A1 | Me | H | O | $CH_2CF_3$ | $3-CF_3OC_6H_4$ | 60-62 |
| 6-109 | A1 | Me | H | O | $CH_2CHF_2$ | $3-CF_3OC_6H_4$ | 82-83 |
| 6-110 | A1 | Me | H | O | $CH(Me)CF_3$ | $3-CF_3OC_6H_4$ | 70-72 |
| 6-111 | A1 | Me | H | O | $CH_2CF_2CF_3$ | $3-CF_3OC_6H_4$ | 2.61(d, 3H, J=1.2Hz), 5.01(tt, 1H, J=1.2Hz, 12.8Hz), 7.11(d, 1H, J=1.2Hz), 7.40(dt, 1H, J=1.2Hz, 8.0Hz), 7.59(t, 1H, J=8.0Hz), 7.78(s, 1H), 7.86(dt, 1H, J=1.6Hz, 8.0Hz). |
| 6-112 | A1 | Me | H | O | $CH_2CF_2CHF_2$ | $3-CF_3OC_6H_4$ | 104-106 |
| 6-113 | A1 | Me | H | O | $C_2H_4CF_3$ | $3-CF_3OC_6H_4$ | 2.60(d, 3H, J=1.2Hz), 2.74(m, 2H), 4.72(t, 2H,J=6.8Hz),7.09(d, 1H, J=1.2Hz), 7.38(dt, 1H, J=1.2Hz, 8.0Hz), 7.58(t, 1H, J=8.0Hz), 7.78(s, 1H), 7.86(dt, 1H, J=1.2Hz, 8.0Hz). |
| 6-114 | A1 | Me | H | O | $CH(Me)CF_2CF_3$ | $3-CF_3OC_6H_4$ | 1.62(d, 3H, J=6.8Hz), 2.60(d, 3H, J=1.2Hz), 6.00(df, 1H, J=8.0Hz, 15.6Hz), 7.10(d, 1H, J=1.2Hz), 7.40(td, 1H, J=8.0Hz, 1.6Hz), 7.58(t, 1H, J=8.0Hz), 7.77(s, 1H),7.86(dt, 1H, J=8.0Hz, 1.6Hz). |
| 6-115 | A1 | Me | H | O | $CH_2CF_3$ | $3-CF_3SC_6H_4$ | 73-75 |
| 6-116 | A1 | Me | H | O | $CH_2CHF_2$ | $3-CF_3SC_6H_4$ | 64-65 |
| 6-117 | A1 | Me | H | O | $CH(Me)CF_3$ | $3-CF_3SC_6H_4$ | 73-74 |
| 6-118 | A1 | Me | H | O | $CH_2CF_2CF_3$ | $3-CF_3SC_6H_4$ | 76-78 |
| 6-119 | A1 | Me | H | O | $CH_2CF_2CHF_2$ | $3-CF_3SC_6H_4$ | 106-107 |
| 6-120 | A1 | Me | H | O | $CH(Me)CF_2CF_3$ | $3-CF_3SC_6H_4$ | 1.62(d, 3H, J=6.4Hz), 2.60(d, 3H, J=1.2Hz), 6.00(df, 1H, J=7.2Hz, 15.6Hz), 7.09(d, 1H, J=1.2Hz),7.62(t, 1H, J=8.0Hz), 7.82(d, 1H, J=8.0Hz), 8.04(dt,1H, J=8.0Hz, 1.2Hz), 8.19(s, 1H). |
| 6-121 | A1 | Me | H | O | $CH_2CF_3$ | $3-CHF_2OC_6H_4$ | 40-42 |
| 6-122 | A1 | Me | H | O | $CH_2CHF_2$ | $3-CHF_2OC_6H_4$ | 60-61 |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-123 | A1 | Me | H | O | $CH(Me)CF_3$ | 3-$CHF_2OC_6H_4$ | 1.60(d, 3H, J=6.0Hz),2.59(d, 3H, J=1.2Hz), 5.87(quant, 1H, J=6.4Hz), 6.61(t, 1H, J=73.6Hz), 7.11(d, 1H, J=1.2Hz), 7.30(dd, 1H, J=8.0Hz, 2.0Hz), 7.54(t, 1H, J=8.0Hz), 7.69(t, 1H, J=2.0Hz), 7.77(dt, 1H, J=8.0Hz, 1.2Hz). |
| 6-124 | A1 | Me | H | O | $CH_2CF_2CF_3$ | 3-$CHF_2OC_6H_4$ | 2.60(d, 3H, J=1.2Hz), 5.00(dt, 1H, J=1.2Hz, 12.8Hz), 6.61(t, 1H,J=73.2Hz),7.12(d, 1H, J=1.2Hz), 7.31(dd, 1H, J=8.0Hz, 2.4Hz), 7.55(t, 1H, J=8.0Hz), 7.69(s, 1H), 7.77(dt, 1H, J=8.0Hz, 1.2Hz). |
| 6-125 | A1 | Me | H | O | $CH_2CF_2CHF_2$ | 3-$CHF_2OC_6H_4$ | 91-92 |
| 6-126 | A1 | Me | H | O | $C_2H_4CF_3$ | 3-$CHF_2OC_6H_4$ | 2.59(d, 3H, J=1.2Hz), 2.73(m, 2H), 4.72(t, 2H,J=6.8Hz), 6.60(t, 1H, J=73.2Hz), 7.10(d, 1H, J=1.2Hz), 7.29(dd, 1H, J=8.0Hz, 2.0Hz), 7.54(t, 1H, J=8.0Hz), 7.69(s, 1H), 7.77(dt, 1H, J=8.0Hz, 1.2Hz). |
| 6-127 | A1 | Me | H | O | $CH_2CF_3$ | 3-$CHF_2SC_6H_4$ | 2.67(d, 3H, J=1.2Hz), 6.27(dd, 1H, J=0.8Hz, 10HZ), 6.64(dd, 1H, J=0.8Hz, 16.8HZ), 7.20(dd, 1H, J=10Hz, 16.8HZ), 7.21(d, 1H, J=1.2Hz), 7.71(d, 1H, J=8.0Hz), 7.83(d, 1H, J=8.0Hz), 8.14(d, 1H, J=8.0Hz), 8.19(s, 1H). |
| 6-128 | A1 | Me | H | O | $CH_2CF_3$ | $OCH_2CF_3$ | 101 |
| 6-129 | A1 | Me | H | O | $CH_2CF_3$ | 3-$CF_3C_6H_4O$ | 124-125 |
| 6-130 | A1 | Me | H | - | $C_2H_4CF_3$ | 3-$CF_3C_6H_4O$ | 85 |
| 6-131 | A1 | Me | H | - | CH(=CHOH) $CH_2CF_3$ | 4-$CF_3C_6H_4$ | 147-148 |
| 6-132 | A1 | Me | H | O | $CH_2CF_3$ | 3-$CF_3OC_6H_4O$ | 89 |
| 6-133 | A1 | Me | H | - | $C_2H_4CF_3$ | 3-$CF_3OC_6H_4O$ | 2.5-2.6(m, 2H), 2.64(d, 3H, J=1.2Hz), 3.10(m, 2H), 7.10(q, 1H, J=1.2Hz), 7.16(m, 3H), 7.46(m, 1H). |
| 6-134 | A1 | Me | H | O | $CH_2CF_3$ | SMe | 97 |
| 6-135 | A1 | Me | H | - | $C_2H_4CF_3$ | SMe | 81-83 |
| 6-136 | A1 | Me | H | O | $CH_2CF_3$ | SOMe | 121-122 |

Table 6 (Continued)

| No. | A | $R^1$ | $R^2$ | $X^1$ | $R^3$ | $Q^2$ | m.p. (°C) $^1$H-NMR ($CDCl_3$), δ(ppm) |
|---|---|---|---|---|---|---|---|
| 6-137 | A1 | Me | H | O | $CH_2CF_3$ | $SO_2Me$ | 138-140 |
| 6-138 | A1 | Me | H | - | $C_2H_4CF_3$ | $SO_2Me$ | 60-61 |
| 6-139 | A1 | Me | H | - | $C_2H_4CF_3$ | OH | 267-269(dec.) |
| 6-140 | A1 | Me | H | S | Me | SMe | 89-90 |
| 6-141 | A1 | Me | H | $SO_2$ | Me | $SO_2Me$ | 199 |
| 6-142 | A1 | Me | H | O | $CH(Me)CH_2OMe$ | 3-$CF_3C_6H_4$ | 63-65 |
| 6-143 | A1 | Me | H | O | $C_2H_4SC_2H_5$ | 3-$CF_3C_6H_4$ | 2.37(s, 6H), 2.58(d, 3H, J=1.2Hz), 2.82(t, 2H, J=6.0Hz), 4.60(t, 2H, J=6.0Hz), 7.05(q, 1H, J=1.2Hz), 7.66(t, 1H, J=8.0Hz), 7.77(d, 1H, J=8.0Hz),8.11(d, 1H, J=8.0Hz), 8.20(s, 1H) |
| 6-144 | A1 | Me | H | O | $C_2H_4NMe_2$ | 3-$CF_3C_6H_4$ | 62-63 |
| 6-145 | A1 | Me | H | O | $C_2H_4SO_2Me$ | 3-$CF_3C_6H_4$ | 231(dec.) |
| 6-146 | A1 | Me | H | O | Ph | 3-$CF_3C_6H_4$ | 101-102 |
| 6-147 | A1 | Me | H | O | Benzyl | 3-$CF_3C_6H_4$ | 2.59(d, 3H, J=1.2Hz), 5.56(s, 2H), 7.05(d, 1H, J=1.2Hz), 7.3(m, 3H), 7.54(m, 2H), 7.66(t, 1H, J=8.0Hz),7.77(d, 1H, J=8.0Hz),8.09(d, 1H, J=8.0Hz), 8.17(s, 1H). |
| 6-148 | A1 | Me | H | O | $SO_2Me$ | 3-$CF_3C_6H_4$ | 109-110 |
| 6-149 | A1 | Me | H | O | $SO_2CH_2Cl$ | 3-$CF_3C_6H_4$ | 95-97 |
| 6-150 | A1 | Me | H | O | $SO_2CH=CH_2$ | 3-$CF_3C_6H_4$ | 2.67(d, 3H, J=1.2Hz), 6.27(dd, 1H, J=0.8Hz, 10HZ), 6.64(dd, 1H, J=0.8Hz, 16.8HZ), 7.20(dd, 1H, J=10Hz, 16.8HZ), 7.21(d, 1H, J=1.2Hz),7.71(d, 1H, J=8.0Hz), 7.83(d, 1H, J=8.0Hz), 8.14(d, 1H, J=8.0Hz), 8.19(s, 1H). |
| 6-151 | A1 | Me | H | O | $SO_2Et$ | 3-$CF_3C_6H_4$ | 1.64(t, 3H, J=7.2Hz), 2.67(d, 3H, J=1.2Hz), 3.78(q, 2H, J=7.2Hz),7.20(d, 1H, J=1.2Hz), 7.71(d, 1H, J=8.0Hz),7.82(d, 1H, J=8.0Hz), 8.14(d, 1H, J=8.0Hz), 8.20(s, 1H). |
| 6-152 | A1 | Me | H | O | $SO_2$n-Pr | 3-$CF_3C_6H_4$ | 90-92 |
| 6-153 | A1 | Me | H | O | $SO_2$i-Pr | 3-$CF_3C_6H_4$ | 1.64(d, 6H, J=6.8Hz), 2.67(d, 3H, J=1.2Hz), 4.20(m, 1H), 7.21(d, 1H, J=1.2Hz), 7.71(t, 1H,J=8.0Hz),7.82(d, 1H,J=8.0Hz), 8.14(d, 1H, J=8.0Hz), 8.20(s, 1H). |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-154 | A1 | Me | H | O | $SO_2$n-Bu | $3-CF_3C_6H_4$ | 93-94 |
| 6-155 | A1 | Me | H | O | $SO_2CF_3$ | $3-CF_3C_6H_4$ | 64-66 |
| 6-156 | A1 | Me | H | O | $SO_2CH_2CF_3$ | $3-CF_3C_6H_4$ | 106-108 |
| 6-157 | A1 | Me | H | O | $SO_2(CH_2)_3Cl$ | $3-CF_3C_6H_4$ | 2.58(m, 2H), 2.68(d, 3H, J=1.2Hz), 3.78(t, 2H, J=6.4Hz), 3.99(t, 2H, 7.6Hz), 7.22(d, 1H,J=1.2Hz), 7.71(d, 1H, J=8.0Hz), 7.84(d, 1H,J=8.0Hz),8.15(d,1H, J=8.0Hz), 8.20(s, 1H). |
| 6-158 | A1 | Me | H | O | $SO_2C_6H_{13}$ | $3-CF_3C_6H_4$ | 0.89(t, 3H,J=7.2Hz), 1.34(m, 4H), 1.54(m, 2H), 2.11(m, 2H), 2.67(d, 3H, J=1.2Hz), 3.76(m, 2H), 7.21(d, 1H, J=1.2Hz),7.71(d, 1H, J=8.0Hz), 7.83(d, 1H, J=8.0Hz), 8.14(d, 1H, J=8.0Hz), 8.20(s, 1H). |
| 6-159 | A1 | Me | H | O | Ts | $3-CF_3C_6H_4$ | 2.46(s, 3H), 2.64(d, 3H, J=1.2Hz), 7.16(d, 2H, J=1.2Hz), 7.36(t, 2H, J=8.0Hz), 7.67(d, 1H, J=8.0Hz), 7.80(d, 1H, J=8.0Hz), 8.03(d, 2H, J=8.0Hz), 8.03(m, 2H). |
| 6-160 | A1 | Me | H | O | $SO_2NMe_2$ | $3-CF_3C_6H_4$ | 65-67 |
| 6-161 | A1 | Me | H | O | $SO_2Me$ | $3-CF_3OC_6H_4$ | 79-80 |
| 6-162 | A1 | Me | H | O | $SO_2CH_2Cl$ | $3-CF_3OC_6H_4$ | 2.68(d, 3H, J=1.2Hz), 5.30(s, 2H), 7.24(d, 1H, J=1.2Hz), 7.44(m, 1H), 7.63(t, 1H, J=8.0Hz), 7.79(s, 1H), 7.88(dt, 1H, J=8.0Hz, 1.6Hz) |
| 6-163 | A1 | Me | H | O | $SO_2$i-Pr | $3-CF_3OC_6H_4$ | 1.63(d, 6H, J=7.2Hz), 2.66(d, 3H, J=1.2Hz), 3.83(7-plet, 1H, J=7.2Hz), 7.22(d, 1H, J=1.2Hz), 7.41(m, 1H), 7.61(t, 1H, J=8.0Hz), 7.81(s, 1H), 7.90(d, 1H, J=8.0Hz). |
| 6-164 | A1 | Me | H | O | $SO_2Me$ | $3-CF_3SC_6H_4$ | 115-117 |
| 6-165 | A1 | Me | H | O | $SO_2CH_2Cl$ | $3-CF_3SC_6H_4$ | 85-87 |
| 6-166 | A1 | Me | H | O | $SO_2Me$ | $3-CHF_2SC_6H_4$ | 76-78 |
| 6-167 | A1 | Me | H | - | NHMe | $3-CF_3C_6H_4$ | 181-183 |
| 6-168 | A1 | Me | H | - | NHMe | $4-CF_3C_6H_4$ | 233 |
| 6-169 | A1 | Me | H | - | $NMe_2$ | $3-CF_3C_6H_4$ | 107 |
| 6-170 | A1 | Me | H | S | Me | $3-CF_3C_6H_4$ | 113 |
| 6-171 | A1 | Me | H | S | Me | $4-CF_3C_6H_4$ | 113 |

Table 6 (Continued)

| No. | A | $R^1$ | $R^2$ | $X^1$ | $R^3$ | $Q^2$ | m.p.(°C) or $^1$H-NMR ($CDCl_3$), δ(ppm) |
|---|---|---|---|---|---|---|---|
| 6-172 | A1 | Me | H | SO | Me | 3-$CF_3C_6H_4$ | 2.75(d, 3H, J=1.2Hz), 3.46(s, 3H), 7.33(d, 1H, J=1.2Hz),7.74(t, 1H, J=8.0Hz), 7.85(d, 1H, J=8.0Hz),8.19(d, 1H, J=8.0Hz), 8.22(s, 1H). |
| 6-173 | A1 | Me | H | SO | Me | 4-$CF_3C_6H_4$ | 171 |
| 6-174 | A1 | Me | H | $SO_2$ | Me | 3-$CF_3C_6H_4$ | 173 |
| 6-175 | A1 | Me | H | $SO_2$ | Me | 4-$CF_3C_6H_4$ | 153 |
| 6-176 | A1 | Me | H | OC(O) | Me | 3-$CF_3C_6H_4$ | 94-95 |
| 6-177 | A1 | Me | H | $OCO_2$ | Et | 3-$CF_3C_6H_4$ | 61-62 |
| 6-178 | A1 | Me | H | OC(O) | $NMe_2$ | 3-$CF_3C_6H_4$ | 2.66(d, 3H, J=1.2Hz), 3.07(s, 3H), 3.19(s, 3H), 7.15(d, 1H, J=1.2Hz), 7.68(t, 1H, J=8.0Hz),7.79(d, 1H, J=8.0Hz), 8.12(d, 1H, J=8.0Hz), 8.19(s, 1H). |
| 6-179 | A1 | Me | H | OC(O) | c-Pen | 3-$CF_3C_6H_4$ | 1.7-2.0(m, 4H), 2.1(m, 4H), 2.65(d, 3H, J=1.2Hz),3.12(fif, 1H, J=8.0Hz), 7.17(d, 1H, J=1.2Hz), 7.68(t, 1H, J=8.0Hz), 7.79(d, 1H, J=8.0Hz), 8.12(d, 1H, J=8.0Hz), 8.19(s, 1H). |
| 6-180 | A1 | Me | H | $CO_2$ | H | 3-$CF_3C_6H_4$ | 226-227(dec.) |
| 6-181 | A1 | Me | H | $CO_2$ | Et | 3-$CF_3C_6H_4$ | 134 |
| 6-182 | A1 | Me | H | CO | $NH_2$ | 3-$CF_3C_6H_4$ | 209 |
| 6-183 | A1 | Me | H | CO | NH-4-$FC_6H_4$ | 3-$CF_3C_6H_4$ | 218-220(dec.) |
| 6-184 | A1 | Me | H | O | 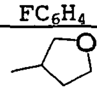 | 3-$CF_3C_6H_4$ | 133-134 |
| 6-185 | A1 | Me | H | O | 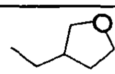 | 3-$CF_3C_6H_4$ | 1.82(m, 1H), 2.17(m, 1H), 2.59(d, 3H, J=1.2Hz), 2.87(m, 1H), 3.8(m, 2H), 4.0(m, 2H), 4.41(dd, 1H, J=8.0Hz, 10.4Hz), 4.47(dd, 1H, J=6.8Hz, 10.4Hz), 7.04(d, 1H, J=1.2Hz), 7.67(t, 1H, J=8.0Hz), 7.78(d, 1H, J=8.0Hz), 8.10(d, 1H, J=8.0Hz), 8.17(s, 1H). |
| 6-186 | A1 | Me | H | O | 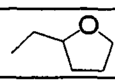 | 3-$CF_3C_6H_4$ | 72-73 |

Table 6 (Continued)

| No. | A | $R^1$ | $R^2$ | $X^1$ | $R^3$ | $Q^2$ | m.p. (°C) or $^1H$-NMR ($CDCl_3$), δ (ppm) |
|---|---|---|---|---|---|---|---|
| 6-187 | A1 | Me | H | O | 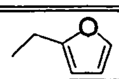 | 3-$CF_3C_6H_4$ | 115-117 |
| 6-188 | A1 | Me | H | O | 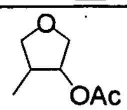 | 3-$CF_3C_6H_4$ | cis-rotational isomer: 2.04(s, 3H), 2.61(d, 3H, J=1.2Hz), 3.89(dd, 1H, J=5.2Hz, 9.2Hz), 4.10(m, 1H),4.33(dd, 1H, J=6.0Hz, 10Hz), 4.60(br.s, 1H), 5.54(q, 1H, J=5.4Hz), 7.07(d, 1H, J=1.2Hz), 7.69(t, 1H, J=8.0Hz), 7.80(d, 1H, J=8.0Hz),8.09(d, 1H, J=8.0Hz), 8.16(s, 1H). cis-rotational isomer : 1.99(s, 3H), 2.59(d, 3H, J=1.2Hz), 3.99(dd, 1H, J=4.8Hz, 10Hz), 4.08(dd, 1H, J=5.6Hz, 10Hz), 4.21(dd, 1H, J=5.6Hz, 10Hz), 4.32(dd, 1H, J=6.4Hz, 10Hz), 5.57(q, 1H, J=5.2Hz), 5.74(q, 1H, J=6.4Hz), 7.06(d, 1H, J=1.2Hz), 7.68(t, 1H, J=8.0Hz), 7.79(d, 1H, J=8.0Hz), 8.10(d, 1H, J=8.0Hz), 8.17(s, 1H). trans form : 2.09(s, 3H), 2.51(d, 3H, J=1.2Hz), 3.82(dd, 1H, J=4.4Hz, 10Hz), 4.1(m, 1H), 4.29(dd, 1H, J=5.2Hz, 9.2Hz), 4.4(m, 1H), 5.33(m, 1H), 6.06(fiftet, 1H, J=4.0Hz), 6.87(d, 1H, J=1.2Hz), 7.52(t, 1H, J=8.0Hz), 7.68(d, 1H, J=8.0Hz),7.86(d, 1H, J=8.0Hz), 8.17(s, 1H). |
| 6-189 | A1 | Me | H | - | pyrrol-1-yl | 3-$CF_3C_6H_4$ | 148-149 |
| 6-190 | A1 | Me | H | - | imidazol-1-yl | 3-$CF_3C_6H_4$ | 179-180 |
| 6-191 | A1 | Me | H | - | imidazol-1-yl | 2-$CF_3C_6H_4$ | 169 |
| 6-192 | A1 | Me | H | - | pyrazol-1-yl | 3-$CF_3C_6H_4$ | 153-155 |
| 6-193 | A1 | Me | H | - | triazol-1-yl | 3-$CF_3C_6H_4$ | 2.69(d, 1H, J=1.2Hz), 7.23(d, 1H, J=1.2Hz), 7.75(t, 1H, J=8.0Hz),7.86(d, 1H, J=8.0Hz), 8.19(d, 1H, J=8.0Hz), 8.21(s, 1H), 8.24(s, 1H), 9.35(s, 1H). |
| 6-194 | A1 | Me | H | - | 2-methylimidazo 1-1-yl | 3-$CF_3C_6H_4$ | 157 |

Table 6 (Continued)

| No. | A | $R^1$ | $R^2$ | $X^1$ | $R^3$ | $Q^2$ | m.p. (°C) or $^1$H-NMR ($CDCl_3$), δ (ppm) |
|---|---|---|---|---|---|---|---|
| 6-195 | A1 | Me | H | - | 3,5-dimethyl pyrazol-1-yl | $3-CF_3C_6H_4$ | 143-144 |
| 6-196 | A1 | Me | H | - | thiazolidin-3-yl | $3-CF_3C_6H_4$ | 2.56(d, 1H, J=1.2Hz), 2.91(br.t, 2H, J=7.6Hz), 3.35(br.t, 2H, J=7.6Hz), 3.61(br.s, 2H), 7.00(d, 1H, J=1.2Hz), 7.63(t, 1H, J=8.0Hz), 7.73(d, 1H, J=8.0Hz), 8.06(d, 1H, J=8.0Hz), 8.21(s, 1H). |
| 6-197 | A1 | Me | H | - | 2-Et-4-Me-imidazol-1-yl | $3-CF_3C_6H_4$ | 102-103 |
| 6-198 | A1 | Me | H | - | $3-CF_3$-pyrazol-1-yl | $3-CF_3C_6H_4$ | 147-148 |
| 6-199 | A1 | Me | H | - | $3-CF_3$-pyrazol-1-yl | $3-CF_3OC_6H_4$ | 116-117 |
| 6-200 | A1 | Me | H | - | $3-CF_3$-pyrazol-1-yl | $3-CF_3$-pyrazol-1-yl | 117-118 |
| 6-201 | A1 | Me | H | - | $3-CF_3$-pyrazol-1-yl | $3-CF_3C_6H_4O$ | 159-161 |
| 6-202 | A1 | Me | H | - | $3-CF_3$-pyrazol-1-yl | $3-CF_3OC_6H_4O$ | 153-155 |
| 6-203 | A1 | Me | H | - | $3-CF_3$-pyrazol-1-yl | SMe | 141-142 |
| 6-204 | A1 | Me | H | - | $3-CF_3$-pyrazol-1-yl | SOMe | 146-147 |
| 6-205 | A1 | Me | H | - | $3-CF_3$-pyrazol-1-yl | $SO_2Me$ | 171-173 |
| 6-206 | A1 | Me | H | - | $3-CF_3$-pyrazol-1-$CH_2$ | $3-CF_3C_6H_4$ | 2.66(d, 3H, J=1.2Hz), 5.71(s, 2H), 6.61(d, 1H, J=2.0Hz), 7.18(d, 1H, J=1.2Hz), 7.67(d, 1H, J=8.0Hz), 7.74(m, 1H), 7.78(t, 1H, J=8.0Hz), 8.06(d, 1H, J=8.0Hz), 8.13(s, 1H). |
| 6-207 | A1 | Me | H | O | $CH_2CF_3$ | $3-CF_3-4-F-C_6H_3$ | 2.62(d, 3H, J=1.2Hz), 4.92(q, 2H, J=8.4Hz), 7.07(d, 1H, J=1.2Hz), 7.39(t, 1H, J=9.0Hz), 8.13(m, 1H), 8.19(dd, 1H, J=2.1Hz, 6.0Hz). |

Table 6 (Continued)

| No. | A | $R^1$ | $R^2$ | $X^1$ | $R^3$ | $Q^2$ | m.p. (°C) or $^1H$-NMR ($CDCl_3$), δ (ppm) |
|---|---|---|---|---|---|---|---|
| 6-208 | A1 | Me | H | O | $CH_2CF_3$ | 3-$CF_3$-4-$CF_3CH_2O$-$C_6H_3$ | 2.61(d, 3H, J=1.2Hz), 4.54(q, 2H, J=7.8Hz), 4.92(q, 2H, J=8.4Hz), 7.08(d, 1H, J=1.2Hz), 7.15(d, 1H, J=8.7Hz), 8.15(dd, 1H, J=2.1Hz, 8.7Hz), 8.22(d, 1H, J=2.1Hz). |
| 6-209 | A1 | Me | H | O | i-Pr | 3-$CNC_6H_4$ | 117-118 |
| 6-210 | A1 | Me | H | O | $CH_2CF_3$ | 3-$CNC_6H_4$ | 132-133 |
| 6-211 | A1 | Me | H | O | $CH_2CF_2CHF_2$ | 3-$CNC_6H_4$ | 141 |
| 6-212 | A1 | Me | H | O | $SO_2Me$ | 3-$CNC_6H_4$ | 121-122 |
| 6-213 | A1 | Me | H | O | i-Pr | 4-$MeC_6H_4$ | 1.44(d, 6H, J=6.0Hz), 2.44(s, 3H), 2.55 (d, 3H, J=1.2Hz), 5.43(7-plet, 1H, J=6.4Hz), 7.10(q, 1H, J=1.2Hz), 7.32(d, 2H, J=8.0Hz), 7.83(d, 2H, J=8.0Hz). |
| 6-214 | A1 | Me | H | O | $CH_2CF_3$ | 4-$MeC_6H_4$ | 109-110 |
| 6-215 | A1 | Me | H | O | i-Pr | 3-$MeC_6H_4$ | 78-80 |
| 6-216 | A1 | Me | H | O | $CH_2CF_3$ | 3-$MeC_6H_4$ | 74-76 |
| 6-217 | A1 | Me | H | O | $CH_2CF_3$ | 2-$MeC_6H_4$ | 87-88 |
| 6-218 | A1 | Me | H | O | $CH_2CF_2CHF_2$ | 2-$MeC_6H_4$ | 111-112 |
| 6-219 | A1 | Me | H | O | $CH_2CH{=}CHCF_3$ | 3-$CF_3C_6H_4$ | 94-96 |
| 6-220 | A1 | Me | H | O | $CH_2CH{=}CHCF_3$ | 3-$CF_3OC_6H_4$ | 97-98 |
| 6-221 | A1 | Me | H | O | $CH_2CH{=}CHCF_3$ | 3-$CF_3SC_6H_4$ | 84-86 |
| 6-222 | A1 | Me | H | - | 4-$CF_3C_6H_4$ | 3-$CF_3C_6H_4O$ | 142-143 |
| 6-223 | A1 | Me | H | - | 4-$CF_3C_6H_4$ | 3-$CF_3OC_6H_4O$ | 157-159 |
| 6-224 | A2 | H | H | O | $CH_2CF_3$ | 3-$CF_3C_6H_4$ | 106 |
| 6-225 | A2 | H | H | O | $CH_2CF_3$ | 3-$CF_3OC_6H_4$ | 101-103 |
| 6-226 | A2 | H | H | O | i-Pr | 3-$CF_3C_6H_4$ | 83-85 |
| 6-227 | A2 | H | H | O | i-Pr | 3-$CF_3OC_6H_4$ | 52-54 |
| 6-228 | A2 | H | H | - | 3-$CF_3$-pyrazol-1-yl | 3-$CF_3OC_6H_4$ | 131 |
| 6-229 | A2 | H | H | O | $CH_2CF_3$ | 3-$CF_3C_6H_4O$ | 4.72(q, 2H, J=8.4Hz), 7.40(d, 1H, J=5.2Hz), 7.49(dt, 2H, J=2.0Hz, 6.4Hz), 7.56(br.s, 1H), 7.59(m, 2H), 7.99(d, 2H, J=5.2Hz). |

Table 6 (Continued)

| No. | A | $R^1$ | $R^2$ | $X^1$ | $R^3$ | $Q^2$ | m.p. (°C) or $^1$H-NMR ($CDCl_3$), δ(ppm) |
|---|---|---|---|---|---|---|---|
| 6-230 | A2 | H | H | O | $CH_2CF_3$ | 3-$CF_3OC_6H_4O$ | 4.72(q, 2H, J=8.4Hz), 7.19(m, 2H), 7.25(ddd, 1H, J=0.8Hz, 2.0Hz, 8.4Hz), 7.40(d, 2H, J=5.6Hz),7.49(t, 2H, J=8.4Hz), 7.98(d, 1H, J=5.6Hz). |
| 6-231 | A2 | Me | H | O | i-Pr | 3-$CF_3C_6H_4$ | 106 |
| 6-232 | A2 | Me | H | O | $CH_2CF_3$ | 3-$CF_3C_6H_4$ | 107-109 |
| 6-233 | A2 | Me | H | - | 3-$CF_3$-pyrazol-1-yl | 3-$CF_3C_6H_4$ | 141-142 |
| 6-234 | A2 | Me | H | O | i-Pr | 3-$CF_3OC_6H_4$ | 88-90 |
| 6-235 | A2 | Me | H | O | $CH_2CF_3$ | 3-$CF_3OC_6H_4$ | 77-78 |
| 6-236 | A2 | Me | H | O | 1-butyn-3-yl | 3-$CF_3OC_6H_4$ | 87-88 |
| 6-237 | A2 | Me | H | O | CH(Me)$CH_2$OMe | 3-$CF_3OC_6H_4$ | 1.45(d, 3H, J=6.4Hz), 2.68(d, 1H, J=1.2Hz), 3.43(s, 3H), 3.55(m, 1H), 3.58(dd, 1H, J=4.8Hz, 10.4Hz), 3.74(dd, 1H, J=6.4Hz, 10.4Hz), 7.10(d, 1H, J=1.2Hz), 7.39(dt, 1H, J=1.2Hz, 8.0Hz), 7.57(d, 1H, J=8.0Hz), 8.05(s, 1H), 8.10(dt, 1H, J=1.2Hz, 8.0Hz). |
| 6-238 | A2 | H | Me | O | i-Pr | 3-$CF_3C_6H_4$ | 73-74 |
| 6-239 | A2 | H | Me | O | $CH_2CF_3$ | 3-$CF_3C_6H_4$ | 99-100 |
| 6-240 | A2 | H | Me | O | $CH_2CF_2CHF_2$ | 3-$CF_3C_6H_4$ | 74-76 |
| 6-241 | A2 | H | Me | O | i-Pr | 3-$CF_3OC_6H_4$ | 63-65 |
| 6-242 | A2 | H | Me | O | $CH_2CF_3$ | 3-$CF_3OC_6H_4$ | 95-96 |
| 6-243 | A2 | H | Me | O | $CH_2CF_2CHF_2$ | 3-$CF_3OC_6H_4$ | 48-50 |
| 6-244 | A1 | Me | H | O | i-Pr | 2,3-$Me_2C_6H_4$ | 1.42(d, 6H, J=6.4Hz), 2.15(s, 1H), 2.36(s, 3H), 2.49(d, 1H, J=1.2Hz), 5.38(7-plet, 1H, J=6.4Hz), 6.59(d, 1H, J=1.2Hz), 7.2-7.3(m, 3H). |
| 6-245 | A1 | Me | H | O | $CH_2CF_3$ | 2,3-$Me_2C_6H_4$ | 98-99 |
| 6-246 | A1 | Me | H | O | $CH_2CF_2CHF_2$ | 2,3-$Me_2C_6H_4$ | 114 |
| 6-247 | A1 | Me | H | O | i-Pr | 2-$CF_3C_6H_4$ | 1.41(d, 6H, J=6.4Hz), 2.49(d, 1H, J=1.2Hz), 5.38(7-plet, 1H, J=6.4Hz), 6.51(d, 1H, J=1.2Hz), 7.45(d, 1H, J=8.0Hz), 7.64(m, 2H), 7.83(d, 1H, J=8.0Hz). |

Table 6 (Continued)

| No. | A | $R^1$ | $R^2$ | $X^1$ | $R^3$ | $Q^2$ | m.p.(°C) or $^1$H-NMR ($CDCl_3$), δ (ppm) |
|---|---|---|---|---|---|---|---|
| 6-248 | A1 | Me | H | O | $CH_2CF_3$ | 2-$CF_3C_6H_4$ | 2.53(d, 1H, J=1.2Hz),4.87(q, 2H, J=8.4Hz), 6.61(d, 1H, J=1.2Hz),7.47(d, 1H, J=8.0Hz), 7.66(m, 2H), 7.86(d, 1H, J=8.0Hz). |
| 6-249 | A1 | Me | H | O | $CH_2CF_2CHF_2$ | 2-$CF_3C_6H_4$ | 93-95 |
| 6-250 | A1 | Me | H | O | i-Pr | 2-$CF_3OC_6H_4$ | 1.43(d, 6H, J=6.4Hz), 2.52(d, 1H, J=1.2Hz), 5.39(7-plet, 1H, J=6.4Hz), 6.67(d, 1H, J=1.2Hz), 7.41(dt, 1H, J=8.0Hz, 1.2Hz), 7.43(dt, 1H, J=8.0Hz, 1.2Hz), 7.53(ddd, 1H, J=8.0Hz, 8.0Hz, 1.2Hz), 7.63(dd, 1H, J=8.0Hz, 2.0Hz). |
| 6-251 | A1 | Me | H | O | $CH_2CF_3$ | 2-$CF_3OC_6H_4$ | 2.56(d, 1H, J=1.2Hz), 4.90(q,2H, J=8.4Hz), 6.75(d, 1H, J=1.2Hz), 7.44(dt, 1H, J=8.0Hz, 1.2Hz), 7.46(dt, 1H, J=8.0Hz, 1.2Hz), 7.57(ddd, 1H, J=8.0Hz, 8.0Hz, 1.2Hz), 7.63(dd, 1H, J=8.0Hz, 2.0Hz). |
| 6-252 | A1 | Me | H | O | $CH_2CF_2CHF_2$ | 2-$CF_3OC_6H_4$ | 2.56(d, 1H, J=1.2Hz), 4.86(dt, 1H, J=12Hz, 1.2Hz), 6.15(tt, 1H, J=2.8Hz, 53.2Hz), 6.74(d, 1H, J=1.2Hz), 7.44(dt, 1H, J=8.0Hz, 1.2Hz), 7.46(dt, 1H, J=8.0Hz, 1.2Hz), 7.58(ddd, 1H, J=8.0Hz, 8.0Hz, 1.2Hz), 7.63(dd, 1H, J=8.0Hz, 2.0Hz). |
| 6-253 | A1 | Me | H | - | $NH_2$ | 3-$CF_3OC_6H_4$ | Solvent:DMSO-d6 2.2(br., 2H), 2.65(d, 3H, J=1.2Hz), 4.03(s, 2H), 7.41(d, 1H, J=1.2Hz), 7.85(t, 1H, J=8.0Hz), 7.98(d, 1H, J=8.0Hz), 8.26(s, 1H), 8.29(d, 1H, J=8.0Hz) |

Formulation Examples of the present invention are shown below. Parts and percents below are parts by weight and percents by weight, respectively.

Formulation Example 1 Wettable Powder

Forty parts of each of the compounds shown in Tables 1 to 6, 20 parts of Carplex #80 (trademark, Shionogi & Co., Ltd.), 35 parts of Kaoline Clay (trademark, Tsuchiya Kaoline K.K.), and 5 parts of a higher alcohol sulfate ester type surface active agent Sorpol 8070 (trademark, Toho Chemical Industry Co., Ltd.) were mixed and then uniformly ground to obtain a wettable powder containing 40% of the active ingredient.

Formulation Example 2 Emulsifiable Concentrate

Ten parts of each of the compounds shown in Tables 1 to 6 were dissolved in a mixed solvent of 40 parts of an aromatic hydrocarbon type solvent Solvesso 200 (trademark, Exxon Chemical Co.) and 40 parts of N,N-dimethylacetamide. Then, 10 parts of a polyoxyethylene type surface active agent Sorpol 3005X (trademark, Toho Chemical Industry Co., Ltd.) was added thereto and dissolved to obtain an emulsifiable concentrate containing 10% of the active ingredient.

Formulation Example 3 Flowable Agent

To 10 parts of each of the compounds shown in Tables 1 to 6 were added and dispersed 5 parts of Runox 1000C (trademark, Toho Chemical Industry Co., Ltd.), 3 parts of Carplex #80 (trademark, Shionogi & Co., Ltd.), 8 parts of ethylene glycol, and 54 parts of water. The resulting slurry mixture was wet type ground in Dynomill (trademark, WAB Co.). Then, 20 parts of a 1% aqueous xanthan gum solution which had been mixed and dissolved beforehand were added thereto and uniformly mixed to obtain a flowable agent containing 10% of the active ingredient.

Formulation Example 4 Granules

One part of each of the compounds shown in Tables 1 to 6, 43 parts of clay (manufactured by Nihon Talc K. K.), 55 parts of bentonite (manufactured by Hojun Yoko K.K.), and 1 part of a succinate type surface active agent Airrol CT-1 (trademark, Toho Chemical Industry Co., Ltd.) were mixed and ground. The resulting ground material were kneaded with 20 parts of water. Thereafter, the blend was extruded from an extrusion granulator through nozzles having a diameter of 0.6 mm, dried at 60°C for 2 hours, and then chopped to a length of 1 to 2 mm to obtain granules containing 1% of the active ingredient.
Test Examples of the herbicides of the present invention are shown below.

Test Example 1: Field Foliar Treatment Test

Alluvial soil from a field was put in a resin-made vat having an area of 200 $cm^2$. After fertilization, the seeds ofEchinochloa crus-galli, Brassica juncea, and Ipomoea purpurea were cast on the soil and uniformly covered with the soil. Then, cultivation was continued in a greenhouse, and when the weeds to be tested reached the 1.0- to 2.0-leaf stage, the wettable powder obtained in Formulation Example 1 was diluted with water and a predetermined amount thereof was sprayed uniformly to the weeds by means of a small-sized power pressure spray to give 10 g of the active ingredient per an are. Thereafter, cultivation was further continued in the greenhouse, and the herbicidal effect was examined on the 21 st day from the treatment. The results obtained are shown in Table 7 (the compound numbers in Table 7 correspond to those in Tables 1 to 6).
The herbicidal effect was evaluated in terms of herbicidal rate obtained from the following equation:

$$\text{Herbicidal rate (\%)} = [1 - (\text{Weight of weeds above the ground in a treated plot})/(\text{Weight of weeds above the ground in a non-treated plot})] \times 100$$

and was expressed by herbicidal index according to the following criteria.

| Herbicidal index | Herbicidal rate (%) |
|---|---|
| 0 | 0 to 5 |
| 1 | 6 to 30 |
| 2 | 31 to 50 |
| 3 | 51 to 70 |
| 4 | 71 to 90 |
| 5 | 91 to 100 |

In the following, the test results in Tables 7 to 9 are described by the index.

Table 7

| No. | Active ingredient amount (g/a) | Herbicidal effect | | |
|---|---|---|---|---|
| | | Echinochloa crus-galli | Brassica juncea | Ipomoea purpurea |
| 5-31 | 10 | 4 | 5 | 5 |

Table 7 (continued)

| No. | Active ingredient amount (g/a) | Herbicidal effect | | |
|---|---|---|---|---|
| | | Echinochloa crus-galli | Brassica juncea | Ipomoea purpurea |
| 6-25 | 10 | 4 | 5 | 5 |
| 6-29 | 10 | 4 | 4 | 5 |
| 6-35 | 10 | 5 | 5 | 5 |
| 6-39 | 10 | 5 | 4 | 5 |
| 6-40 | 10 | 4 | 5 | 5 |
| 6-61 | 10 | 4 | 4 | 4 |
| 6-80 | 10 | 5 | 5 | 5 |
| 6-83 | 10 | 5 | 4 | 5 |
| 6-85 | 10 | 5 | 4 | 5 |
| 6-90 | 10 | 5 | 5 | 5 |
| 6-92 | 10 | 5 | 5 | 4 |
| 6-96 | 10 | 4 | 5 | 5 |
| 6-97 | 10 | 5 | 5 | 5 |
| 6-98 | 10 | 5 | 4 | 5 |
| 6-109 | 10 | 5 | 5 | 4 |
| 6-112 | 10 | 5 | 5 | 5 |
| 6-115 | 10 | 5 | 5 | 5 |
| 6-125 | 10 | 5 | 5 | 5 |
| 6-130 | 10 | 2 | 4 | 3 |
| 6-142 | 10 | 5 | 4 | 5 |
| 6-184 | 10 | 5 | 4 | 5 |
| 6-187 | 10 | 4 | 5 | 5 |
| 6-192 | 10 | 4 | 4 | 5 |
| 6-193 | 10 | 4 | 4 | 5 |
| 6-194 | 10 | 4 | 5 | 5 |
| 6-202 | 10 | 4 | 4 | 5 |
| 6-207 | 10 | 5 | 5 | 5 |
| 6-215 | 10 | 5 | 5 | 5 |
| 6-217 | 10 | 4 | 5 | 5 |
| 6-231 | 10 | 4 | 5 | 5 |
| 6-235 | 10 | 5 | 5 | 5 |
| 6-236 | 10 | 5 | 5 | 5 |
| 6-245 | 10 | 4 | 5 | 5 |
| 6-249 | 10 | 4 | 5 | 5 |
| 6-251 | 10 | 5 | 5 | 5 |

Test Example 2: Field soil Treatment Test

Alluvial soil from a field was put in a resin-made vat having an area of 200 $cm^2$. After fertilization, the seeds of Digitaria ciliaris, Chenopodium album and Polygonum lapathifolium were cast on the soil and uniformly covered with the soil. The wettable powder obtained in Formulation Example 1 was diluted with water and a predetermined amount thereof was sprayed uniformly to the soil by means of a small-sized power pressure spray so as to give 10 g of the active ingredient per an are. Thereafter, cultivation was further continued in the greenhouse, and the herbicidal effect was examined on the 21 st day from the treatment. The results obtained are shown in Table 8 (the compound numbers in Table 8 correspond to those in Tables 1 to 6).

Table 8

| No. | Active ingredient amount (g/a) | Herbicidal effect | | |
|---|---|---|---|---|
| | | Digitaria ciliaris | Polygonum lapathifolium | Chenopodium album |
| 6-27 | 10 | 5 | 4 | 5 |
| 6-30 | 10 | 5 | 5 | 5 |
| 6-36 | 10 | 5 | 5 | 5 |
| 6-43 | 10 | 5 | 2 | 5 |
| 6-88 | 10 | 5 | 5 | 5 |
| 6-100 | 10 | 5 | 5 | 5 |
| 6-101 | 10 | 5 | 4 | 5 |
| 6-103 | 10 | 5 | 5 | 5 |
| 6-107 | 10 | 5 | 5 | 5 |
| 6-108 | 10 | 5 | 5 | 5 |
| 6-110 | 10 | 5 | 5 | 5 |
| 6-111 | 10 | 5 | 5 | 5 |
| 6-119 | 10 | 5 | 5 | 5 |
| 6-121 | 10 | 5 | 5 | 5 |
| 6-122 | 10 | 5 | 5 | 5 |
| 6-148 | 10 | 5 | 4 | 5 |
| 6-189 | 10 | 5 | 3 | 4 |
| 6-198 | 10 | 5 | 5 | 5 |
| 6-199 | 10 | 5 | 5 | 5 |

Test Example 3: Submerged Foliar Treatment Test

Alluvial soil from a paddy field was put in a resin-made vat having an area of 200 $cm^2$. After fertilization, seeds ofEchinochloa oryzicola, Rotala indica and Scirpus juncoides were cast on the soil and uniformly covered with the soil, and water was poured onto the soil to keep a water depth of 3 cm. Then, cultivation was continued in a greenhouse, and when the weeds to be tested reached the cotyledon stage to 1-leaf stage, the wettable powder obtained in Formulation Example 1 was diluted with water and a predetermined amount thereof was added dropwise uniformly to the weeds by means of a pipette to give 10 g of the active ingredient per an are. Thereafter, cultivation was further continued in the greenhouse, and the herbicidal effect was examined on the 28th day from the treatment. The results obtained are shown in Table 9 (the compound numbers in Table 9 correspond to those in Tables 1 to 6).

Table 9

| No. | Active ingredient amount (g/a) | Herbicidal effect | | |
|---|---|---|---|---|
| | | Echinochloa oryzicola | Scirpus juncoides | Rotala indica |
| 5-3 | 10 | 5 | 3 | 5 |
| 5-9 | 10 | 5 | 4 | 5 |
| 5-15 | 10 | 5 | 3 | 5 |
| 6-5 | 10 | 3 | 5 | 5 |
| 6-8 | 10 | 5 | 4 | 4 |
| 6-10 | 10 | 5 | 4 | 5 |
| 6-17 | 10 | 5 | 3 | 5 |
| 6-18 | 10 | 3 | 1 | 4 |
| 6-24 | 10 | 5 | 4 | 5 |
| 6-34 | 10 | 5 | 5 | 5 |
| 6-41 | 10 | 5 | 4 | 5 |
| 6-47 | 10 | 4 | 1 | 3 |
| 6-50 | 10 | 3 | 3 | 4 |
| 6-52 | 10 | 5 | 3 | 5 |
| 6-54 | 10 | 5 | 4 | 5 |
| 6-56 | 10 | 5 | 4 | 5 |
| 6-60 | 10 | 5 | 5 | 5 |
| 6-62 | 10 | 5 | 5 | 5 |
| 6-72 | 10 | 5 | 3 | 5 |
| 6-86 | 10 | 5 | 4 | 5 |
| 6-116 | 10 | 5 | 4 | 5 |
| 6-127 | 10 | 5 | 4 | 5 |
| 6-129 | 10 | 5 | 2 | 4 |
| 6-146 | 10 | 4 | 2 | 5 |
| 6-149 | 10 | 5 | 4 | 5 |
| 6-157 | 10 | 5 | 4 | 5 |
| 6-160 | 10 | 5 | 4 | 5 |
| 6-164 | 10 | 5 | 4 | 5 |
| 6-169 | 10 | 5 | 2 | 5 |
| 6-174 | 10 | 1 | 3 | 5 |
| 6-188 | 10 | 5 | 4 | 5 |
| 6-191 | 10 | 5 | 3 | 5 |
| 6-200 | 10 | 3 | 3 | 5 |
| 6-201 | 10 | 5 | 3 | 5 |

Industrial Applicability

According to the present invention, compounds which exhibit a high herbicidal effect and a sufficient safety

for important crops and which are useful as herbicides can be obtained.

## Claims

1. A herbicide comprising, as an active ingredient, a substituted thienopyrimidine derivative represented by formula (I):

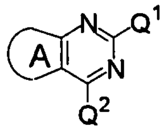

(I)

wherein A represents the following A1 or A2:

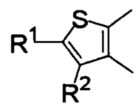

A1

wherein $R^1$ represents hydrogen or alkyl which may be substituted, and
$R^2$ represents hydrogen, halogen or alkyl which may be substituted:

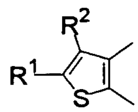

A2

wherein $R^1$ and $R^2$ have the same meanings as described above,
$Q^1$ represents hydrogen, halogen, cyano, hydroxyl, carboxyl, or $-X^1R^3$;
wherein $X^1$ is a single bond, -O-, $-SO_n-$ in which n represents an integer of 0 to 2, $-OSO_n-$ in which n has the same meaning as described above, -CO-, $-CO_2-$, $-OCO_2-$, or -OC(O)-, and
$R^3$ represents alkyl which may be substituted, alkenyl which may be substituted, alkynyl which may be substituted, amino which may be substituted, aryl which may be substituted, or a heterocyclic group which may be substituted,
$Q^2$ represents hydrogen, halogen, hydroxyl, or $-X^2R^4$,
wherein $X^2$ is a single bond, -O-, $-SO_n-$ in which n has the same meaning as described above, $-OSO_n-$ in which n has the same meaning as described above, -CO-, $-CO_2-$, $-OCO_2-$, or -OC(O)-, and
$R^4$ represents alkyl which may be substituted, alkenyl which may be substituted, alkynyl which may be substituted, amino which may be substituted, aryl which may be substituted, or a heterocyclic group which may be substituted.

2. A herbicide comprising, as an active ingredient, a substituted thienopyrimidine derivative represented by formula (I):

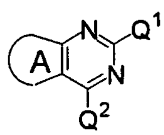

(I)

wherein A represents the following A1 or A2:

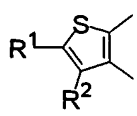

A1

wherein $R^1$ represents hydrogen, $(C_1\text{-}C_6)$alkyl, or halo$(C_1\text{-}C_6)$alkyl, and
$R^2$ represents hydrogen, halogen, $(C_1\text{-}C_6)$alkyl, or halo$(C_1\text{-}C_6)$alkyl:

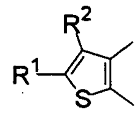

A2

wherein $R^1$ and $R^2$ have the same meanings as described above,
$Q^1$ represents hydrogen, halogen, cyano, hydroxyl, carboxyl, or $-X^1R^3$;
wherein $X^1$ is a single bond, -O-, $-SO_n-$ in which n represents an integer of 0 to 2, $-OSO_n-$ in which n has the same meaning as described above, -CO-, $-CO_2-$, $-OCO_2-$, or -OC(O)-, and

$R^3$ represents $(C_1\text{-}C_{10})$alkyl; halo$(C_1\text{-}C_{10})$alkyl; cyclo$(C_3\text{-}C_6)$alkyl; halocyclo$(C_3\text{-}C_6)$alkyl; $(C_1\text{-}C_6)$alkoxy$(C_1\text{-}C_6)$alkyl; halo$(C_1\text{-}C_6)$alkoxy$(C_1\text{-}C_6)$alkyl; $(C_1\text{-}C_4)$alkyl$(C_3\text{-}C_6)$cycloalkyl; cyclo$(C_3\text{-}C_6)$alkyl$(C_1\text{-}C_4)$alkyl; $(C_1\text{-}C_3)$alkoxycarbonyl$(C_1\text{-}C_3)$alkyl; (amino)hydroxy$(C_2\text{-}C_6)$alkyl; $(C_1\text{-}C_4)$alkylthio$(C_1\text{-}C_4)$alkyl; $(C_1\text{-}C_4)$alkylsulfinyl$(C_1\text{-}C_4)$alkyl; $(C_1\text{-}C_4)$alkylsulfonyl$(C_1\text{-}C_4)$alkyl; $(C_2\text{-}C_6)$alkenyl; halo$(C_2\text{-}C_6)$alkenyl; hydroxy$(C_2\text{-}C_6)$alkenyl; hydroxyhalo$(C_2\text{-}C_6)$alkenyl; phenyl$(C_2\text{-}C_6)$alkenyl; $(C_2\text{-}C_6)$alkynyl, halo$(C_2\text{-}C_6)$alkynyl; amino; mono$(C_1\text{-}C_6)$alkylamino; monohalo$(C_1\text{-}C_6)$alkylamino; di$(C_1\text{-}C_6)$alkylamino of which alkyl moieties are the same or different; dihalo$(C_1\text{-}C_6)$alkylamino of which dihaloalkyl moieties are the same or different;

phenylamino; substituted phenylamino having on its ring one or more substituents which are the same or different and are selected from the group consisting of halogen, cyano, nitro, hydroxyl, amino, SH, $(C_1\text{-}C_6)$alkyl, halo$(C_1\text{-}C_6)$alkyl, cyclo$(C_3\text{-}C_6)$alkyl, halocyclo$(C_3\text{-}C_6)$alkyl, $(C_2\text{-}C_6)$alkenyl, halo$(C_2\text{-}C_6)$alkenyl, $(C_2\text{-}C_6)$alkynyl, halo$(C_2\text{-}C_6)$alkynyl, $(C_1\text{-}C_6)$alkoxy, halo$(C_1\text{-}C_6)$alkoxy, cyclo$(C_3\text{-}C_6)$alkoxy, halocyclo$(C_3\text{-}C_6)$alkoxy, $(C_1\text{-}C_6)$alkylthio, halo$(C_1\text{-}C_6)$alkylthio, cyclo$(C_3\text{-}C_6)$alkylthio, halocyclo$(C_3\text{-}C_6)$alkylthio, $(C_1\text{-}C_6)$alkylsulfinyl, halo$(C_1\text{-}C_6)$alkylsulfinyl, cyclo$(C_3\text{-}C_6)$alkylsulfinyl, halocyclo$(C_3\text{-}C_6)$alkylsulfinyl, $(C_1\text{-}C_6)$alkylsulfonyl, halo$(C_1\text{-}C_6)$alkylsulfonyl, cyclo$(C_3\text{-}C_6)$alkylsulfonyl, halocyclo$(C_3\text{-}C_6)$alkylsulfonyl, mono$(C_1\text{-}C_6)$alkylamino, and di$(C_1\text{-}C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1\text{-}C_6)$alkyl, halo$(C_1\text{-}C_6)$alkyl, cyclo$(C_3\text{-}C_6)$alkyl, halocyclo$(C_3\text{-}C_6)$alkyl, $(C_2\text{-}C_6)$alkenyl, halo$(C_2\text{-}C_6)$alkenyl, $(C_2\text{-}C_6)$alkynyl, halo$(C_2\text{-}C_6)$alkynyl, $(C_1\text{-}C_6)$alkoxy, halo$(C_1\text{-}C_6)$alkoxy, cyclo$(C_3\text{-}C_6)$alkoxy, halocyclo$(C_3\text{-}C_6)$alkoxy, $(C_1\text{-}C_6)$alkylthio, halo$(C_1\text{-}C_6)$alkylthio, cyclo$(C_3\text{-}C_6)$alkylthio, halocyclo$(C_3\text{-}C_6)$alkylthio, $(C_1\text{-}C_6)$alkylsulfinyl, halo$(C_1\text{-}C_6)$alkylsulfinyl, cyclo$(C_3\text{-}C_6)$alkylsulfinyl, halocyclo$(C_3\text{-}C_6)$alkylsulfinyl, $(C_1\text{-}C_6)$alkylsulfonyl, halo$(C_1\text{-}C_6)$alkylsulfonyl, cyclo$(C_3\text{-}C_6)$alkylsulfonyl, halocyclo$(C_3\text{-}C_6)$alkylsulfonyl, mono$(C_1\text{-}C_6)$alkylamino, and di$(C_1\text{-}C_6)$alkylamino of which alkyl moieties are the same or different;

aryl$(C_1\text{-}C_6)$alkyl; substituted aryl$(C_1\text{-}C_6)$alkyl having on its ring one or more substituents which are the same or different and are selected from the group consisting of halogen atom, cyano, nitro, hydroxyl, amino, SH, $(C_1\text{-}C_6)$alkyl, halo$(C_1\text{-}C_6)$alkyl, cyclo$(C_3\text{-}C_6)$alkyl, halocyclo$(C_3\text{-}C_6)$alkyl, $(C_2\text{-}C_6)$alkenyl, halo$(C_2\text{-}C_6)$alkenyl, $(C_2\text{-}C_6)$alkynyl, halo$(C_2\text{-}C_6)$alkynyl, $(C_1\text{-}C_6)$alkoxy, halo$(C_1\text{-}C_6)$alkoxy, cyclo$(C_3\text{-}C_6)$alkoxy, halocyclo$(C_3\text{-}C_6)$alkoxy, $(C_1\text{-}C_6)$alkylthio, halo$(C_1\text{-}C_6)$alkylthio, cyclo$(C_3\text{-}C_6)$alkylthio, halocyclo$(C_3\text{-}C_6)$alkylthio, $(C_1\text{-}C_6)$alkylsulfinyl, halo$(C_1\text{-}C_6)$alkylsulfinyl, cyclo$(C_3\text{-}C_6)$alkylsulfinyl, halocyclo$(C_3\text{-}C_6)$alkylsulfinyl, $(C_1\text{-}C_6)$alkylsulfonyl, halo$(C_1\text{-}C_6)$alkylsulfonyl, cyclo$(C_3\text{-}C_6)$alkylsulfonyl, halocyclo$(C_3\text{-}C_6)$alkylsulfonyl, mono$(C_1\text{-}C_6)$alkylamino, and di$(C_1\text{-}C_6)$alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazole, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, $(C_1\text{-}C_6)$alkyl, halo$(C_1\text{-}C_6)$alkyl, cyclo$(C_3\text{-}C_6)$alkyl, halocyclo$(C_3\text{-}C_6)$alkyl, $(C_1\text{-}C_6)$alkylcarbonyloxy, $(C_1\text{-}C_6)$alkoxy, halo$(C_1\text{-}C_6)$alkoxy, cyclo$(C_3\text{-}C_6)$alkoxy, and halocyclo$(C_3\text{-}C_6)$alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy,

$Q^2$ represents hydrogen, halogen, hydroxyl, or $-X^2R^4$;

wherein $X^2$ represents a single bond, -O-, $-SO_n-$ in which n has the same meaning as described above, $-OSO_n-$ in which n has the same meaning as described above, -CO-, $-CO_2-$, $-OCO_2-$, or -OC(O)-, and

$R^4$ represents ($C_1$-$C_{10}$)alkyl; halo($C_1$-$C_{10}$)alkyl; cyclo($C_3$-$C_6$)alkyl; halocyclo($C_3$-$C_6$)alkyl; ($C_1$-$C_6$)alkoxy ($C_1$-$C_6$)alkyl; halo($C_1$-$C_6$)alkoxy($C_1$-$C_6$)alkyl; ($C_1$-$C_4$)alkylcyclo($C_3$-$C_6$)alkyl; cyclo($C_3$-$C_6$)alkyl($C_1$-$C_4$)alkyl; ($C_1$-$C_3$)alkoxycarbonyl($C_1$-$C_3$)alkyl; ($C_1$-$C_4$)alkylthio($C_1$-$C_4$)alkyl; ($C_1$-$C_4$)alkylsulfinyl($C_1$-$C_4$)alkyl; ($C_1$-$C_4$)alkylsulfonyl($C_1$-$C_4$)alkyl; ($C_2$-$C_6$)alkenyl; halo($C_2$-$C_6$)alkenyl; hydroxy($C_2$-$C_6$)alkenyl; hydroxyhalo($C_2$-$C_6$)alkenyl; phenyl ($C_2$-$C_6$)alkenyl; ($C_2$-$C_6$)alkynyl; halo($C_2$-$C_6$)alkynyl; amino; mono($C_1$-$C_6$)alkylamino; monohalo($C_1$-$C_6$)alkylamino; di($C_1$-$C_6$)alkylamino of which alkyl moieties are the same or different; dihalo($C_1$-$C_6$)alkylamino of which haloalkyl moieties are the same or different;

phenylamino; substituted phenylamino having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo ($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

aryl; substituted aryl having one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

aryl($C_1$-$C_6$)alkyl; substituted aryl($C_1$-$C_6$)alkyl having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo ($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

a heterocyclic group of which heterocycle has the same meaning as described above; a substituted heterocyclic group of which heterocycle has the same meaning as described above, having one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy.

**3.** The herbicide according to claim 1 or 2,

wherein $Q^1$ is $OR^3$,

wherein $R^3$ represents ($C_1$-$C_{10}$)alkyl; halo($C_1$-$C_{10}$)alkyl; cyclo($C_3$-$C_6$)alkyl; ($C_1$-$C_6$)alkoxy($C_1$-$C_6$)alkyl; halo ($C_1$-$C_6$)alkoxy($C_1$-$C_6$)alkyl; ($C_1$-$C_4$)alkyl($C_3$-$C_6$)cycloalkyl; cyclo($C_3$-$C_6$)alkyl($C_1$-$C_4$)alkyl; ($C_1$-$C_3$)alkoxycarbonyl ($C_1$-$C_3$)alkyl; (amino)hydroxy($C_2$-$C_6$)alkyl; ($C_1$-$C_4$)alkylthio($C_1$-$C_4$)alkyl; ($C_1$-$C_4$)alkylsulfinyl($C_1$-$C_4$)alkyl; ($C_1$-$C_4$)alkylsulfonyl($C_1$-$C_4$)alkyl; ($C_2$-$C_6$)alkenyl; halo($C_2$-$C_6$)alkenyl; hydroxy($C_2$-$C_6$)alkenyl; hydroxyhalo($C_2$-$C_6$)alkenyl; phenyl($C_2$-$C_6$)alkenyl; ($C_2$-$C_6$)alkynyl; halo($C_2$-$C_6$)alkynyl; amino; mono($C_1$-$C_6$)alkylamino; monohalo($C_1$-$C_6$)alkylamino; di($C_1$-$C_6$)alkylamino of which alkyl moieties are the same or different; dihalo($C_1$-$C_6$)alkylamino of which

dihaloalkyl moieties are the same or different;

phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo $(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo $(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;

aryl$(C_1-C_6)$alkyl; substituted aryl$(C_1-C_6)$alkyl having on its ring one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo $(C_1-C_6)$alkoxy, $(C_1-C_6)$alkylthio, halo$(C_1-C_6)$alkylthio, (Ci-$C_6$)alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazole, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, $(C_1-C_6)$alkyl, halo$(C_1-C_6)$alkyl, $(C_1-C_6)$alkylcarbonyloxy, $(C_1-C_6)$alkoxy, and halo$(C_1-C_6)$alkoxy;

heterocyclic $(C_1-C_6)$alkyl of which heterocycle has the same meaning as described above; or substituted heterocyclic $(C_1-C_6)$alkyl of which heterocycle has the same meaning as described above, having, on its ring, one or more substituents which are same or different and are selected from the group consisting of halogen, cyano, nitro, $(C_1-C_6)$alkyl, halo$(C_1-C_6)$alkyl, $(C_1-C_6)$alkylcarbonyloxy, $(C_1-C_6)$alkoxy, and halo$(C_1-C_6)$alkoxy.

**4.** The herbicide according to any one of claims 1 to 3,

wherein $Q^2$ is $(C_1-C_{10})$alkyl; cyclo$(C_3-C_6)$alkyl; $(C_1-C_6)$alkylthio; cyclo$(C_3-C_6)$alkylthio; $(C_1-C_6)$alkylsulfinyl; cyclo$(C_3-C_6)$alkylsulfinyl; $(C_1-C_6)$alkylsulfonyl; cyclo$(C_3-C_6)$alkylsulfonyl;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazole, tetrahydropyran, pyridine, pyrimidine, pyridazine, morpholine, thiomorpholine, piperazine, piperidine, or oxazine;

a substituted heterocyclic group of which heterocycle has the same meaning as described above, having, its on ring, one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

heterocyclic oxy of which heterocycle has the same meaning as described above; heterocyclic oxy of which heterocycle has the same meaning as described above, having, its on ring, one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio; or

$-X^2R^4$,

wherein $X^2$ is a single bond, -O-, -S-, -SO- or $-SO_2-$; and

$R^4$ is phenyl which is substituted with one or more substituents which are the same or different and selected from the group consisting of fluorine-containing $(C_1-C_6)$alkyl, fluorine-containing $(C_1-C_6)$alkoxy, and fluorine-containing $(C_1-C_6)$alkylthio and which is optionally substituted with one or more substituents which are the same or different and are selected from the group consisting of halogen, cyano, $(C_1-C_6)$alkyl, halo$(C_1-C_6)$alkyl, cyclo$(C_3-C_6)$ alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$ alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, and halocyclo$(C_3-C_6)$alkylsulfonyl.

**5.** The herbicide according to claim 1 or 2,

wherein $Q^1$ is hydrogen; halogen; cyano; hydroxyl; carboxyl; $(C_1\text{-}C_{10})$alkyl; halo$(C_1\text{-}C_6)$alkyl; $(C_1\text{-}C_6)$alkoxy$(C_1\text{-}C_6)$alkyl; halo$(C_1\text{-}C_6)$alkoxy$(C_1\text{-}C_6)$alkyl; $(C_1\text{-}C_{10})$alkoxy; halo$(C_1\text{-}C_6)$alkoxy; cyclo$(C_3\text{-}C_6)$alkoxy; halocyclo$(C_3\text{-}C_6)$alkoxy; $(C_1\text{-}C_6)$alkoxy$(C_1\text{-}C_6)$alkoxy; halo$(C_1\text{-}C_6)$alkoxy$(C_1\text{-}C_6)$alkoxy;$(C_1\text{-}C_3)$alkoxycarbonyl$(C_1\text{-}C_3)$alkoxy; (amino)hydroxy$(C_2\text{-}C_6)$alkoxy; $(C_1\text{-}C_6)$alkylthio$(C_1\text{-}C_6)$alkoxy; $(C_1\text{-}C_6)$alkylsulfinyl$(C_1\text{-}C_6)$alkoxy; $(C_1\text{-}C_6)$alkylsulfonyl$(C_1\text{-}C_6)$alkoxy; di$(C_1\text{-}C_3)$alkylamino$(C_1\text{-}C_3)$alkoxy of which alkyl moieties are the same or different; $(C_2\text{-}C_6)$alkenyl; halo$(C_2\text{-}C_6)$alkenyl; hydroxyhalo$(C_2\text{-}C_6)$alkenyl; $(C_2\text{-}C_6)$alkenyloxy; halo$(C_2\text{-}C_6)$alkenyloxy; $(C_2\text{-}C_6)$alkynyloxy; amino; mono$(C_1\text{-}C_6)$alkylamino; di$(C_1\text{-}C_6)$alkylamino of which alkyl moieties are the same or different; substituted phenyl which is substituted with halo$(C_1\text{-}C_3)$alkyls which are the same or different; pyrrolyl; imidazolyl; substituted imidazolyl which is substituted with one or more $(C_1\text{-}C_3)$alkyl which are the same or different; pyrazolyl; substituted pyrazolyl which is substituted with one or more $(C_1\text{-}C_3)$alkyls or halo$(C_1\text{-}C_3)$alkyls which are the same or different; substituted pyrazolyl$(C_1\text{-}C_3)$alkyl which is substituted with one or more halo$(C_1\text{-}C_3)$alkyls which are the same or different; triazolyl; phenoxy; substituted phenoxy which is substituted with one or more substituents which are the same or different and are selected from the group consisting of halo$(C_1\text{-}C_3)$alkyl and halo$(C_1\text{-}C_3)$alkoxy; $(C_1\text{-}C_3)$alkylthio; $(C_1\text{-}C_3)$alkylsulfinyl; $(C_1\text{-}C_3)$alkylsulfonyl; $(C_1\text{-}C_6)$alkylsulfonyloxy; halo$(C_1\text{-}C_6)$alkylsulfonyloxy; phenylsulfonyloxy; substituted phenylsulfonyloxy which is substituted with one or more $(C_1\text{-}C_3)$alkyls which are the same or different; di$(C_1\text{-}C_3)$alkylaminosulfonyloxy of which alkyl moieties are the same or different; $(C_2\text{-}C_4)$alkenylsulfonyloxy; $(C_1\text{-}C_3)$alkylcarbonyloxy; $(C_1\text{-}C_3)$alkoxycarbonyloxy; $(C_1\text{-}C_3)$alkoxycarbonyl; aminocarbonyl; or substituted phenylaminocarbonyl which is substituted with one or more halogens which are the same or different,

$Q^2$ represents halogen; hydroxyl; $(C_1\text{-}C_6)$alkyl; halo$(C_1\text{-}C_6)$alkyl; cyclo$(C_3\text{-}C_6)$alkyl; halocyclo$(C_3\text{-}C_6)$alkyl; $(C_1\text{-}C_6)$alkoxy; halo$(C_1\text{-}C_6)$alkoxy; $(C_1\text{-}C_6)$alkylthio; $(C_1\text{-}C_6)$alkylsulfinyl; $(C_1\text{-}C_6)$alkylsulfonyl; substituted phenyl having one or more substituents which are the same or different and are selected from the group consisting of halogen, cyano, $(C_1\text{-}C_3)$alkyl, halo$(C_1\text{-}C_3)$alkyl, $(C_1\text{-}C_3)$alkoxy, halo$(C_1\text{-}C_3)$alkoxy, and halo$(C_1\text{-}C_3)$alkylthio; substituted pyrazolyl having one or more substituents which are the same or different and are selected from the group consisting of halogen, $(C_1\text{-}C_3)$alkyl, and halo$(C_1\text{-}C_3)$alkyl; furyl; substituted thienyl which is substituted with one or more $(C_1\text{-}C_3)$alkyls which are the same or different; substituted pyridyl having one halogens which are the same or different; or substituted phenoxy having one or more substituents which are the same or different and are selected from the group consisting of halogen, cyano, $(C_1\text{-}C_3)$alkyl, halo$(C_1\text{-}C_3)$alkyl, $(C_1\text{-}C_3)$alkoxy, halo$(C_1\text{-}C_3)$alkoxy, and halo$(C_1\text{-}C_3)$alkylthio.

**6.** A herbicidal method, which comprises carrying out a soil treatment, a field foliar treatment, or an irrigation treatment with an effective amount of the herbicide according to any one of claims 1 to 5.

**7.** A substituted thienopyrimidine derivative represented by formula (I):

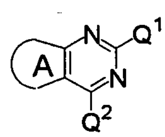

( I )

wherein A represents the following A1 or A2:

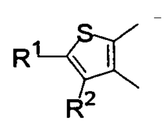

A1

wherein $R^1$ represents hydrogen, $(C_1\text{-}C_6)$alkyl, or halo$(C_1\text{-}C_6)$alkyl,
$R^2$ represents hydrogen, halogen, $(C_1\text{-}C_6)$alkyl, or halo$(C_1\text{-}C_6)$alkyl:

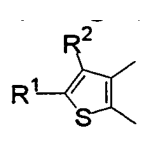

A2

wherein $R^1$ and $R^2$ have the same meanings as described above,

$Q^1$ represents hydrogen, halogen, cyano, hydroxyl, carboxyl, or $-X^1R^3$,

wherein $X^1$ is a single bond, -O-, $-SO_n-$ in which n represents an integer of 0 to 2, $-OSO_n-$ in which n has the same meaning as described above, -CO-, $-CO_2-$, $-OCO_2-$, or -OC(O)-,

$R^3$ represents $(C_1-C_{10})$alkyl; halo$(C_1-C_{10})$alkyl; cyclo$(C_3-C_6)$alkyl; halocyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkyl$(C_3-C_6)$cycloalkyl; cyclo$(C_3-C_6)$alkyl$(C_1-C_4)$alkyl; $(C_1-C_3)$alkoxycarbonyl$(C_1-C_3)$alkyl; (amino)hydroxy$(C_2-C_6)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which haloalkyl moieties are the same or different; phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl$(C_1-C_6)$alkyl; substituted aryl$(C_1-C_6)$alkyl having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazole, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy;

heterocyclic $(C_1-C_6)$alkyl of which heterocycle has the same meaning as described above; or substituted heterocyclic $(C_1-C_6)$alkyl, wherein the heterocycle has the same meaning as described above, having, on its ring, one or more substituents which are the same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy,

$Q^2$ represents hydrogen, halogen, hydroxyl, or $-X^2R^4$;

wherein $X^2$ is a single bond, -O-, $-SO_n-$ in which n has the same meaning as described above, $-OSO_n-$ in which n has the same meaning as described above, -CO-, $-CO_2-$, $-OCO_2-$, or -OC(O)-, and

$R^4$ represents $(C_1-C_{10})$alkyl; halo$(C_1-C_{10})$alkyl; cyclo$(C_3-C_6)$alkyl; halocyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkylcyclo$(C_3-C_6)$alkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which haloalkyl moieties are the same or different;

phenylamino; substituted phenylamino having on its ring one or more substituents which are the same or

different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo $(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$ alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$ alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl$(C_1-C_6)$alkyl; substituted aryl$(C_1-C_6)$alkyl having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo $(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

a heterocyclic group of which heterocycle has the same meaning as described above; a substituted heterocyclic group of which heterocycle has the same meaning as described above, having one or more substituents which are same or different and are selected from the group consisting of halogen, cyano, nitro, $(C_1-C_6)$alkyl, halo $(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$alkoxy, halo$(C_1-C_6)$alkoxy, cyclo$(C_3-C_6)$alkoxy, and halocyclo$(C_3-C_6)$alkoxy;

heterocyclic $(C_1-C_6)$alkyl of which heterocycle has the same meaning as described above; or substituted heterocyclic $(C_1-C_6)$alkyl of which heterocycle has the same meaning as described above, having, on its ring, one or more substituents which are the same or different and are selected from the group consisting of halogen, cyano, nitro, $(C_1-C_6)$alkyl, halo$(C_1-C_6)$alkyl, cyclo$(C_3-C_6)$alkyl, halocyclo$(C_3-C_6)$alkyl, $(C_1-C_6)$alkylcarbonyloxy, $(C_1-C_6)$ alkoxy, halo$(C_1-C_6)$alkoxy, cyclo$(C_3-C_6)$alkoxy, and halocyclo$(C_3-C_6)$alkoxy.

**8.** The substituted thienopyrimidine derivative according to claim 7,

wherein A represents A1,

$Q^1$ represents $OR^3$, wherein $R^3$ has the same meaning as defined in claim 7,

$Q^2$ represents hydrogen, halogen, hydroxyl, or $-X^2R^4$,

wherein $X^2$ has the same meaning as defined in claim 7, and

$R^4$ is $(C_1-C_{10})$alkyl; halo$(C_1-C_{10})$alkyl; cyclo$(C_3-C_6)$alkyl; $(C_1-C_6)$alkoxy$(C_1-C_6)$alkyl; halo$(C_1-C_6)$alkoxy $(C_1-C_6)$alkyl; $(C_1-C_4)$alkylcyclo$(C_3-C_6)$alkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which haloalkyl moieties are the same or different;

phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo $(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;

substituted aryl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;

a heterocyclic ring of which heterocycle has the same meaning as defined in claim 7; a substituted heterocyclic ring of which heterocycle has the same meaning as described above, having one or more substituents which are the same or different and are selected from the group consisting of halogen, cyano, nitro, $(C_1-C_6)$alkyl, halo $(C_1-C_6)$alkyl, $(C_1-C_6)$alkylcarbonyloxy, $(C_1-C_6)$alkoxy, and halo$(C_1-C_6)$alkoxy, and

wherein

(1) when $X^2$ is a single bond, $R^4$ is not amino, mono($C_1$-$C_6$)alkylamino or monohalo($C_1$-$C_6$)alkylamino, and
(2) when $X^2$ is -O-, $R^4$ is not ($C_1$-$C_{10}$)alkyl.

**9.** The substituted thienopyrimidine derivative according to claim 7,
wherein A represents A1,
$Q^1$ represents hydrogen, halogen, cyano, hydroxyl, carboxyl, or -$X^1R^3$;
wherein $X^1$ is a single bond, -$SO_n$- in which n represents an integer of 0 to 2, -$OSO_n$- in which n has the same meaning as described above, -CO-, -$CO_2$-, -$OCO_2$-, or -OC(O)-;
$R^3$ has the same meaning as defined in claim 7,
$Q^2$ represents substituted aryl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio;
substituted aryloxy having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio;
substituted arylthio having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio;
substituted arylsulfinyl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio; or
substituted arylsulfonyl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio, and
wherein, when $Q^1$ is ($C_1$-$C_6$)alkyl in which $X^1$ is a single bond and $R^3$ is ($C_1$-$C_6$)alkyl, $Q^2$ is not substituted aryloxy which is substituted with at least one fluorine-containing alkyl.

**10.** The substituted thienopyrimidine derivative according to claim 7,
wherein A represents A1,
$Q^1$ represents hydrogen; halogen; cyano; hydroxyl; carboxyl; ($C_1$-$C_{10}$)alkyl; halo($C_1$-$C_6$)alkyl; ($C_1$-$C_6$)alkoxy($C_1$-$C_6$)alkyl; halo($C_1$-$C_6$)alkoxy($C_1$-$C_6$)alkyl; ($C_1$-$C_{10}$)alkoxy; halo($C_1$-$C_6$)alkoxy; cyclo($C_3$-$C_6$)alkoxy; halocyclo($C_3$-$C_6$)alkoxy; ($C_1$-$C_6$)alkoxy($C_1$-$C_6$)alkoxy; halo($C_1$-$C_6$)alkoxy($C_1$-$C_6$)alkoxy; ($C_1$-$C_3$)alkoxycarbonyl($C_1$-$C_3$)alkoxy; (amino)hydroxy($C_2$-$C_6$)alkoxy; ($C_1$-$C_6$)alkylthio($C_1$-$C_6$)alkoxy; ($C_1$-$C_6$)alkylsulfinyl($C_1$-$C_6$)alkoxy; ($C_1$-$C_6$)alkylsulfonyl($C_1$-$C_6$)alkoxy; di($C_1$-$C_3$)alkylamino($C_1$-$C_3$) alkoxy of which alkyl moieties are the same or different; ($C_2$-$C_6$)alkenyl; halo($C_2$-$C_6$)alkenyl; hydroxyhalo($C_2$-$C_6$)alkenyl; ($C_2$-$C_6$)alkenyloxy; halo($C_2$-$C_6$)alkenyloxy; ($C_1$-$C_6$)alkynyloxy; amino; mono($C_1$-$C_6$)alkylamino; di($C_1$-$C_6$)alkylamino of which alkyl moieties are the same or different; substituted phenyl which is substituted with one or more halo($C_1$-$C_3$)alkyls which are the same or different; pyrrolyl; imidazolyl; substituted imidazolyl which is substituted with one or more ($C_1$-$C_3$)alkyls which are the same or different; pyrazolyl; substituted pyrazolyl having one or more substituents which are the same or different and are selected from ($C_1$-$C_3$)alkyl and halo($C_1$-$C_3$)alkyl; substituted pyrazolyl($C_1$-$C_3$)alkyl having one or more substituents which are the same or different and are selected from the group consisting of halo($C_1$-$C_3$)alkyl; triazolyl; phenoxy; substituted phenoxy having one or more substituents which are the same or different and are selected from the group consisting of halo($C_1$-$C_3$)alkyl and halo($C_1$-$C_3$)alkoxy; ($C_1$-$C_3$)alkylthio; ($C_1$-$C_3$)alkylsulfinyl; ($C_1$-$C_3$)alkylsulfonyl; ($C_1$-$C_6$)alkylsulfonyloxy; halo($C_1$-$C_6$)alkylsulfonyloxy; phenylsulfonyloxy; substituted phenylsulfonyloxy having one or more substituents which is substituted with one or more ($C_1$-$C_3$)alkyls which are the same or different; di($C_1$-$C_3$)alkylaminosulfonyloxy of which alkyl moieties are the same or different; ($C_2$-$C_4$)alkenylsulfonyloxy; ($C_1$-$C_3$)alkylcarbonyloxy; ($C_1$-$C_3$)alkoxycarbonyl; aminocarboxyl; substituted phenylaminocarbonyl which is substituted with one or more halogens on its ring which are the same or different,
$Q^2$ represents halogen; hydroxyl; ($C_1$-$C_6$)alkyl; halo($C_1$-$C_6$)alkyl; cyclo($C_3$-$C_6$)alkyl; halocyclo($C_3$-$C_6$)alkyl; ($C_1$-$C_6$)alkoxy; halo($C_1$-$C_6$)alkoxy; ($C_1$-$C_6$)alkylthio; ($C_1$-$C_6$)alkylsulfinyl; ($C_1$-$C_6$)alkylsulfonyl; substituted phenyl having one or more substituents which are the same or different and are selected from the group consisting of halogen, cyano, ($C_1$-$C_3$)alkyl, halo($C_1$-$C_3$)alkyl, ($C_1$-$C_3$)alkoxy, halo($C_1$-$C_3$)alkoxy, and halo($C_1$-$C_3$)alkylthio; substituted pyrazolyl having one or more substituents which are the same or different and are selected from the group consisting of halogen, ($C_1$-$C_3$)alkyl, and halo($C_1$-$C_3$)alkyl; furyl; substituted thienyl substituted with one or more ($C_1$-$C_3$)alkyls which are the same or different; substituted pyridyl substituted with one or more halogens which are the same or different; or substituted phenoxy having one or more substituents which are the same or different and

are selected from the group consisting of halogen, cyano, $(C_1-C_3)$alkyl, halo$(C_1-C_3)$alkyl, $(C_1-C_3)$alkoxy, halo$(C_1-C_3)$alkoxy, and halo$(C_1-C_3)$alkylthio.

**11.** The substituted thienopyrimidine derivative according to any one of claims 7, 9 and 10, wherein A represents A1, and $Q^1$ represents halogen or hydroxyl.

**12.** The substituted thienopyrimidine derivative according to claim 7,
wherein A is A2,
$Q^1$ represents $-OR^3$, wherein $R^3$ has the same meaning as defined in claim 7,
$Q^2$ represents hydrogen, halogen, hydroxyl, or $-X^2R^4$,
wherein $X^2$ is a single bond, -O-, $-SO_n-$ in which n represents an integer of 0 to 2, $-OSO_n-$ in which n has the same meaning as described above, $-CO_2-$, $-OCO_2-$, or -OC(O)-;
$R^4$ represents $(C_1-C_{10})$alkyl; halo$(C_1-C_{10})$alkyl; cyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkylcyclo$(C_3-C_6)$alkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl$(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which alkyl moieties are the same or different;
phenylamino; substituted phenylamino which having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different;
aryl; substituted aryl which having one or more substituents which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different, and
wherein, when $X^2$ is a single bond, $R^4$ is not amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which alkyl moieties are the same or different; phenylamino; substituted phenylamino which has one or more substituents on its ring which are the same or different and are selected from the group consisting of halogen, cyano, nitro, hydroxyl, amino, SH, $(C_1-C_6)$alkyl, halo$(C_1-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, $(C_1-C_6)$alkylthio, halo$(C_1-C_6)$alkylthio, $(C_1-C_6)$alkylsulfinyl, halo$(C_1-C_6)$alkylsulfinyl, $(C_1-C_6)$alkylsulfonyl, halo$(C_1-C_6)$alkylsulfonyl, mono$(C_1-C_6)$alkylamino, and di$(C_1-C_6)$alkylamino of which alkyl moieties are the same or different; $(C_1-C_{10})$alkyl; halo$(C_1-C_{10})$alkyl; cyclo$(C_3-C_6)$alkyl; $(C_1-C_6)$alkoxy$(C_1-C_6)$alkyl; halo$(C_1-C_6)$alkoxy$(C_1-C_6)$alkyl; $(C_1-C_4)$alkyl$(C_3-C_6)$cycloalkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; or $(C_1-C_4)$alkylsulfonyl$(C_1-C_4)$alkyl.

**13.** The substituted thienopyrimidine derivative according to claim 7,
wherein A is A2,
$Q^1$ represents halogen, cyano, carboxyl, or $-X^1R^3$;
wherein $X^1$ is a single bond, $-SO_n-$ in which n represents an integer of 0 to 2, $-OSO_n-$ in which n has the same meaning as described above, -CO-, $-CO_2-$, $-OCO_2-$, or -OC(O)-, and
$R^3$ has the same meaning as defined in claim 7, and
$Q^2$ represents:

substituted aryl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
substituted aryloxy having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
substituted arylthio having one or more substituents which are the same or different and are selected from the group consisting of fluorine-containing $(C_1-C_6)$alkoxy, and fluorine-containing $(C_1-C_6)$alkylthio;

substituted arylsulfinyl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio; or

substituted arylsulfonyl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio.

**14.** The substituted thienopyrimidine derivative according to claim 7,

wherein A is A2,

$Q^1$ represents halogen; cyano; carboxyl; ($C_1$-$C_{10}$)alkyl; halo($C_1$-$C_6$)alkyl; ($C_1$-$C_6$)alkoxy($C_1$-$C_6$)alkyl; halo ($C_1$-$C_6$)alkoxy($C_1$-$C_6$)alkyl; ($C_1$-$C_{10}$)alkoxy; halo($C_1$-$C_6$)alkoxy; cyclo($C_3$-$C_6$)alkoxy; halocyclo($C_3$-$C_6$)alkoxy; ($C_1$-$C_6$)alkoxy($C_1$-$C_6$)alkoxy; halo($C_1$-$C_6$)alkoxy($C_1$-$C_6$)alkoxy; ($C_1$-$C_3$)alkoxycarbonyl($C_1$-$C_3$)alkoxy; hydroxyamino($C_1$-$C_3$)alkoxy($C_1$-$C_3$)alkoxy; ($C_1$-$C_6$)alkylthio($C_1$-$C_6$)alkoxy; ($C_1$-$C_6$)alkylsulfinyl($C_1$-$C_6$)alkoxy; ($C_1$-$C_6$)alkylsulfonyl($C_1$-$C_6$)alkoxy; di($C_1$-$C_3$)alkylamino($C_1$-$C_3$)alkoxy; ($C_2$-$C_6$)alkenyl; halo($C_2$-$C_6$)alkenyl; hydroxyhalo($C_2$-$C_6$) alkenyl; ($C_2$-$C_6$)alkenyloxy; halo($C_2$-$C_6$)alkenyloxy; ($C_2$-$C_6$)alkynyloxy; amino; mono($C_1$-$C_6$)alkylamino; di($C_1$-$C_6$) alkylamino of which alkyl moieties are the same or different; substituted phenyl which is substituted with one or more halo($C_1$-$C_3$)alkyls which are the same or different; pyrolyl; imidazolyl; substituted imidazolyl which is substituted with one or more ($C_1$-$C_3$)alkyls which are the same or different; pyrazolyl; substituted pyrazolyl having one or more substituents which are the same or different and are selected from the group consisting of ($C_1$-$C_3$)alkyl and halo($C_1$-$C_3$)alkyl; substituted pyrazolyl($C_1$-$C_3$)alkyl which is substituted on its ring with one or more halo($C_1$-$C_3$) alkyls which are the same or different; triazolyl; phenoxy; substituted phenoxy having one or more substituents which are the same or different and are selected from the group consisting of halo($C_1$-$C_3$)alkyl and halo($C_1$-$C_3$) alkoxy; ($C_1$-$C_3$)alkylthio; ($C_1$-$C_3$)alkylsulfinyl; ($C_1$-$C_3$)alkylsulfonyl; ($C_1$-$C_3$)alkylsulfonyloxy; halo($C_1$-$C_3$)alkylsulfonyloxy; phenylsulfonyloxy; substituted phenylsulfonyloxy which is substituted with one or more ($C_1$-$C_3$)alkyls which are the same or different; di($C_1$-$C_3$)alkylaminosulfonyloxy of which alkyl moieties are the same or different; ($C_2$-$C_4$) alkenylsulfonyloxy; ($C_1$-$C_3$)alkylcarbonyloxy; ($C_1$-$C_3$)alkoxycarbonyloxy; ($C_1$-$C_3$)alkoxycarbonyl; aminocarbonyl; or substituted phenylaminocarbonyl which is substituted with one or more halogens which are the same or different,

$Q^2$ is halogen; hydroxyl; ($C_1$-$C_6$)alkyl; halo($C_1$-$C_6$)alkyl; cyclo($C_3$-$C_6$)alkyl; halocyclo($C_3$-$C_6$)alkyl; ($C_1$-$C_6$) alkoxy; halo($C_1$-$C_6$)alkoxy; ($C_1$-$C_6$)alkylthio; ($C_1$-$C_6$)alkylsulfinyl; ($C_1$-$C_6$)alkylsulfonyl; substituted phenyl having one or more substituents which are the same or different and are selected from the group consisting of halogen, cyano, ($C_1$-$C_3$)alkyl, halo($C_1$-$C_3$)alkyl, ($C_1$-$C_3$)alkoxy, halo($C_1$-$C_3$)alkoxy, and halo($C_1$-$C_3$)alkylthio; or substituted phenoxy having one or more substituents which are the same or different and are selected from the group consisting of halogen, cyano, ($C_1$-$C_3$)alkyl, halo($C_1$-$C_3$)alkyl, ($C_1$-$C_3$)alkoxy, halo($C_1$-$C_3$)alkoxy, and halo($C_1$-$C_3$)alkylthio.

**15.** A method for producing a substituted thienopyrimidine derivative represented by formula (I-2):

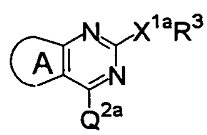

( I - 2 )

wherein A, $R^3$, $X^{1a}$ and $Q^{2a}$ have the same meanings as described below;

which comprises reacting a compound of formula (I-1):

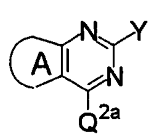

( I - 1)

wherein A represents the following A1 or A2:

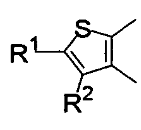

A1

wherein $R^1$ represents hydrogen, $(C_1-C_6)$alkyl, or halo$(C_1-C_6)$alkyl, and
$R^2$ represents hydrogen, halogen, $(C_1-C_6)$alkyl, or halo$(C_1-C_6)$alkyl:

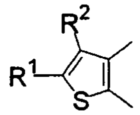

A2

wherein $R^1$ and $R^2$ have the same meanings as described above,
Y represents halogen,
$Q^{2a}$ represents:

substituted aryl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
substituted aryloxy having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
substituted arylthio having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
substituted arylsulfinyl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
substituted arylsulfonyl having one or more substituents which are the same or different and are selected from the group consisting of fluorine-containing $(C_1-C_6)$alkyl, fluorine-containing $(C_1-C_6)$alkoxy, and fluorine-containing $(C_1-C_6)$alkylthio;
with a compound represented by formula (II):

$$R^3X^{1a}H \qquad (II)$$

wherein $X^{1a}$ is a single bond, -O-, or -S-,

$R^3$ represents $(C_1-C_{10})$alkyl; halo$(C_1-C_{10})$alkyl; cyclo$(C_3-C_6)$alkyl; halocyclo$(C_3-C_6)$alkyl; $(C_1-C_6)$alkoxy $(C_1-C_6)$alkyl; halo$(C_1-C_6)$alkoxy$(C_1-C_6)$alkyl; $(C_1-C_4)$alkyl$(C_3-C_6)$cycloalkyl; cyclo$(C_3-C_6)$alkyl$(C_1-C_4)$alkyl; $(C_1-C_3)$alkoxycarbonyl$(C_1-C_3)$alkyl; $(C_1-C_4)$alkylthio$(C_1-C_4)$alkyl; $(C_1-C_4)$alkylsulfinyl$(C_1-C_4)$alkyl; $(C_1-C_4)$alkylsulfonyl$(C_1-C_4)$alkyl; $(C_2-C_6)$alkenyl; halo$(C_2-C_6)$alkenyl; hydroxy$(C_2-C_6)$alkenyl; hydroxyhalo$(C_2-C_6)$alkenyl; phenyl $(C_2-C_6)$alkenyl; $(C_2-C_6)$alkynyl; halo$(C_2-C_6)$alkynyl; amino; mono$(C_1-C_6)$alkylamino; monohalo$(C_1-C_6)$alkylamino; di$(C_1-C_6)$alkylamino of which alkyl moieties are the same or different; dihalo$(C_1-C_6)$alkylamino of which dihaloalkyl moieties are the same or different;

phenylamino; substituted phenylamino having on its ring one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo $(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl; substituted aryl having one or more substituents which are the same or different and are selected from the group consisting of halogen, cyano, nitro, hydroxyl, amino, SH, $(C_1-C_6)$alkyl, halo$(C_1-C_6)$alkyl, cyclo$(C_3-C_6)$ alkyl, halocyclo$(C_3-C_6)$alkyl, $(C_2-C_6)$alkenyl, halo$(C_2-C_6)$alkenyl, $(C_2-C_6)$alkynyl, halo$(C_2-C_6)$alkynyl, $(C_1-C_6)$ alkoxy, halo$(C_1-C_6)$alkoxy, cyclo$(C_3-C_6)$alkoxy, halocyclo$(C_3-C_6)$alkoxy, $(C_1-C_6)$alkylthio, halo$(C_1-C_6)$alkylthio, cyclo$(C_3-C_6)$alkylthio, halocyclo$(C_3-C_6)$alkylthio, $(C_1-C_6)$alkylsulfinyl, halo$(C_1-C_6)$alkylsulfinyl, cyclo$(C_3-C_6)$alkylsulfinyl, halocyclo$(C_3-C_6)$alkylsulfinyl, $(C_1-C_6)$alkylsulfonyl, halo$(C_1-C_6)$alkylsulfonyl, cyclo$(C_3-C_6)$alkylsulfonyl, halocyclo$(C_3-C_6)$alkylsulfonyl, mono$(C_1-C_6)$alkylamino, and di$(C_1-C_6)$alkylamino of which alkyl moieties are the same or different;

aryl($C_1$-$C_6$)alkyl; substituted aryl($C_1$-$C_6$)alkyl having on its ring one or more substituents which are the same or different and are selected from the group consisting of halogen, cyano, nitro, hydroxyl, amino, SH, ($C_1$-$C_6$)alkyl, halo($C_1$-$C_6$)alkyl, cyclo($C_3$-$C_6$)alkyl, halocyclo($C_3$-$C_6$)alkyl, ($C_2$-$C_6$)alkenyl, halo($C_2$-$C_6$)alkenyl, ($C_2$-$C_6$)alkynyl, halo($C_2$-$C_6$)alkynyl, ($C_1$-$C_6$)alkoxy, halo($C_1$-$C_6$)alkoxy, cyclo($C_3$-$C_6$)alkoxy, halocyclo($C_3$-$C_6$)alkoxy, ($C_1$-$C_6$)alkylthio, halo($C_1$-$C_6$)alkylthio, cyclo($C_3$-$C_6$)alkylthio, halocyclo($C_3$-$C_6$)alkylthio, ($C_1$-$C_6$)alkylsulfinyl, halo($C_1$-$C_6$)alkylsulfinyl, cyclo($C_3$-$C_6$)alkylsulfinyl, halocyclo($C_3$-$C_6$)alkylsulfinyl, ($C_1$-$C_6$)alkylsulfonyl, halo($C_1$-$C_6$)alkylsulfonyl, cyclo($C_3$-$C_6$)alkylsulfonyl, halocyclo($C_3$-$C_6$)alkylsulfonyl, mono($C_1$-$C_6$)alkylamino, and di($C_1$-$C_6$)alkylamino of which alkyl moieties are the same or different;

a heterocyclic group of which heterocycle is oxirane, oxetane, tetrahydrofuran, furan, thiophene, pyrrole, pyrrolidine, oxazole, oxazoline, oxazolidine, thiazole, thiazoline, thiazolidine, imidazole, imidazoline, imidazolidine, triazole, triazolidine, isoxazole, isoxazoline, isothiazole, isothiazolidine, pyrazole, pyrazoline, pyrazolidine, tetrazole, tetrahydropyran, pyridine, pyrimidine, pyridazine, morpholine, thiomorpholine, piperazine, piperidine, or oxazine;

a substituted heterocyclic group of which heterocycle has the same meaning as described above, having one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy;

heterocyclic ($C_1$-$C_6$)alkyl of which heterocycle has the same meaning as described above; or substituted heterocyclic ($C_1$-$C_6$)alkyl of which heterocycle has the same meaning as described above, having, on its ring, one or more substituents which are the same or different and are selected from the group consisting of halogen, cyano, nitro, ($C_1$-$C_6$)alkyl, halo($C_1$-$C_6$)alkyl, cyclo($C_3$-$C_6$)alkyl, halocyclo($C_3$-$C_6$)alkyl, ($C_1$-$C_6$)alkylcarbonyloxy, ($C_1$-$C_6$)alkoxy, halo($C_1$-$C_6$)alkoxy, cyclo($C_3$-$C_6$)alkoxy, and halocyclo($C_3$-$C_6$)alkoxy.

**16.** A process for producing a substituted thieno[2,3-d]pyrimidine derivative represented by formula (I-3):

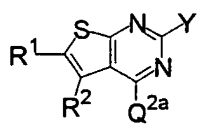

(I - 3)

wherein $R^1$, $R^2$, $Q^{2a}$, and Y have the same meanings as described below,

which comprises carrying out a coupling reaction of 2,4-dihalogenothieno[2,3-d]pyrimidine derivative represented by formula(III):

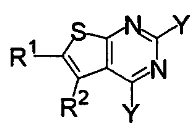

(III)

wherein $R^1$ represents hydrogen, ($C_1$-$C_6$)alkyl, or halo($C_1$-$C_6$)alkyl,

$R^2$ represents hydrogen, halogen, ($C_1$-$C_6$)alkyl, or halo($C_1$-$C_6$)alkyl, and

Y represents halogen which are the same or different,

with a compound represented by formula (IV):

$$Q^{2a}\text{-L} \qquad \text{(IV)}$$

wherein $Q^{2a}$ represents substituted aryl having one or more substituents which are the same or different and are selected from the group consisting of fluorine-containing ($C_1$-$C_6$)alkyl, fluorine-containing ($C_1$-$C_6$)alkoxy, and fluorine-containing ($C_1$-$C_6$)alkylthio, and

L represents a leaving group.

**17.** 2-Amino-3-(substituted benzoyl)thiophene derivative represented by formula (V):

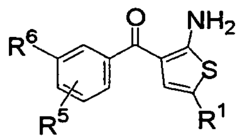

( V )

wherein $R^1$ represents hydrogen, $(C_1\text{-}C_6)$alkyl, or halo$(C_1\text{-}C_6)$alkyl,
$R^5$ represents hydrogen, halogen, $(C_1\text{-}C_6)$alkyl, or halo$(C_1\text{-}C_6)$alkyl,
$R^6$ represents fluorine-containing $(C_1\text{-}C_6)$alkyl, fluorine-containing $(C_1\text{-}C_6)$alkoxy, or fluorine-containing $(C_1\text{-}C_6)$alkylthio.

**18.** A ureidethiophene derivative represented by formula (VI):

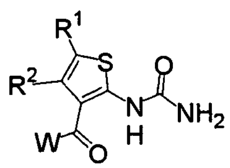

( VI )

wherein $R^1$ represents hydrogen, $(C_1\text{-}C_6)$alkyl, or halo$(C_1\text{-}C_6)$alkyl,
$R^2$ represents hydrogen, halogen, $(C_1\text{-}C_6)$alkyl, or halo$(C_1\text{-}C_6)$alkyl, and
W represents aryl or substituted aryl having one or more substituents which are the same or different and are selected from the group consisting of halogen, cyano, nitro, $(C_1\text{-}C_6)$alkyl, halo$(C_1\text{-}C_6)$alkyl, $(C_1\text{-}C_6)$alkoxy, halo $(C_1\text{-}C_6)$alkoxy, and halo$(C_1\text{-}C_6)$alkylthio.

**19.** A substituted benzoylacetonitrile derivative represented by formula (VII):

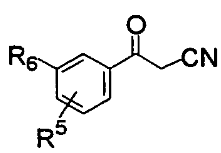

(VII)

wherein $R^5$ represents hydrogen, halogen, $(C_1\text{-}C_6)$alkyl, or halo$(C_1\text{-}C_6)$alkyl,
$R^6$ represents fluorine-containing $(C_1\text{-}C_6)$alkyl, fluorine-containing $(C_1\text{-}C_6)$alkoxy, and fluorine-containing $(C_1\text{-}C_6)$alkylthio,
and wherein, when $R^6$ is fluorine-containing $(C_1\text{-}C_6)$alkyl, $R^5$ is not hydrogen.

**20.** 3-(Trifluoromethoxy)ethyl benzoate.

## INTERNATIONAL SEARCH REPORT

International application No.
PCT/JP03/12356

A. CLASSIFICATION OF SUBJECT MATTER
Int.Cl[7] C07D495/04, A01N43/90

According to International Patent Classification (IPC) or to both national classification and IPC

B. FIELDS SEARCHED
Minimum documentation searched (classification system followed by classification symbols)
Int.Cl[7] C07D495/04, A01N43/90

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| X | TAKAHASHI, A. et al., 'Mitotic disruption by a novel pyrimidine herbicide, NS-245852, in oat (Avena sativa L.) roots.', Weed Biology and Management, 2001, Vol.1, No.3, pages 182 to 188, full text, Registry No. 158350-38-0 | 1-16 |
| X | TANAKA, K. et al., 'Synthesis and herbicidal activity of pyrimidine derivatives.' Pesticide Science, 1999, Vol.55, No.3, pages 370 to 372, full text, page 371, table 1, compounds 16, 17, 22, registry No. 158350-34-6, 158350-38-0, 224446-53-1 | 1-16 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 December, 2003 (04.12.03) | 24 December, 2003 (24.12.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/12356

C (Continuation). DOCUMENTS CONSIDERED TO BE RELEVANT

| Category* | Citation of document, with indication, where appropriate, of the relevant passages | Relevant to claim No. |
|---|---|---|
| X | EP 579424 A1 (ROHM AND HAAS CO.), 19 January, 1994 (19.01.94), Full text; registry No. 18678-14-3 & US 5300477 A & JP 6-87835 A & CA 2099928 A1 & US 5378678 A & US 5451565 A | 1-16 |
| X | WO 94/8975 A1 (NIPPON SODA CO., LTD.), 28 April, 1994 (28.04.94), Full text; Claims; test examples 1, 2; registry No. 158350-29-9, 158350-34-6, 158350-38-0, 158350-52-8, 158350-95-9, 158350-96-0, 158351-32-7, 158351-33-8, 158351-34-9, 158351-41-8, 158351-58-7, 158351-68-9, 158351-69-0, 158351-70-3, 158351-71-4, 158351-72-5, 158351-75-8 & AU 9351611 B & EP 665224 A1 & JP 7-48359 A | 1-16 |
| X | IFE, R. J. et al., 'Reversible inhibitors of the gastric($H^+/K^+$-ATPase. 5., Substituted 2, 4-diamino quinazolines and thienopyrimidines.', J.Med.Chem., 1995, Vol.38, pages 2763 to 2773, full text, pages 2766 to 2767, scheme 7-9, page 2769, table 3, registry No. 16233-53-7, 134372-90-0, 134372-91-1, 134372-92-2, 134372-93-3, 134372-95-5, 134372-96-6, 134372-97-7, 134372-98-8, 169270-49-9 | 7-16 |
| X | YAMAGUCHI, H. et al., 'Synthesis and reactions of 2-chloro-3, 4-dihydrothienopyrimidines and-quina zolines.', J.Med.Chem., 1981, Vol.18, pages 67 to 70, full text, schems 1, registry No. 16234-14-3, 42518-42-3 | 7-16 |
| X | RAM, V. J. et al., 'Thieno [2,3-d]pyrimidines as potential chemotherapeutic agents.II.', J.Hetero cyclic Chem., 1981, Vol.18, No.7, pages 1277 to 1280, full text, pages 1277, compounds 4b, 6b, 8a-f, registry No. 81136-42-7, 18593-51-6, 81136-43-8, 81136-44-9 | 7-16 |
| X | GB 1570494 A (IMPERIAL CHEMICAL INDUSTRIES LTD.), 02 July, 1980 (02.07.80), & DE 2654090 A1 & AU 7619799 B & AU 7619796 B & JP 52-66678 A & US 4146716 A & FR 2332992 A1 & FR 2353637 A1 & JP 52-68197 A | 7-16 |
| X | WO 02/62803 A1 (YAMANOUCHI PHARM. CO., LTD.), 15 August, 2002 (15.08.02), & JP 2002-308882 A | 7-16 |

Form PCT/ISA/210 (continuation of second sheet) (July 1998)

## INTERNATIONAL SEARCH REPORT

International application No.
PCT/JP03/12356

C (Continuation). DOCUMENTS CONSIDERED TO BE RELEVANT

| Category* | Citation of document, with indication, where appropriate, of the relevant passages | Relevant to claim No. |
|---|---|---|
| X | WO 02/55524 A1 (VERNALIS RES. LTD.), 18 July, 2002 (18.07.02), & EP 1349861 A1 | 7-16 |
| X | WO 01/32632 A1 (LILLY & CO. ELI), 10 May, 2001 (10.05.01), & AU 2001/10713 B & EP 1230225 A1 | 7-16 |
| X | WO 01/2409 A1 (VERNALIS RES. LTD.), 11 January, 2001 (11.01.01), & AU 2000/55578 B & EP 1192164 A1 & JP 2003-503504 A | 7-16 |
| X | US 6133271 A (CELL PATHWAYS INC.), 17 October, 2000 (17.10.00), (Family: none) | 7-16 |
| X | US 5948911 A (CELL PATHWAYS INC.), 07 September, 1999 (07.09.99), (Family: none) | 7-16 |
| X | EP 899263 A2 (FUJIREBIO INC.), 03 March, 1999 (03.03.99), & JP 11-124371 A & US 2001/6969 A | 7-16 |
| X | WO 98/17668 A1 (MERCK PATENT GMBH.), 30 April, 1998 (30.04.98), & DE 19644228 A1 & AU 97/49450 B & EP 934321 A1 & US 6130223 A & JP 2001-502342 A | 7-16 |
| X | WO 98/6722 A1 (MERK PATENT GMBH.), 19 February, 1998 (19.02.98), & DE 19632423 A1 & AU 9742035 B & EP 920431 A1 & US 6110920 A & JP 2000-516223 A | 7-16 |
| X | WO 98/17021 A1 (PFIZER INC.), 02 September, 1993 (02.09.93), & EP 626964 A1 & JP 7-500115 A & US 5583137 A | 7-16 |
| X | EP 404356 A1 (SMITHKLINE BEECHAM INTERCREDIT), 27 December, 1990 (27.12.90), & JP 3-17083 A & US 5280026 A | 7-16 |
| X | EP 82023 A1 (SANKYO CO., LTD.), 22 June, 1983 (22.06.83), & JP 58-121291 A & JP 58-225089 A | 7-16 |
| X | US 4196207 A (IMPERIAL CHEM. IND. LTD.), 01 April, 1980 (01.04.80), & GB 1597786 A | 7-16 |

Form PCT/ISA/210 (continuation of second sheet) (July 1998)

## INTERNATIONAL SEARCH REPORT

International application No.
PCT/JP03/12356

C (Continuation). DOCUMENTS CONSIDERED TO BE RELEVANT

| Category* | Citation of document, with indication, where appropriate, of the relevant passages | Relevant to claim No. |
|---|---|---|
| X | KOUROUNAKIS, A.P. et al., 'Elucidation of structure-activity relationships of 2-amino-3-benzoylthiophenes: study of their allosteric enhancing vs. antagonistic activity on adenosin $A_1$ receptors.', Drug Development Research, 2000, Vol.49, No.4, pages 227 to 237, full text, page 233, table 1, compound 4a, registry No. 288142-34-7 | 17 |
| X | EP 202538 A1 (BAYER AG.), 26 November, 1986 (26.11.86), Full text; Claims & DE 3529247 A1 & JP 61-268678 A | 18 |
| X | WO 01/40202 A1 (BAYER AG.), 07 June, 2001 (07.06.01), Full text; registry No. 267880-81-9, 267880-84-2, 267881-02-7, 267881-04-9 & DE 19958164 A1 | 19 |
| X | WO 00/27812 A1 (BAYER AG.), 18 May, 2000 (18.05.00), Full text; registry No. 267880-84-2, 267881-02-7, 267881-04-9 & DE 19851986 A1 & EP 1129071 A1 & JP 2002-529450 A & US 6455472 A & US 2003/130125 A | 19 |
| X | JP 5-78271 A (Sagami Chemical Research Center), 30 March, 1993 (30.03.93), Full text; Claim 4; Par. Nos. [0056], [0078] (Family: none) | 20 |

Form PCT/ISA/210 (continuation of second sheet) (July 1998)

**5INTERNATIONAL SEARCH REPORT**

International application No.
PCT/JP03/12356

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

(See extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

INTERNATIONAL SEARCH REPORT

International application No.
PCT/JP03/12356

Continuation of Box No. II of continuation of first sheet(1)

<Concerning the unity of invention>

Compounds falling within the scope prescribed by the general formula (I) in claim 1 or that prescribed by the general formula (I) in claim 7 and herbicides containing the compounds as the active ingredient are matters known before the priority date of this application as described in any of the documents cited in Box C, that is,

· TAKAHASHI,A. et al. "Mitotic disruption by a novel pyrimidine herbicide, NS-245852, in oat (Avena sativa L.) roots." Weed Biology and Management, 2001, Vol.1, No.3, pages 182 to 188, full text, registry No. 158350-38-0

· TANAKA, K. et al. "Synthesis and herbicidal activity of pyrimidine derivatives." Pesticide Science, 1999, Vol.55, No.3, pages 370 to 372, full text; page 371, table 1, compounds 16, 17, 22 registry No. 158350-34-6, 158350-38-0, 224446-53-1

· EP 579424 A1 (ROHM AND HAAS CO.), 19 January, 1994 (19.01.94), full text, registry No. 18678-14-3 & US 5300477 A & JP 6-87835 A & CA 2099928 A1 & US 5378678 A & US 5451565 A

· WO 94/8975 A1 (NIPPON SODA CO., LTD.) 28 April, 1994 (28.04.94), full text, claim, test examples 1, 2 registry No. 158350-29-9, 158350-34-6, 158350-38-0, 158350-52-8, 158350-95-9, 158350-96-0, 158351-32-7, 158351-33-8, 158351-34-9, 158351-41-8, 158351-58-7, 158351-68-9, 158351-69-0, 158351-70-3, 158351-71-4, 158351-72-5, 158351-75-8 & AU 9351611 B & EP 665224 A1 & JP 7-48359 A,

and compounds represented by the general formula (V) in claim 17 and those represented by the general formula (VII) in claim 19 are also matters known before the priority date of this application as described in any of the documents cited in Box C, that is,

· KOUROUNAKIS, A.P. et al. "Elucidation of structure-activity relationships of 2-amino-3-benzoylthiophenes: study of their allosteric enhancing vs. antagonistic activity on adenosin $A_1$ receptors." Drug Development Research, 2000, Vol.49, No.4, pages 227 to 237, full text; page 233, table 1. compound 4a registry No. 288142-34-7

· WO 01/40202 A1 (BAYER AG.) 07 June, 2001 (07.06.01), full text, registry No. 267880-81-9, 267880-84-2, 267881-02-7, 267881-04-9 & DE 19958164 A1

· WO 00/27812 A1 (BAYER AG.) 18 May, 2000 (18.05.00), full text, registry No. 267880-84-2, 267881-02-7 267881-04-9 & DE 19851986 A1 & EP 1129071 A1 & JP 2002-529450 A & US 6455472 A & US 2003/130125 A.

Thus, all of the groups [1] to [5] of inventions:

[1] a group of inventions of claims 1-6, 7-14, 15, 16,
[2] claim 17,
[3] claim 18,
[4] claim 19, and
[5] claim 20

do not have a special technical feature in common and the groups [1] to [5] of inventions are not considered as being so linked as to form a single general inventive concept.

(continue to next sheet)

**INTERNATIONAL SEARCH REPORT**

International application No.
PCT/JP03/12356

<Concerning the scope of search>

The general formulae in claims 7-16 each include extremely many compounds, but only a few of the claimed compounds are supported by the description within the meaning of PCT Article 6 and disclosed within the meaning of PCT Article 5.

Therefore, this international search covers only prior documents which describe or suggest not only compounds represented by the general formulae in the above claims but also that the compounds are useful as active ingredients of herbicides.

Form PCT/ISA/210 (extra sheet) (July 1998)